# EUROPEAN PATENT APPLICATION

(11) **EP 4 570 796 A1**
(43) Date of publication of application: **18.06.2025**
(21) Application number: 23888646.9
(22) Date of filing: 06.11.2023
(51) Int. Cl.: C07D 407/06, C07B 61/00, C07D 303/16, C07F 5/02, C07F 5/04

(54) **METHOD FOR PRODUCING MACROLIDE COMPOUND**

(30) Priority: 07.11.2022 JP 2022178188
(71) Applicant: Eisai R&D Management Co., Ltd., Tokyo 112-8088 (JP)
(72) Inventor: YONEDA Naoki, Tsukuba-shi, Ibaraki 300-2635 (JP); SATO Nobuaki, Tsukuba-shi, Ibaraki 300-2635 (JP); NAKAUE Tsubasa, Tsukuba-shi, Ibaraki 300-2635 (JP); NAKATANI Yusuke, Tsukuba-shi, Ibaraki 300-2635 (JP); YAMASHITA Taro, Tsukuba-shi, Ibaraki 300-2635 (JP); ITO Yoko, Tsukuba-shi, Ibaraki 300-2635 (JP)
(74) Representative: Hoffmann Eitle
(86) International application number: PCT/JP2023/039845
(87) International publication number: WO 2024/101298

(57) **Abstract**

Disclosed are a method for producing pladienolide D that includes a step that reacts a compound represented by formula (A1) with a compound represented by formula (B1) in the presence of a metal catalyst to obtain a compound represented by formula (C1) (in the formulas, X means hydrogen, optionally substituted boryl, optionally substituted stannyl, or optionally substituted silyl, R⁴ is hydrogen, etc., R⁵ is optionally substituted benzoyl, etc., and R¹ and R² each independently are hydrogen, etc.) and crystals of a solvate of pladienolide D.

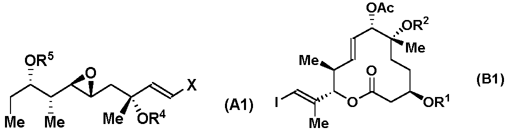

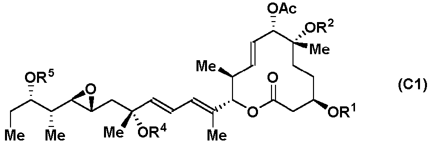

## Description

### Technical Field

The present invention relates to a method of producing pladienolide D, which is a macrolide-based natural product having an antitumor activity, and a crystal of pladienolide D.

### Background Art

Pladienolide D represented by the following formula (1): 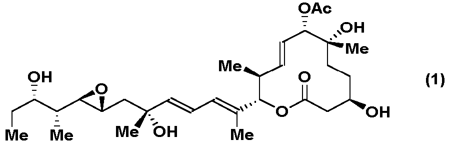 is a 12-membered ring macrolide-based compound having an antitumor activity. Examples of the major production method include fermentation production using a strain such as Streptomyces sp. Mer-11107 (Patent Literature 1). However, in the production by fermentation, there remains a problem in purification from a culture, it is difficult to isolate high purity pladienolide D, and the production amount is limited, which is not suitable for mass production. On the other hand, studies by chemical total synthesis have also been conducted, and several reports have been made on total synthesis of pladienolide D so far, but further improvement of the production method is desired in order to perform mass synthesis (Patent Literature 2 and Non Patent Literature 1).

Pladienolide D is also used as a starting material for conversion to an analog having various antitumor activities (Patent Literature 3). Therefore, it is expected to establish a production method incorporating a synthesis route enabling synthesis with high yield and a purification method enabling stable quality control without using a silica gel column, and it is desired to establish a production method for pladienolide D applicable to mass synthesis.

### Citation List

### Patent Literature

Patent Literature 1: WO 02/060890 A
Patent Literature 2: WO 2007/043621 A
Patent Literature 3: WO 2015/175594 A

### Non Patent Literature

Non Patent Literature 1: Regina M. Kanada et al., "Total Synthesis of the Potent Antitumor Macrolides Pladienolide B and D", Angew. Chem. Int. Ed. 2007, 46, 4350-4355

### Summary of Invention

### Technical Problem

An object of the present invention is to provide an efficient method of synthesizing pladienolide D which has an antitumor activity and is used as a synthetic intermediate of an antitumor agent.

### Solution to Problem

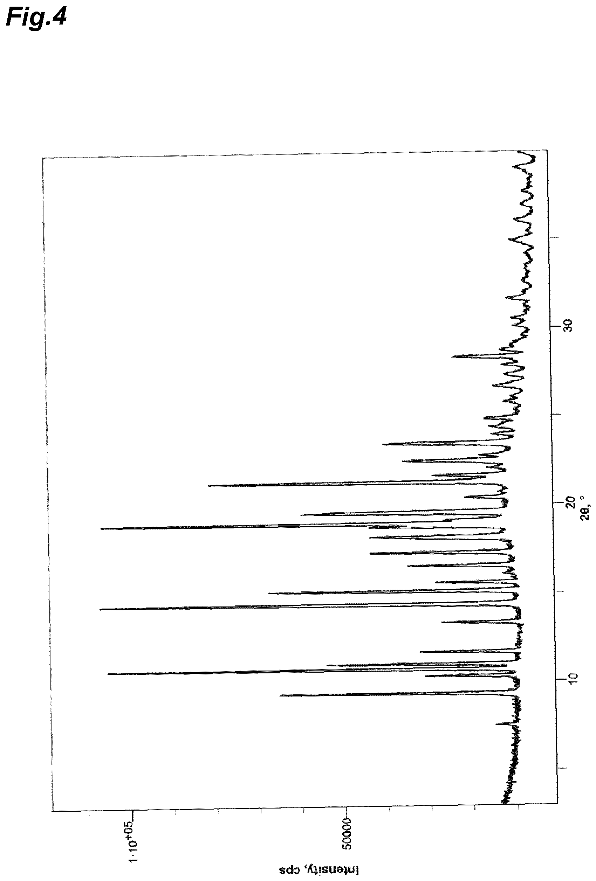

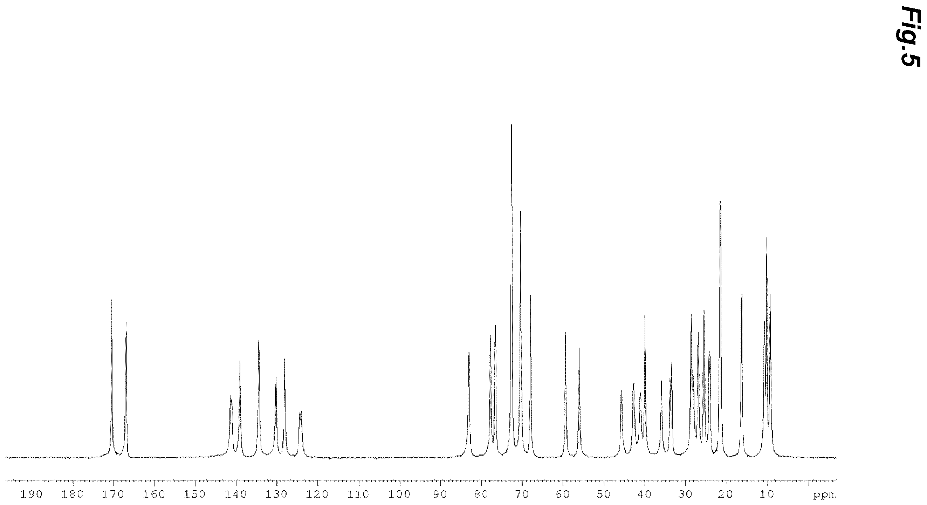

The present invention is as follows.
[1] A method of producing pladienolide D represented by formula (1): 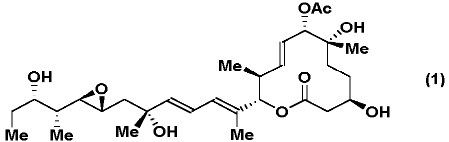 or a salt thereof, or a solvate thereof,
   the method comprising the following steps:
   step 1-1) allowing a compound represented by formula (A1): 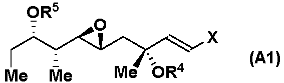
   wherein X is hydrogen, optionally substituted boryl, optionally substituted stannyl, or optionally substituted silyl, R⁴ is hydrogen or a silyl protecting group, and R⁵ is hydrogen, optionally substituted benzoyl, or a silyl protecting group,
   to react with a compound represented by formula (B1): 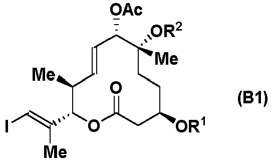
   wherein R¹ and R² are each independently hydrogen or a silyl protecting group, in the presence of a metal catalyst to obtain a compound represented by formula (C1): 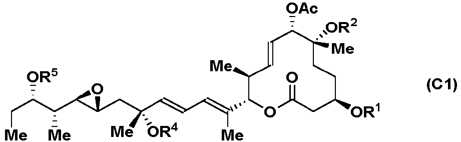
   wherein R¹, R², and R⁴ are each independently hydrogen or a silyl protecting group, and R⁵ is hydrogen, optionally substituted benzoyl, or a silyl protecting group; and
   optionally, step 1-3) converting pladienolide D to a solvate,
   with the proviso that when any one or more of R¹, R², R⁴, and R⁵ in the compound represented by formula (C1) obtained in step 1-1 is not hydrogen, the method further comprising the following step:
      step 1-2) removing the protecting group(s) of the compound represented by formula (C1) obtained in step 1-1 to obtain pladienolide D after step 1-1.
[2] A method of producing pladienolide D represented by formula (1): 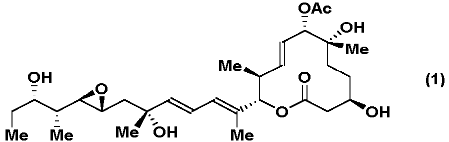 or a salt thereof, or a solvate thereof,
   the method comprising the following steps:
   step 2-1) allowing a compound represented by formula (A2): 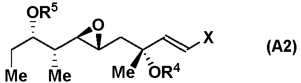
   wherein X is hydrogen or optionally substituted boryl, R⁴ is hydrogen or a silyl protecting group, and R⁵ is hydrogen or optionally substituted benzoyl, to react with a compound represented by formula (B1): 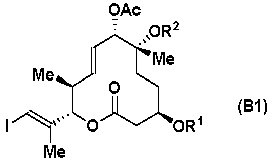
   wherein R¹ and R² are each independently hydrogen or a silyl protecting group,
   in the presence of a metal catalyst to obtain a compound represented by formula (C2): 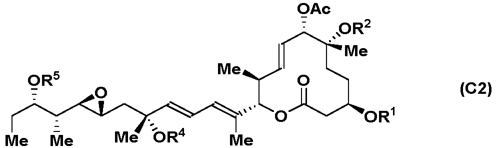
   wherein R¹, R², and R⁴ are each independently hydrogen or a silyl protecting group, and R⁵ is hydrogen or optionally substituted benzoyl;
   step 2-2) removing the protecting group(s) of the compound represented by formula (C2) obtained in step 2-1 to obtain pladienolide D; and
   optionally, step 2-3) converting pladienolide D obtained in step 2-2 to a solvate.
[3] The method according to [2], wherein R⁵ is hydrogen.
[4] The method according to [2] or [3], wherein R⁵ is 2,4-dinitrobenzoyl.
[5] The method according to [2] or [3], wherein R⁵ is 3,5-dinitrobenzoyl.
[6] The method according to any one of [2] to [5], wherein R⁴ is hydrogen.
[7] The method according to any one of [2] to [6], wherein R² is hydrogen.
[8] The method according to any one of [2] to [7], wherein R¹ is hydrogen.
[9] The method according to any one of [2] to [7], wherein R¹ is a silyl protecting group.
[10] The method according to any one of [2] to [7], wherein R¹ is silyl represented by SiR⁶R⁷R⁸, and R⁶, R⁷, and R⁸ are each independently selected from the group consisting of methyl, ethyl, isopropyl, tert-butyl, and phenyl.
[11] The method according to any one of [2] to [7], wherein R¹ is tert-butyl(dimethyl)silyl.
[12] The method according to any one of [2] to [11], wherein X is hydrogen.
[13] The method according to any one of [2] to [11], wherein X is optionally substituted boryl.
[14] The method according to any one of [2] to [11], wherein X is boronic acid, pinacol boronate ester, or trifluoroborate.
[15] The method according to any one of [2] to [14], wherein the metal catalyst is a palladium catalyst.
[16] The method according to any one of [2] to [14], wherein the metal catalyst is palladium(II) acetate.
[17] The method according to any one of [2] to [14], wherein the metal catalyst is tris(dibenzylideneacetone)dipalladium(0).
[18] The method according to any one of [2] to [14], wherein the metal catalyst is (1,1'-bis(diphenylphosphino)ferrocene)dichloropalladium(H).
[19] The method according to any one of [2] to [18], wherein the step of removing the protecting group(s) comprises removal using an acid and removal using a base.
[20] The method according to any one of [2] to [18], wherein the step of removing the protecting group(s) comprises removal using a fluoride ion and removal using a base.
[21] The method according to any one of [2] to [18], wherein the step of removing the protecting group(s) comprises removal using a base.
[22] The method according to any one of [2] to [18], wherein the step of removing the protecting group(s) comprises removal using a fluoride ion.
[23] The method according to any one of [2] to [18], wherein the step of removing the protecting group(s) comprises removal using lithium hydroxide.
[24] The method according to any one of [2] to [18], wherein the step of removing the protecting group(s) comprises removal using tetrabutylammonium fluoride.
[25] The method according to any one of [2] to [18], wherein the step of removing the protecting group(s) comprises removal using tetrabutylammonium fluoride and removal using lithium hydroxide.
[26] A method of producing pladienolide D represented by formula (1): 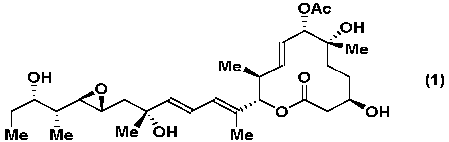 or a salt thereof, or a solvate thereof,
   the method comprising the following steps:
   step 3-1) allowing a compound represented by formula (A1): 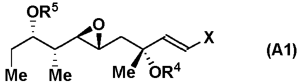
   wherein X is hydrogen, optionally substituted boryl, optionally substituted stannyl, or optionally substituted silyl, R⁴ and R⁵ are each independently hydrogen, optionally substituted benzoyl, or a silyl protecting group,
   to react with a compound represented by formula (B2): 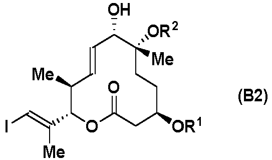 wherein R¹ and R² are each independently hydrogen or a silyl protecting group,
   in the presence of a metal catalyst to obtain a compound represented by formula (C3): 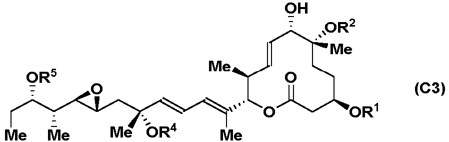
   wherein R¹, R², and R⁴ are each independently hydrogen or a silyl protecting group, and R⁵ is hydrogen, optionally substituted benzoyl, or a silyl protecting group; and
   step 3-2) acetylating a hydroxy group of the compound represented by formula (C3) obtained in step 3-1 to obtain a compound represented by formula (C1): 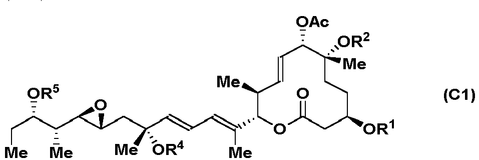
   wherein R¹, R², and R⁴ are each independently hydrogen or a silyl protecting group, and R⁵ is hydrogen, optionally substituted benzoyl, or a silyl protecting group,
   with the proviso that when any one or more of R¹, R², R⁴, and R⁵ is not hydrogen,
   the method further comprising the following step:
      step 3-3) removing the protecting group(s) of the compound represented by formula (C1) obtained in step 3-2 to obtain pladienolide D.
[27] A method of producing pladienolide D represented by formula (1): 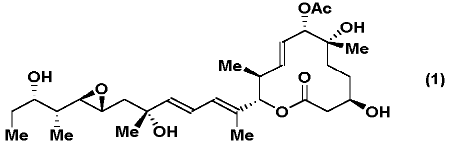 or a salt thereof, or a solvate thereof,
   the method comprising the following steps:
   step 4-1) allowing a compound represented by formula (A2): 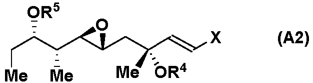
   wherein X is hydrogen or optionally substituted boryl, R⁴ is hydrogen or a silyl protecting group, and R⁵ is hydrogen or optionally substituted benzoyl, to react with a compound represented by formula (B2): 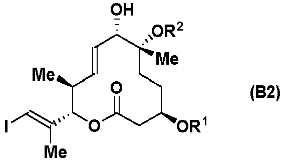
   wherein R¹ and R² are each independently hydrogen or a silyl protecting group,
   in the presence of a metal catalyst to obtain a compound represented by formula (C4): 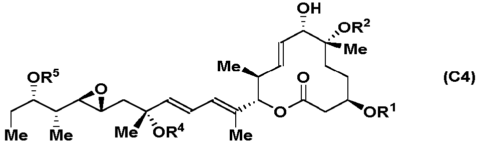
   wherein R¹, R², and R⁴ are each independently hydrogen or a silyl protecting group, and R⁵ is hydrogen or optionally substituted benzoyl;
   step 4-2) acetylating a hydroxy group of the compound represented by formula (C4) obtained in step 4-1 to obtain a compound represented by formula (C2): 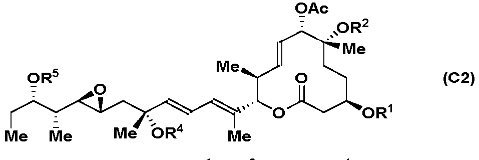
   wherein R¹, R¹, and R⁴ are each independently hydrogen or a silyl protecting group, and R⁵ is optionally substituted benzoyl;
   step 4-3) removing the protecting group(s) of the compound represented by formula (C2) obtained in step 4-2 to obtain pladienolide D; and
   optionally, step 4-4) converting pladienolide D obtained in step 4-3 to a solvate.
[28] The method according to [27], wherein R⁵ is hydrogen.
[29] The method according to [27] or [28], wherein R⁵ is 2,4-dinitrobenzoyl.
[30] The method according to [27] or [28], wherein R⁵ is 3,5-dinitrobenzoyl.
[31] The method according to any one of [27] to [30], wherein R⁴ is hydrogen.
[32] The method according to any one of [27] to [31], wherein R² is hydrogen.
[33] The method according to any one of [27] to [32], wherein R¹ is hydrogen.
[34] The method according to any one of [27] to [32], wherein R¹ is a silyl protecting group.
[35] The method according to any one of [27] to [32], wherein R¹ is silyl represented by SiR⁶R⁷R⁸, and R⁶, R⁷, and R⁸ are each independently selected from the group consisting of methyl, ethyl, isopropyl, tert-butyl, and phenyl.
[36] The method according to any one of [27] to [32], wherein R¹ is tert-butyl(dimethyl)silyl.
[37] The method according to any one of [27] to [36], wherein X is hydrogen.
[38] The method according to any one of [27] to [36], wherein X is optionally substituted boryl.
[39] The method according to any one of [27] to [36], wherein X is boronic acid, pinacol boronate ester, or trifluoroborate.
[40] The method according to any one of [27] to [39], wherein the metal catalyst is a palladium catalyst.
[41] The method according to any one of [27] to [39], wherein the metal catalyst is palladium(II) acetate.
[42] The method according to any one of [27] to [39], wherein the metal catalyst is tris(dibenzylideneacetone)dipalladium(0).
[43] The method according to any one of [27] to [39], wherein the metal catalyst is (1,1'-bis(diphenylphosphino)ferrocene)dichloropalladium(H).
[44] The method according to any one of [27] to [43], wherein the step of removing the protecting group(s) comprises removal using an acid and removal using a base.
[45] The method according to any one of [27] to [43], wherein the step of removing the protecting group(s) comprises removal using a fluoride ion and removal using a base.
[46] The method according to any one of [27] to [43], wherein the step of removing the protecting group(s) comprises removal using a base.
[47] The method according to any one of [27] to [43], wherein the step of removing the protecting group(s) comprises removal using a fluoride ion.
[48] The method according to any one of [27] to [43], wherein the step of removing the protecting group(s) comprises removal using lithium hydroxide.
[49] The method according to any one of [27] to [43], wherein the step of removing the protecting group(s) comprises removal using tetrabutylammonium fluoride.
[50] The method according to any one of [27] to [43], wherein the step of removing the protecting group(s) comprises removal using tetrabutylammonium fluoride and removal using lithium hydroxide.
[51] The method according to any one of [1] to [50], wherein the pladienolide D or the salt thereof or the solvate thereof is pladienolide D 2-methyltetrahydrofuran solvate.
[52] A method of producing a compound represented by formula (B3): 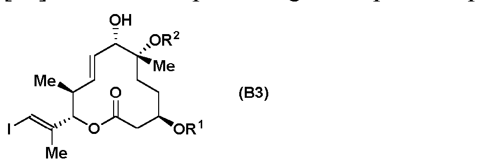 or a salt thereof,
   wherein R¹ and R² are each independently a silyl protecting group,
   the method comprising the following steps:
      step 5-1) forming an ester between a compound represented by formula (D1): 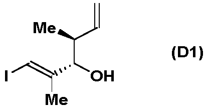 and a compound represented by formula (D2): 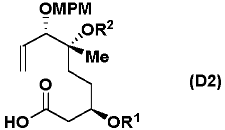
      wherein R¹ and R² are each independently a silyl protecting group, to obtain a compound represented by formula (D3): 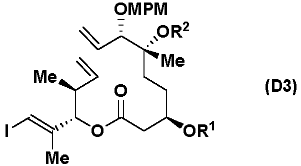
      wherein R¹ and R² are each independently a silyl protecting group;
      step 5-2) removing the para-methoxybenzyl (MPM) group from the compound represented by formula (D3) obtained in step 5-1 to obtain a compound represented by formula (D4): 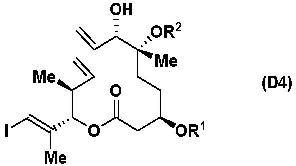
      wherein R¹ and R² are each independently a silyl protecting group; and
      step 5-3) allowing the compound represented by formula (D4) obtained in step 5-2 to react in the presence of a ruthenium catalyst to obtain the compound represented by formula (B3).
[53] A compound represented by formula (C5): 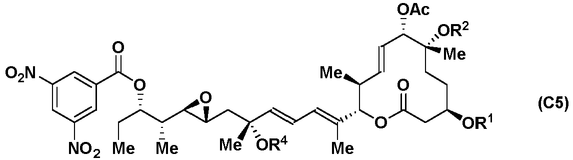 or a salt thereof,
   wherein R¹, R², and R⁴ are each independently hydrogen or a silyl protecting group.
[54] A compound represented by formula (C6): 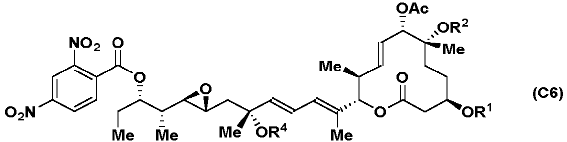 or a salt thereof,
   wherein R¹, R², and R⁴ are each independently hydrogen or a silyl protecting group.
[55] A compound represented by formula (C7): 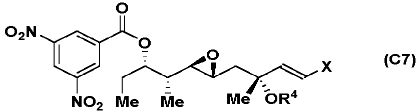 or a salt thereof,
   wherein X is hydrogen or optionally substituted boryl and R⁴ is hydrogen or a silyl protecting group.
[56] A compound represented by formula (C8): 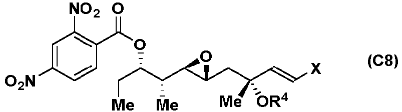 or a salt thereof,
   wherein X is hydrogen or optionally substituted boryl and R⁴ is hydrogen or a silyl protecting group.
[57] A crystal of pladienolide D toluene solvate represented by formula (1a): 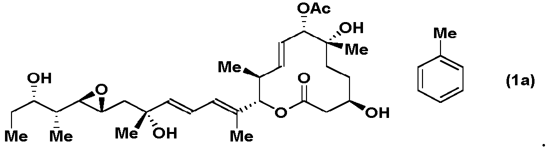
[58] The crystal according to [57], having one or more diffraction peaks at diffraction angles (2θ ± 0.2°) selected from the group consisting of 3.6°, 6.8°, 7.8°, 8.7°, 15.4°, 16.6°, 17.8°, and 18.3° in powder X-ray diffraction.
[59] A crystal of pladienolide D chlorobenzene solvate represented by formula (1b): 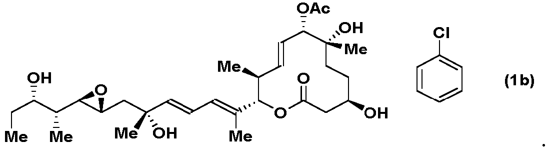
[60] The crystal according to [59], having one or more diffraction peaks at diffraction angles (2θ ± 0.2°) selected from the group consisting of 3.6°, 7.8°, 15.4°, 16.2°, 16.6°, 17.9°, 18.3°, 20.4°, 21.3°, and 21.7° in powder X-ray diffraction.
[61] A crystal of pladienolide D tetrahydrofuran solvate represented by formula (1c): 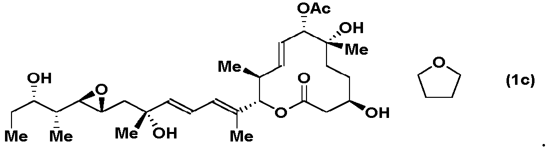
[62] The crystal according to [61], having one or more diffraction peaks at diffraction angles (2θ ± 0.2°) selected from the group consisting of 9.4°, 10.8°, 11.2°, 14.5°, 15.1°, 18.1°, 19.3°, 19.8°, 21.6°, and 22.8° in powder X-ray diffraction.
[63] A crystal of pladienolide D 2-methyltetrahydrofuran solvate represented by formula (1d): 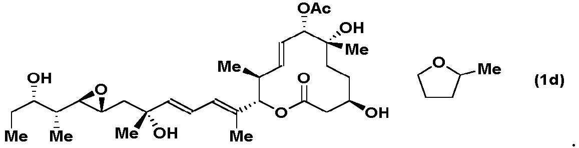
[64] The crystal according to [63], having one or more diffraction peaks at diffraction angles (2θ ± 0.2°) selected from the group consisting of 10.3°, 10.7°, 11.7°, 13.3°, 14.4°, 17.2°, 18.9°, 21.2°, 22.5°, and 23.4° in powder X-ray diffraction.
[65] The crystal according to [63], having a diffraction peak at a diffraction angle (2θ ± 0.2°) of 18.9° in powder X-ray diffraction.
[66] The crystal according to [63], having diffraction peaks at diffraction angles (2θ ± 0.2°) of 10.7°, 14.4°, and 18.9° in powder X-ray diffraction.
[67] The crystal according to [63], having diffraction peaks at diffraction angles (2θ ± 0.2°) of 10.7°, 14.4°, 17.2°, 18.9°, and 21.2° in powder X-ray diffraction.
[68] The crystal according to [63], having diffraction peaks at diffraction angles (2θ ± 0.2°) of 10.3°, 10.7°, 11.7°, 13.3°, 14.4°, 17.2°, 18.9°, 21.2°, 22.5°, and 23.4° in powder X-ray diffraction.
[69] The crystal according to [63], having a powder X-ray pattern substantially identical to the powder X-ray diffraction pattern shown in FIG. 4 in powder X-ray diffraction.
[70] The crystal according to any one of [63] to [69], having one or more peaks at chemical shifts (δ ± 0.5 ppm) selected from the group consisting of 9.1 ppm, 10.0 ppm, 10.5 ppm, 16.2 ppm, 21.4 ppm, 23.9 ppm, 24.2 ppm, 25.4 ppm, 26.8 ppm, 28.0 ppm, 28.5 ppm, 33.3 ppm, 33.7 ppm, 35.9 ppm, 39.9 ppm, 41.1 ppm, 42.7 ppm, 45.7 ppm, 56.0 ppm, 59.3 ppm, 67.9 ppm, 70.4 ppm, 72.6 ppm, 76.5 ppm, 77.7 ppm, 83.0 ppm, 124.0 ppm, 124.4 ppm, 128.0 ppm, 130.2 ppm, 134.4 ppm, 139.1 ppm, 141.0 ppm, 141.3 ppm, 166.9 ppm, and 170.4 ppm in a solid state ¹³C NMR spectrum.
[71] The crystal according to any one of [63] to [69], having a peak at a chemical shift (δ ± 0.5 ppm) of 72.6 ppm in a solid state ¹³C NMR spectrum.
[72] The crystal according to any one of [63] to [69], having peaks at chemical shifts (δ ± 0.5 ppm) of 72.6 ppm, 134.4 ppm, and 170.4 ppm in a solid state ¹³C NMR spectrum.
[73] The crystal according to any one of [63] to [69], having peaks at chemical shifts (δ ± 0.5 ppm) of 21.4 ppm, 70.4 ppm, 72.6 ppm, 134.4 ppm, and 170.4 ppm in a solid state ¹³C NMR spectrum.
[74] The crystal according to any one of [63] to [69], having peaks at chemical shifts (δ ± 0.5 ppm) of 16.2 ppm, 21.4 ppm, 67.9 ppm, 70.4 ppm, 72.6 ppm, 76.5 ppm, 77.7 ppm, 134.4 ppm, 166.9 ppm, and 170.4 ppm in a solid state ¹³C NMR spectrum.
[75] The crystal according to any one of [63] to [69], having peaks at chemical shifts (δ ± 0.5 ppm) of 10.0 ppm, 16.2 ppm, 21.4 ppm, 56.0 ppm, 59.3 ppm, 67.9 ppm, 70.4 ppm, 72.6 ppm, 76.5 ppm, 77.7 ppm, 83.0 ppm, 128.0 ppm, 130.2 ppm, 134.4 ppm, 139.1 ppm, 166.9 ppm, and 170.4 ppm in a solid state ¹³C NMR spectrum.
[76] The crystal according to any one of [63] to [69], having peaks at chemical shifts (δ ± 0.5 ppm) of 9.1 ppm, 10.0 ppm, 10.5 ppm, 16.2 ppm, 21.4 ppm, 23.9 ppm, 24.2 ppm, 25.4 ppm, 26.8 ppm, 28.0 ppm, 28.5 ppm, 33.3 ppm, 33.7 ppm, 35.9 ppm, 39.9 ppm, 41.1 ppm, 42.7 ppm, 45.7 ppm, 56.0 ppm, 59.3 ppm, 67.9 ppm, 70.4 ppm, 72.6 ppm, 76.5 ppm, 77.7 ppm, 83.0 ppm, 124.0 ppm, 124.4 ppm, 128.0 ppm, 130.2 ppm, 134.4 ppm, 139.1 ppm, 141.0 ppm, 141.3 ppm, 166.9 ppm, and 170.4 ppm in a solid state ¹³C NMR spectrum.
[77] The crystal according to any one of [63] to [69], having a solid state ¹³C NMR spectrum substantially identical to the solid state ¹³C NMR spectrum shown in FIG. 5 in a solid state ¹³C NMR spectrum.

### Advantageous Effects of Invention

The present invention provides a method of producing a macrolide-based compound pladienolide D having an antitumor activity, a synthetic intermediate for producing pladienolide D, and a crystal of a solvate of pladienolide D. The production method of the present invention can provide high quality pladienolide D with good reproducibility, and is also applicable to scaled-up production. In the production method of the present invention, since a synthetic intermediate, pladienolide D or a solvate thereof can be obtained as a solid, and therefore purification by crystallization becomes possible, so that high purity pladienolide D or a solvate thereof can be produced by removing impurities.

### Brief Description of Drawings

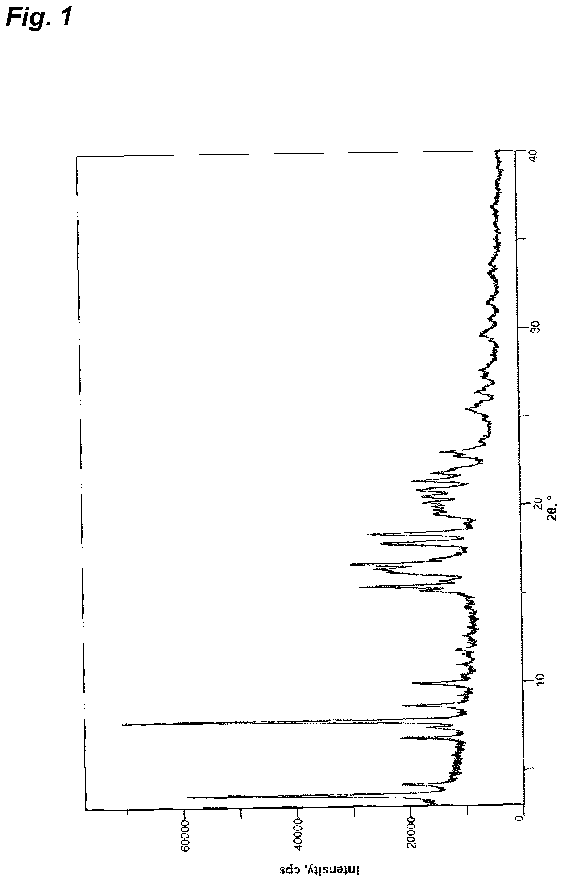

FIG. 1 is a powder X-ray diffraction pattern of a crystal of pladienolide D toluene solvate obtained in Example D-2. The horizontal axis represents the diffraction angle (2θ), and the vertical axis represents the peak intensity.

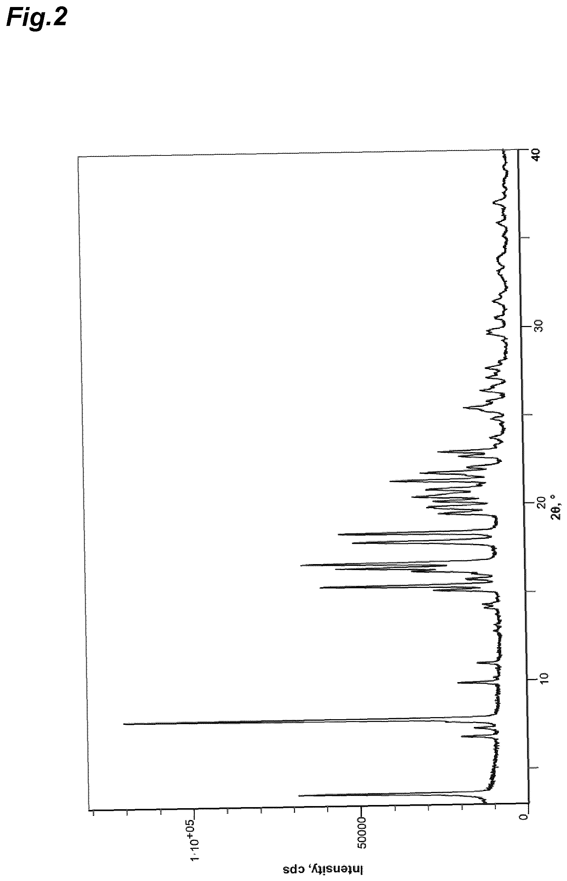

FIG. 2 is a powder X-ray diffraction pattern of a crystal of pladienolide D chlorobenzene solvate obtained in Example D-3. The horizontal axis represents the diffraction angle (2θ), and the vertical axis represents the peak intensity.

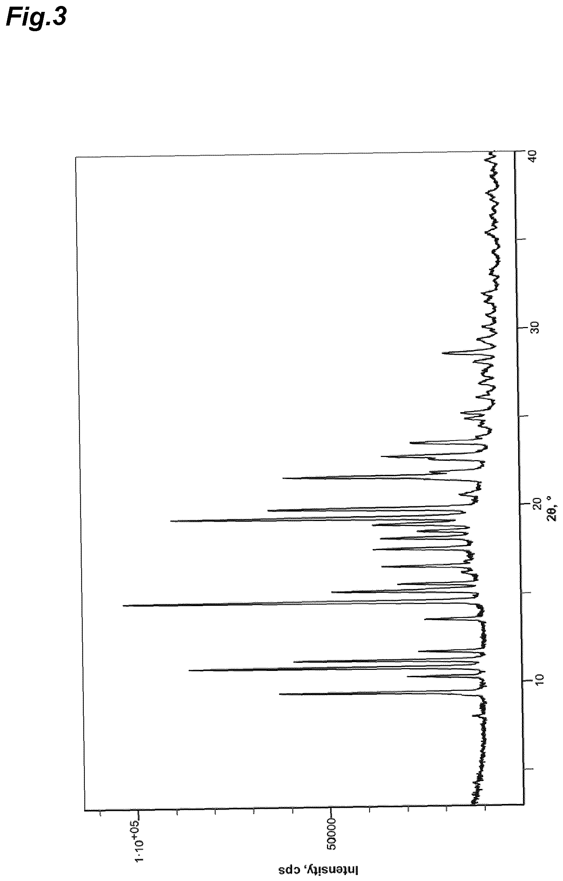

FIG. 3 is a powder X-ray diffraction pattern of a crystal of pladienolide D tetrahydrofuran solvate obtained in Example D-4. The horizontal axis represents the diffraction angle (2θ), and the vertical axis represents the peak intensity.
FIG. 4 is a powder X-ray diffraction pattern of a crystal of pladienolide D 2-methyltetrahydrofuran solvate obtained in Example D-6. The horizontal axis represents the diffraction angle (2θ), and the vertical axis represents the peak intensity.
FIG. 5 is a solid state ¹³C NMR spectrum of a crystal of pladienolide D 2-methyltetrahydrofuran solvate obtained in Example D-6. The horizontal axis represents the chemical shift (δ), and the vertical axis represents the peak intensity.

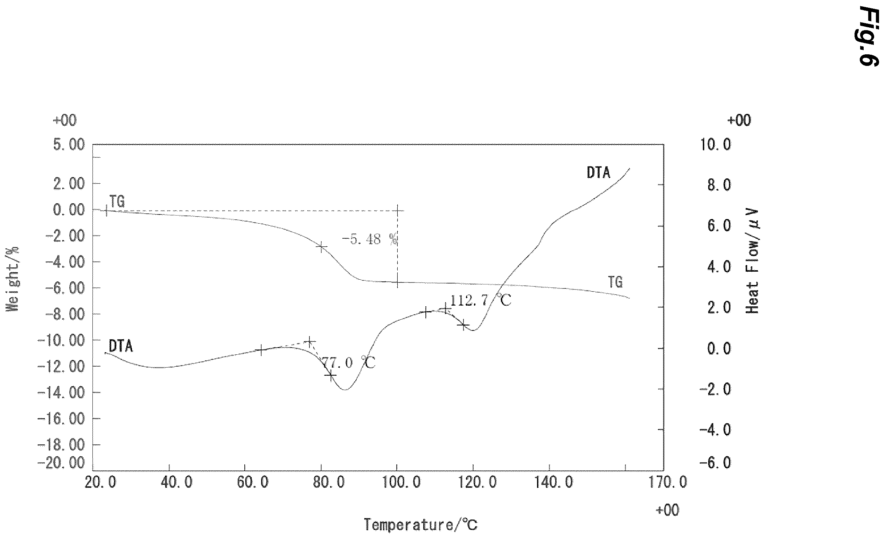

FIG. 6 is a thermal analysis TG-DTA chart of a crystal of pladienolide D toluene solvate obtained in Example D-2. The horizontal axis represents the temperature, the left vertical axis represents the weight change in TG, and the right vertical axis represents the heat flow in DTA.

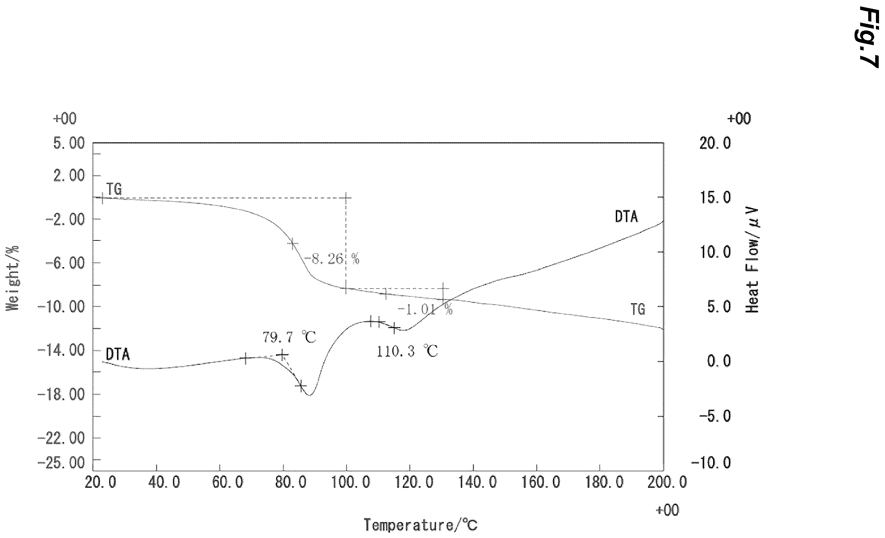

FIG. 7 is a thermal analysis TG-DTA chart of a crystal of pladienolide D chlorobenzene solvate obtained in Example D-3. The horizontal axis represents the temperature, the left vertical axis represents the weight change in TG, and the right vertical axis represents the heat flow in DTA.

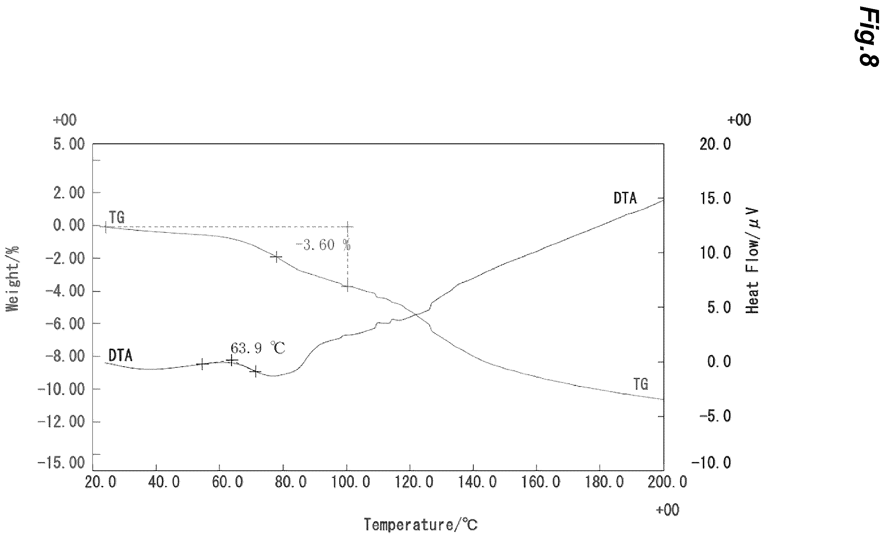

FIG. 8 is a thermal analysis TG-DTA chart of a crystal of pladienolide D tetrahydrofuran solvate obtained in Example D-4. The horizontal axis represents the temperature, the left vertical axis represents the weight change in TG, and the right vertical axis represents the heat flow in DTA.

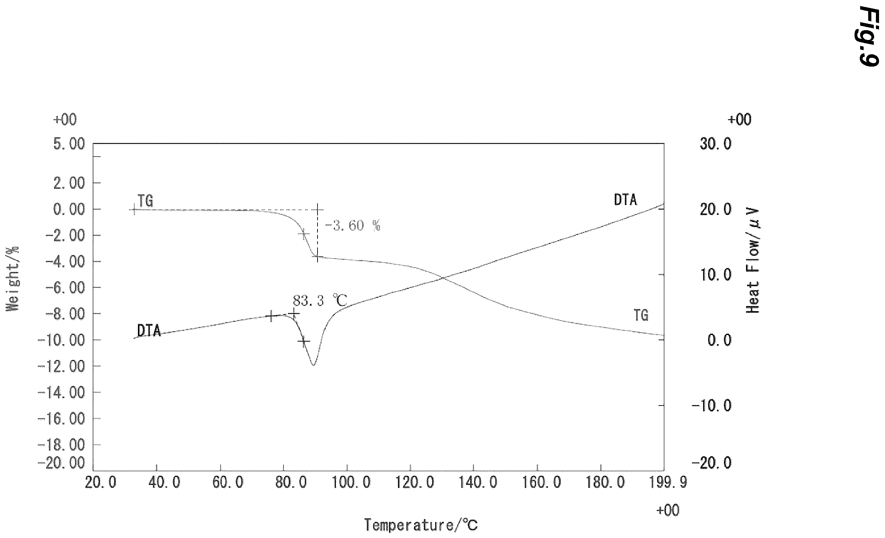

FIG. 9 is a thermal analysis TG-DTA chart of a crystal of pladienolide D 2-methyltetrahydrofuran solvate obtained in Example D-6. The horizontal axis represents the temperature, the left vertical axis represents the weight change in TG, and the right vertical axis represents the heat flow in DTA.

### Description of Embodiments

Hereinafter, meanings of symbols, terms, and the like described in the present description will be described, and the present invention will be described in detail.

In the present description, the structural formula of the compound is not limited to the description of the formula for convenience, and may form a salt or may be a solvate. Further, a crystal polymorph may exist, but the crystal form is not limited in the same manner, and may be any single crystal form or a mixture of crystal forms, and may partially contain an amorphous form. These are all encompassed by the present invention.

In the present description, examples of the "salt" of a compound include alkali metal salts (such as a lithium salt, a sodium salt, and a potassium salt) and alkaline earth metal salts (such as a magnesium salt and a calcium salt).

In the present description, the "solvate" refers to a molecular species formed by combining a compound or a salt thereof and a solvent molecule, and includes a hydrate. Examples of the solvent that forms the solvate include water, hexane, heptane, methyl acetate, ethyl acetate, isopropyl acetate, 1,4-dioxane, dichloromethane, dichloroethane, chloroform, carbon tetrachloride, diethyl ether, diisopropyl ether, cyclopentyl methyl ether, tetrahydrofuran, 2-methyltetrahydrofuran, 1,2-dimethoxyethane, methanol, ethanol, 1-propanol, 2-propanol, 1-butanol, acetonitrile, acetone, dimethyl sulfoxide, N,N-dimethylformamide, N,N-dimethylacetamide, benzene, toluene, anisole, cumene, chlorobenzene, and trifluorobenzene. The number of solvent molecules with respect to a compound or a salt thereof is not particularly limited, and may be, for example, one molecule or two molecules.

The present invention also encompasses isotopically labeled compounds of the compounds described herein and production methods using the same. The isotopically labeled compounds are identical to the compounds described herein except that one or more atoms are replaced with an atom having an atomic mass or mass number different from the atomic mass or mass number normally found in nature. Isotopes that can be incorporated in the compound according to the present invention are isotopes of hydrogen, carbon, nitrogen, oxygen, and fluorine constituting the compound, and include ²H, ³H, ¹¹C, ¹³C, ¹⁴C, ¹³N, ¹⁵O, ¹⁸F, and the like.

In the present description, the "optionally substituted boryl" includes boronic acid, a boronate ester, trifluoroborate, triolborate, and the like. Trifluoroborate and triolborate can be stabilized with a counter cation. Examples of the optionally substituted boryl include groups having a structure below.

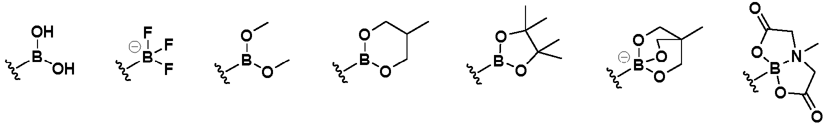

In the chemical formulae in the present description, preferred examples of the "optionally substituted boryl" in X include boronic acid, pinacol boronate ester, and trifluoroborate, and more preferred examples include pinacol boronate ester.

In the present description, the "optionally substituted stannyl" means a substituted or unsubstituted stannyl group including tin(IV). Examples of the optionally substituted stannyl include tri(n-butyl)stannyl.

In the present description, the "optionally substituted silyl" means a substituted or unsubstituted silyl group. Examples of the optionally substituted silyl include hydroxy(dimethyl)silyl, benzyl(dimethyl)silyl, and trimethoxysilyl.

In the present description, the "optionally substituted benzoyl" means a benzoyl group which may have a substituent on a benzene ring. The optionally substituted benzoyl works as a protecting group for a hydroxy group in a synthesis step, and can be removed by hydrolysis using a base. Examples of the optionally substituted benzoyl include substituents having a structure below.

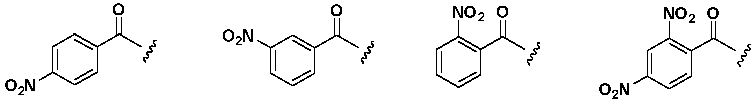

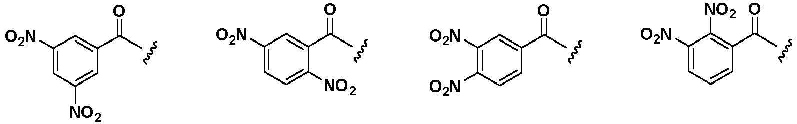

In the chemical formulae in the present description, preferred examples of the "optionally substituted benzoyl" in R⁵ include 2,4-dinitrobenzoyl and 3,5-dinitrobenzoyl. One aspect of the optionally substituted benzoyl may be 2,4-dinitrobenzoyl, and another aspect may be 3,5-dinitrobenzoyl. When the optionally substituted benzoyl is 3,5-dinitrobenzoyl, a synthetic intermediate for producing pladienolide D is easily obtained as a solid, so that the intermediate and pladienolide D can be easily obtained with high purity by recrystallization, crystallization, or the like.

In the present description, the "silyl protecting group" means a protecting group of a hydroxy group in which silicon is directly bonded to a hydroxy group, and may be any group generally used in organic synthesis. For example, the silyl protecting group for a hydroxy group described in Greene's Protective Groups In Organic Synthesis (4th Edition, Wuts Peter G. M. et al.) can be used. The silyl protecting group is, for example, silyl represented by SiR⁶R⁷R⁸, and R⁶, R⁷, and R⁸ are each independently selected from the group consisting of methyl, ethyl, isopropyl, tert-butyl, and phenyl. Examples of the silyl protecting group include trimethylsilyl (TMS), triethylsilyl (TES), diethyl(isopropyl)silyl (DEIPS), triisopropylsilyl (TIPS), tert-butyl(dimethyl)silyl (TBS), and tert-butyl(diphenyl)silyl (TBDPS).

In the chemical formulae in the present description, the "silyl protecting group" in R¹ may preferably be TES, DEIPS, TBS, TIPS, or TBDPS, and may preferably be TBS, TIPS, or TBDPS. The silyl protecting group in R¹ may be TES in one aspect, TBS in another aspect, TIPS in another aspect, and TBDPS in still another aspect.

In the chemical formulae in the present description, the "silyl protecting group" in R² may preferably be TMS, TES, DEIPS, TBS, or TIPS, and may more preferably be TES, TBS, or TIPS. The silyl protecting group in R² may be TMS in one aspect, TES in another aspect, TBS in another aspect, and TIPS in still another aspect.

In the chemical formulae in the present description, the "silyl protecting group" in R⁴ may preferably be TES, TBS, TIPS or TBDPS, and may more preferably be TES, TBS, or TIPS. The silyl protecting group in R⁴ may be TES in one aspect, TBS in another aspect, TIPS in another aspect, and TBDPS in still another aspect.

Conditions and reagents that can be used for removing the protecting group in the method of the present disclosure vary depending on the type of the protecting group, and for example, conditions and reagents for removing various protecting groups described in Greene's Protective Groups In Organic Synthesis (4th Edition, Wuts Peter G. M. et al.) can be used. As the conditions and reagents used for removing the protecting group, multiple conditions and reagents can be used simultaneously or in stages according to various protecting groups.

When the protecting group to be removed is a "silyl protecting group", the protecting group can be removed using, for example, an acid such as hydrochloric acid, bromic acid, trifluoroacetic acid (TFA), p-toluenesulfonic acid, pyridinium p-toluenesulfonate (PPTS), or camphor-sulfonic acid (CSA), or using a fluoride ion, and as a fluoride ion source, for example, tetrabutylammonium fluoride (TBAF), pyridine hydrofluoride, triethylamine trihydrofluoride, tris(dimethylamino)sulfonium difluorotrimethylsilicate (TASF), ammonium fluoride, potassium fluoride, cesium fluoride, or the like can be used. Preferred examples of the acid include hydrochloric acid, p-toluenesulfonic acid, and pyridinium p-toluenesulfonate (PPTS). The fluoride ion source may preferably be tetrabutylammonium fluoride (TBAF).

When the protecting group to be removed is "optionally substituted benzoyl", the protecting group can be removed using, for example, a base such as lithium hydroxide, sodium hydroxide, potassium hydroxide, sodium carbonate, potassium carbonate, or cesium carbonate, and can be removed by hydrolysis using the base. The base may preferably be lithium hydroxide or sodium hydroxide.

When the protecting group to be removed is "dinitrobenzoyl" and one or more selected from the group consisting of R¹, R², and R⁴ in the chemical formula are silyl protecting groups, dinitrobenzoyl can be removed simultaneously with the above-mentioned one or more silyl protecting groups using tetrabutylammonium fluoride in addition to the removal using the base. Examples of the dinitrobenzoyl include 2,4-dinitrobenzoyl, 3,5-dinitrobenzoyl, and 2,5-dinitrobenzoyl.

In the case of removing the silyl protecting group and the optionally substituted benzoyl, in the step of removing the protecting group, removal using an acid or a fluoride ion used for removing the silyl protecting group and removal using a base can be performed simultaneously or in stages. One aspect of multiple removals that can be performed in stages may be removal using an acid and removal using a base. One aspect of multiple removals that can be performed simultaneously or in stages may be removal using a fluoride ion and removal using a base. Another specific aspect may be removal using tetrabutylammonium fluoride and removal using lithium hydroxide, and still another specific aspect may be removal using tetrabutylammonium fluoride and removal using sodium hydroxide.

In the method of the present disclosure, the acetylation step can be performed by any method capable of acetylating a hydroxy group, and examples of the reagent (hereinafter also referred to as "acetylating reagent") that can be used for acetylation include a reagent that enables acetylation alone, a reagent that can cause a reaction with a base, and a reagent that enables acetylation by condensation. Examples of the acetylating reagent include acetic acid, acetic anhydride, acetyl chloride, and acetyl bromide, and the acetylating reagent may preferably be acetic anhydride or acetyl chloride, and the acetylating reagent may preferably be acetic anhydride.

Examples of the "base" that can be used together with the "acetylating reagent" include triethylamine, diisopropyl (ethyl) amine, pyridine, and 4-(dimethylamino)pyridine.

In the present description, the "metal catalyst" means a transition metal catalyst used in a coupling reaction such as a Suzuki coupling reaction, a Heck reaction, a Stille coupling reaction, or a Hiyama coupling reaction, and can also be used together with a ligand. Examples of the "metal catalyst" include a palladium catalyst, a nickel catalyst, an iron catalyst, a copper catalyst, a rhodium catalyst, an iridium catalyst, and a gold catalyst. The metal catalyst may be any metal catalyst that is generally used in organic synthesis, and a commercially available metal catalyst can be used.

In the present description, examples of the "palladium catalyst" include tetrakis(triphenylphosphine)palladium(0), tris(dibenzylideneacetone)dipalladium(0), palladium(II) acetate, palladium(II) chloride, bis(triphenylphosphine)palladium(II) dichloride, dichlorobis(benzonitrile)palladium(II), (1,1'-bis(diphenylphosphino)ferrocene)dichloropalladium(II), and [1,3-bis(2,6-diisopropylphenyl)imidazol-2-ylidene](3-chloropyridyl)palladium(II) dichloride.

Examples of the "ligand" that can be used together with the "metal catalyst" include XPhos (2-dicyclohexylphosphino-2',4',6'-triisopropylbiphenyl), Amphos (di-tert-butyl(4-dimethylaminophenyl)phosphine), SPhos (2-dicyclohexylphosphino-2',6'-dimethoxybiphenyl), Xantphos (4,5'-bis(diphenylphosphino)-9,9'-dimethylxanthene), BrettPhos (2-(dicyclohexylphosphino)-3,6-dimethoxy-2',4',6'-triisopropyl-1,1'-biphenyl), and RuPhos (2-dicyclohexylphosphino-2',6'-diisopropoxybiphenyl). A catalyst in which such a ligand is bound to a transition metal, for example, palladium, is commercially available and can be used as the "metal catalyst".

The coupling reaction using the "metal catalyst" may be performed together with the "base". Examples of the "base" used in the coupling reaction include sodium carbonate, potassium carbonate, cesium carbonate, silver(I) carbonate, trisodium phosphate, tripotassium phosphate, sodium acetate, potassium acetate, silver(I) acetate, silver(I) oxide, triethylamine, diisopropyl (ethyl) amine, sodium hydroxide, tert-butoxy sodium, and tert-butoxy potassium.

In the present description, the "ruthenium catalyst" means a ruthenium catalyst for performing a cross-metathesis reaction or a ring-closing metathesis reaction. Examples of the ruthenium catalyst include Grubbs-II ((1,3-bis(2,4,6-trimethylphenyl)-2-imidazolidinylidene)dichloro(phenylmethylene)(tricyclohexylphosphine)ruthe nium) and Hoveyda-Grubbs-II (1,3-bis-(2,4,6-trimethylphenyl)-2-imidazolidinylidene)dichloro(o-isopropoxyphenylmethylene)ruthenium. One aspect of the ruthenium catalyst may be Grubbs-II ((1,3-bis(2,4,6-trimethylphenyl)-2-imidazolidinylidene)dichloro(phenylmethylene)(tricyclohexylphosphine)ruthe nium) and another aspect may be Hoveyda-Grubbs-II (1,3-bis-(2,4,6-trimethylphenyl)-2-imidazolidinylidene)dichloro(o-isopropoxyphenylmethylene)ruthenium.

Step 1-1, step 2-1, step 3-1, and step 4-1 are steps of performing a coupling reaction under a metal catalyst. The metal catalyst described above can be used, and the amount of the metal catalyst is not particularly limited, and for example, 0.01 to 0.2 equivalents with respect to the compound represented by formula (B1) or formula (B2) as the starting material can be used. In addition, the reaction may be performed together with a ligand, and as the ligand, the above-described ligand can be used, and the amount of the ligand is not particularly limited, and for example, 0.01 to 0.2 equivalents with respect to the compound represented by formula (B1) or formula (B2) as the starting material can be used. Furthermore, the reaction may be performed together with a base, and the above-described base can be used as the base, and the amount of the base is not particularly limited, and for example, 1 to 5 equivalents with respect to the compound represented by formula (B1) or formula (B2) as the starting material can be used.

The solvent in the coupling reaction is not particularly limited as long as it dissolves the starting material and does not inhibit the reaction, and for example, water, acetonitrile, tetrahydrofuran, 1,2-dimethoxyethane, 1,4-dioxane, N,N-dimethylformamide, N,N-dimethylacetamide, toluene, or the like, or a mixed solvent thereof can be used. The reaction temperature usually varies depending on the starting material, the solvent, and other reagents used in the reaction, and ranges from room temperature to the boiling point of the solvent, preferably from room temperature to 100°C. When the reaction temperature exceeds the boiling point of the solvent, heating under reflux may be performed. The reaction time may be set to a time until which the starting material disappears by stirring, and may be, for example, 1 hour to 24 hours.

In the compounds represented by formula (A1) and formula (A2), when X is hydrogen, the metal catalyst may preferably be palladium(II) acetate. The base may preferably be silver(I) carbonate or silver(I) acetate, and may more preferably be silver(I) carbonate. In one aspect, the metal catalyst is palladium(II) acetate and the base is silver(I) carbonate, and in another aspect, the metal catalyst is palladium(II) acetate and the base is silver(I) acetate. The reaction temperature is preferably 40°C to 100°C, and more preferably 60°C to 80°C.

In the compounds represented by formulae (A1) and (A2), when X is optionally substituted boryl, the metal catalyst may preferably be (1,1'-bis(diphenylphosphino)ferrocene)dichloropalladium(II) or tris(dibenzylideneacetone)dipalladium(0), and may more preferably be (1,1'-bis(diphenylphosphino)ferrocene)dichloropalladium(H). The base may be silver(I) oxide. In one aspect, the metal catalyst is (1,1'-bis(diphenylphosphino)ferrocene)dichloropalladium(II) and the base is silver(I) oxide, and in another aspect, the metal catalyst is tris(dibenzylideneacetone)dipalladium(0) and the base is silver(I) oxide. The reaction temperature is preferably room temperature to 60°C, and more preferably room temperature.

Step 1-2, step 2-2, step 3-3, and step 4-3 are steps of removing a protecting group, and are steps of removing a silyl protecting group and an optionally substituted benzoyl group. The above-described conditions and reagents can be used for removal of the protecting group. The protecting group may be removed simultaneously or in stages depending on the type of the protecting group of the starting material. When suitable conditions and reagents for removal are different between the optionally substituted benzoyl and the silyl protecting group, this step can also be performed in stages, and the silyl protecting group may be removed after the optionally substituted benzoyl is removed, and the optionally substituted benzoyl may be removed after the silyl protecting group is removed. When the protecting group is removed in stages, after the removal of one of the optionally substituted benzoyl and the silyl protecting group is completed, the reaction can be performed by adding an acid, a base, a fluoride ion source, or the like corresponding to the conditions for removing the other protecting group in the reaction system, and after the removal of the one protecting group is completed, the reaction is stopped to isolate a crude product containing a synthetic intermediate, and purification is performed as necessary, and then the other protecting group may be removed. When the optionally substituted benzoyl and the silyl protecting group can be simultaneously removed, a desired product can be obtained by subjecting them to one reaction condition.

In the case of removing the silyl protecting group, the above-described acid or fluoride ion source can be used for the removal of the protecting group. In the case of using an acid, for example, 0.05 to 5 equivalents with respect to the compound represented by formula (C1) or formula (C2) as the starting material can be used although it varies depending on the number of silyl protecting groups. In the case of using a fluoride ion source, for example, 1 to 10 equivalents with respect to the compound represented by formula (C1) or formula (C2) as the starting material can be used although it varies depending on the number of silyl protecting groups. The acid may preferably be hydrochloric acid, p-toluenesulfonic acid, or pyridinium p-toluenesulfonate (PPTS), and the fluoride ion source may preferably be tetrabutylammonium fluoride.

The solvent in the removal of the silyl protecting group is not particularly limited as long as it dissolves the starting material and does not inhibit the reaction, and for example, water, methanol, ethanol, acetonitrile, methylene chloride, chloroform, tetrahydrofuran, 1,2-dimethoxyethane, 1,4-dioxane, N,N-dimethylformamide, N,N-dimethylacetamide, toluene, or the like, or a mixed solvent thereof can be used. The reaction temperature usually varies depending on the starting material, the solvent, and other conditions and reagents used in the reaction, and ranges from room temperature to the boiling point of the solvent, preferably from room temperature to 40°C, and more preferably room temperature. The reaction time may be set to a time until which the starting material disappears by stirring, and may be, for example, 1 hour to 24 hours.

In the case of removing the optionally substituted benzoyl, the base mentioned in the above-described conditions for removing the optionally substituted benzoyl can be used for the removal of the protecting group, and the amount of the base is not particularly limited, and for example, 1 to 5 equivalents with respect to the compound represented by formula (C1) or formula (C2) as the starting material can be used. The base may preferably be lithium hydroxide or sodium hydroxide.

The solvent in the removal of the optionally substituted benzoyl is not particularly limited as long as it dissolves the starting material and does not inhibit the reaction, and for example, water, methanol, ethanol, acetonitrile, methylene chloride, chloroform, tetrahydrofuran, 1,2-dimethoxyethane, 1,4-dioxane, N,N-dimethylformamide, N,N-dimethylacetamide, toluene, or the like, or a mixed solvent thereof can be used. The reaction temperature usually varies depending on the starting material, the solvent, and other conditions and reagents used in the reaction, and ranges from room temperature to the boiling point of the solvent, preferably from room temperature to 40°C, and more preferably room temperature. The reaction time may be set to a time until which the starting material disappears by stirring, and may be, for example, 1 hour to 24 hours.

When the silyl protecting group and the optionally substituted benzoyl are removed in stages, in one aspect of the step of removing the protecting group, removal is performed using an acid and then removal is performed using a base, and in another aspect, removal is performed using a fluoride ion and then removal is performed using a base, and in another aspect, removal is performed using a base and then removal is performed using an acid, and in still another aspect, removal is performed using a base and then removal is performed using a fluoride ion. In another aspect, removal is performed using tetrabutylammonium fluoride and then removal is performed using lithium hydroxide, and in still another aspect, removal is performed using lithium hydroxide and then removal is performed using tetrabutylammonium fluoride. In another aspect, removal is performed using tetrabutylammonium fluoride and then removal is performed using sodium hydroxide, and in still another aspect, removal is performed using sodium hydroxide and then removal is performed using tetrabutylammonium fluoride.

Step 3-2 and step 4-2 are steps of acetylating a hydroxy group. In the acetylation, the above-described acetylating reagent can be used, and the amount of the acetylating reagent is not particularly limited, and for example, 1 to 5 equivalents with respect to the compound represented by formula (C3) or formula (C4) as the starting material can be used. The acetylating reagent may preferably be acetic anhydride. The acetylating reagent can be used together with a base. As the base, a base that can be used together with the above-described acetylating reagent can be used, and the amount of the base is not particularly limited, and for example, 1 to 5 equivalents with respect to the compound represented by formula (C3) or formula (C4) as the starting material can be used. The base may preferably be triethylamine, and triethylamine and 4-(dimethylamino)pyridine can also be used simultaneously.

The solvent in the acetylation is not particularly limited as long as it dissolves the starting material and does not inhibit the reaction, and for example, acetonitrile, methylene chloride, chloroform, tetrahydrofuran, 1,2-dimethoxyethane, 1,4-dioxane, N,N-dimethylformamide, N,N-dimethylacetamide, toluene, pyridine, or the like, or a mixed solvent thereof can be used. The reaction temperature usually varies depending on the starting material, the solvent, and other conditions and reagents used in the reaction, and ranges from room temperature to the boiling point of the solvent, preferably from room temperature to 40°C, and more preferably room temperature. The reaction time may be set to a time until which the starting material disappears by stirring, and may be, for example, 1 hour to 24 hours.

Step 5-1 is a step of performing ester formation. The ester formation can be performed by a method generally used in organic synthesis, and for example, a carboxylic acid chloride as a synthetic intermediate may be prepared with a chlorinating agent, and the resulting carboxylic acid chloride may be used, or a commercially available condensing agent may be used. Examples of the chlorinating agent or the condensing agent include thionyl chloride, oxalyl chloride, 2,4,6-trichlorobenzoyl chloride, 2-methyl-6-nitrobenzoic anhydride (MNBA), 1-[3-(dimethylamino)propyl]-3-ethylcarbodiimide (WSC), N,N-dicyclohexylcarbodiimide (DCC), N,N'-carbonyldiimidazole (CDI), 1H-benzotriazol-1-yloxytris(dimethylamino)phosphonium hexafluorophosphate (BOP), and O-(benzotriazol-1-yl)-N,N,N,N'-tetramethyluronium hexafluorophosphate (HBTU). The chlorinating agent or the condensing agent may preferably be 2,4,6-trichlorobenzoyl chloride. The chlorinating agent or the condensing agent can be used in an amount of, for example, 1 to 3 equivalents with respect to the compound represented by formula (D2) as the starting material. For ester formation, a base may be further used in addition to the chlorinating agent or the condensing agent, and the amount of the base is not particularly limited, and for example, 1 to 5 equivalents with respect to the compound represented by formula (D2) as the starting material can be used. The base may preferably be triethylamine, diisopropyl (ethyl) amine, pyridine, or 4-(dimethylamino)pyridine.

The solvent in the ester formation is not particularly limited as long as it dissolves the starting material and does not inhibit the reaction, and for example, acetonitrile, methylene chloride, chloroform, tetrahydrofuran, 1,2-dimethoxyethane, 1,4-dioxane, N,N-dimethylformamide, N,N-dimethylacetamide, toluene, or the like, or a mixed solvent thereof can be used. The reaction temperature usually varies depending on the starting material, the solvent, and other conditions and reagents used in the reaction, and ranges from room temperature to the boiling point of the solvent, preferably from room temperature to 40°C, and more preferably room temperature. When the ester formation is performed in stages, the reaction temperature is preferably 60°C to 100°C in the stage in which the compound represented by formula (D2) as the starting material forms an acid anhydride with a condensing agent, and heating and refluxing may be performed when the temperature exceeds the boiling point of the solvent. The reaction time may be set to a time until which the starting material disappears by stirring, and may be, for example, 1 hour to 24 hours.

Step 5-2 is a step of removing a para-methoxybenzyl group. The removal can be performed according to the method described in Greene's Protective Groups In Organic Synthesis (4th Edition, Wuts Peter G. M. et al.), but for example, the removal can be performed using 2,3-dichloro-5,6-dicyano-p-benzoquinone. The amount of 2,3-dichloro-5,6-dicyano-p-benzoquinone is not particularly limited, and for example, 1 to 5 equivalents with respect to the compound represented by formula (D3) as the starting material can be used.

The solvent in the removal of a para-methoxybenzyl group is not particularly limited as long as it dissolves the starting material and does not inhibit the reaction, and for example, acetonitrile, methylene chloride, chloroform, tetrahydrofuran, 1,2-dimethoxyethane, 1,4-dioxane, N,N-dimethylformamide, N,N-dimethylacetamide, toluene, or the like, or a mixed solvent thereof can be used. The reaction temperature usually varies depending on the starting material, the solvent, and other conditions and reagents used in the reaction, and ranges from room temperature to the boiling point of the solvent, preferably from room temperature to 40°C, and more preferably room temperature. The reaction time may be set to a time until which the starting material disappears by stirring, and may be, for example, 1 hour to 24 hours.

Step 5-3 is a step of performing a ring-closing metathesis reaction using a ruthenium catalyst. The above-described ruthenium catalyst can be used, and the amount of the ruthenium catalyst is not particularly limited, and for example, 0.01 to 0.1 equivalents with respect to the compound represented by formula (D4) as the starting material can be used. The ruthenium catalyst may preferably be Grubbs-II (1,3-bis-(2,4,6-trimethylphenyl)-2-imidazolidinylidene)dichloro(o-isopropoxyphenylmethylene)ruthenium.

The solvent in the ring-closing metathesis reaction using a ruthenium catalyst is not particularly limited as long as it dissolves the starting material and does not inhibit the reaction, and for example, acetonitrile, methylene chloride, chloroform, tetrahydrofuran, 1,2-dimethoxyethane, 1,4-dioxane, toluene, or the like, or a mixed solvent thereof can be used. The reaction temperature usually varies depending on the starting material, the solvent, and other reagents used in the reaction, and ranges from room temperature to the boiling point of the solvent, preferably from 80°C to 120°C. When the reaction temperature exceeds the boiling point of the solvent, heating under reflux may be performed. The reaction time may be set to a time until which the starting material disappears by stirring, and may be, for example, 1 hour to 24 hours.

In the compound represented by formula (C5), in one aspect, R¹ is hydrogen, R² is hydrogen, and R⁴ is hydrogen, and in another aspect, R¹ is a silyl protecting group, R² is hydrogen, and R⁴ is hydrogen, and in still another aspect, R¹ is a silyl protecting group, R² is a silyl protecting group, and R⁴ is hydrogen. As the silyl protecting group in R¹, R², and R⁴, the above-mentioned protecting group can be selected, and examples thereof include TMS, TES, TBS, TIPS, and TBDPS. The compound represented by formula (C5) is easily crystallized by having optionally substituted benzoyl, and thus can be purified by crystallization, recrystallization, or the like.

In the compound represented by formula (C6), in one aspect, R¹ is hydrogen, R² is hydrogen, and R⁴ is hydrogen, and in another aspect, R¹ is a silyl protecting group, R² is hydrogen, and R⁴ is hydrogen, and in still another aspect, R¹ is a silyl protecting group, R² is a silyl protecting group, and R⁴ is hydrogen. As the silyl protecting group in R¹, R², and R⁴, the above-mentioned protecting group can be selected, and examples thereof include TMS, TES, TBS, TIPS, and TBDPS. The compound represented by formula (C6) is easily crystallized by having optionally substituted benzoyl, and thus can be purified by crystallization, recrystallization, or the like.

In the compound represented by formula (C7), in one aspect, X is hydrogen and R⁴ is hydrogen, and in another aspect, X is hydrogen and R⁴ is a silyl protecting group, and in another aspect, X is optionally substituted boryl and R⁴ is hydrogen, and in still another aspect, X is optionally substituted boryl and R⁴ is a silyl protecting group. The optionally substituted boryl in X may be the optionally substituted boryl described above. As the silyl protecting group in R⁴, the above-mentioned protecting group can be selected, and examples thereof include TMS, TES, DEIPS, TBS, TIPS, and TBDPS. The compound represented by formula (C7) is easily crystallized by having optionally substituted benzoyl, and thus can be purified by crystallization, recrystallization, or the like.

In the compound represented by formula (C8), in one aspect, X is hydrogen and R⁴ is hydrogen, and in another aspect, X is hydrogen and R⁴ is a silyl protecting group, and in another aspect, X is optionally substituted boryl and R⁴ is hydrogen, and in still another aspect, X is optionally substituted boryl and R⁴ is a silyl protecting group. The optionally substituted boryl in X may be the optionally substituted boryl described above. As the silyl protecting group in R⁴, the above-mentioned protecting group can be selected, and examples thereof include TMS, TES, DEIPS, TBS, TIPS, and TBDPS. The compound represented by formula (C8) is easily crystallized by having optionally substituted benzoyl, and thus can be purified by crystallization, recrystallization, or the like.

Step 1-3, step 2-3, and step 4-4 are steps of converting pladienolide D to a solvate. The solvate obtained may be a crystal. For example, a crystal of a solvate of pladienolide D can be produced by dissolving pladienolide D in a solvent, heating the solution, and then cooling the solution under stirring to cause crystallization. Further, for example, a crystal of a solvate of pladienolide D can also be produced by dissolving pladienolide D in a solvent, adding a poor solvent, to cause crystallization, or by dissolving pladienolide D in a solvent, distilling off the solvent by distillation under reduced pressure to cause crystallization. Further, a crystal of a solvate of pladienolide D can also be produced by dissolving pladienolide D in a solvent, adding a seed crystal for a desired crystal of a solvate of pladienolide D to cause crystallization. When a seed crystal is used, the seed crystal may be added at room temperature or under heating.

In the present description, the solvate of pladienolide D represented by formula (1) or a salt thereof is not particularly limited as long as it is a solvate formed between pladienolide D or a salt thereof and a solvent capable of forming a solvate therewith. The solvate of pladienolide D represented by formula (1) or a salt thereof may be, in one aspect, pladienolide D toluene solvate, or in another aspect, pladienolide D chlorobenzene solvate, or in another aspect, pladienolide D tetrahydrofuran solvate, or in still another aspect, pladienolide D 2-methyltetrahydrofuran solvate.

In the present description, the diffraction angle and the diffraction pattern of powder X-ray diffraction mean a diffraction angle and a diffraction pattern obtained by irradiation with a copper Kα ray. In addition, in the present description, the chemical shift of a solid state ¹³C NMR spectrum means a chemical shift with glycine as an external standard (176.03 ppm).

In the present description, pladienolide D toluene solvate can exist as a crystal. The crystal of pladienolide D toluene solvate may be, in one aspect, a crystal having one or more diffraction peaks at diffraction angles (2θ ± 0.2°) selected from the group consisting of 3.6°, 6.8°, 7.8°, 8.7°, 15.4°, 16.6°, 17.8°, and 18.3° in powder X-ray diffraction, or in another aspect, a crystal having a diffraction peak at a diffraction angle (2θ ± 0.2°) of 7.8° in powder X-ray diffraction, or in another aspect, a crystal having diffraction peaks at diffraction angles (2θ ± 0.2°) of 3.6°, 7.8°, and 16.6° in powder X-ray diffraction, or in another aspect, a crystal having diffraction peaks at diffraction angles (2θ ± 0.2°) of 3.6°, 7.8°, 15.4°, 16.6°, and 18.3° in powder X-ray diffraction, or in still another aspect, a crystal having diffraction peaks at diffraction angles (2θ ± 0.2°) of 3.6 °, 6.8°, 7.8°, 8.7°, 15.4°, 16.6°, 17.8°, and 18.3° in powder X-ray diffraction.

In the present description, pladienolide D chlorobenzene solvate can exist as a crystal. The crystal of pladienolide D chlorobenzene solvate may be, in one aspect, a crystal having one or more diffraction peaks at diffraction angles (2θ ± 0.2°) selected from the group consisting of 3.6°, 7.8°, 15.4°, 16.2°, 16.6°, 17.9°, 18.3°, 20.4°, 21.3°, and 21.7° in powder X-ray diffraction, or in another aspect, a crystal having a diffraction peak at a diffraction angle (2θ ± 0.2°) of 7.8° in powder X-ray diffraction, or in another aspect, a crystal having diffraction peaks at diffraction angles (2θ ± 0.2°) of 3.6°, 7.8°, and 16.6° in powder X-ray diffraction, or in another aspect, a crystal having diffraction peaks at diffraction angles (2θ ± 0.2°) of 3.6°, 7.8°, 15.4°, 16.6°, and 18.3° in powder X-ray diffraction, or in still another aspect, a crystal having diffraction peaks at diffraction angles (2θ ± 0.2°) of 3.6°, 7.8°, 15.4°, 16.2°, 16.6°, 17.9°, 18.3°, 20.4°, 21.3°, and 21.7° in powder X-ray diffraction.

In the present description, pladienolide D tetrahydrofuran solvate can exist as a crystal. The crystal of pladienolide D tetrahydrofuran solvate may be, in one aspect, a crystal having one or more diffraction peaks at diffraction angles (2θ ± 0.2°) selected from the group consisting of 9.4°, 10.8°, 11.2°, 14.5°, 15.1°, 18.1°, 19.3°, 19.8°, 21.6°, and 22.8° in powder X-ray diffraction, or in another aspect, a crystal having a diffraction peak at a diffraction angle (2θ ± 0.2°) of 14.5° in powder X-ray diffraction, or in another aspect, a crystal having diffraction peaks at diffraction angles (2θ ± 0.2°) of 10.8°, 14.5°, and 19.3° in powder X-ray diffraction, or in another aspect, a crystal having diffraction peaks at diffraction angles (2θ ± 0.2°) of 9.4°, 10.8°, 14.5°, 19.3°, and 19.8° in powder X-ray diffraction, or in still another aspect, a crystal having diffraction peaks at diffraction angles (2θ ± 0.2°) of 9.4°, 10.8°, 11.2°, 14.5°, 15.1°, 18.1°, 19.3°, 19.8°, 21.6°, and 22.8° in powder X-ray diffraction.

In the present description, pladienolide D 2-methyltetrahydrofuran solvate can exist as a crystal. The crystal of pladienolide D 2-methyltetrahydrofuran solvate, may be, in one aspect, a crystal having one or more diffraction peaks at diffraction angles (2θ ± 0.2°) selected from the group consisting of 10.3°, 10.7°, 11.7°, 13.3°, 14.4°, 17.2°, 18.9°, 21.2°, 22.5°, and 23.4° in powder X-ray diffraction, or in another aspect, a crystal having a diffraction peak at a diffraction angle (2θ ± 0.2°) of 18.9° in powder X-ray diffraction, or in another aspect, a crystal having diffraction peaks at diffraction angles (2θ ± 0.2°) of 10.7°, 14.4°, and 18.9° in powder X-ray diffraction, or in another aspect, a crystal having diffraction peaks at diffraction angles (2θ ± 0.2°) of 10.7°, 14.4°, 17.2°, 18.9°, and 21.2° in powder X-ray diffraction, or in still another aspect, a crystal having diffraction peaks at diffraction angles (2θ ± 0.2°) of 10.3°, 10.7°, 11.7°, 13.3°, 14.4°, 17.2°, 18.9°, 21.2°, 22.5°, and 23.4° in powder X-ray diffraction.

The crystal of pladienolide D 2-methyltetrahydrofuran solvate may be, in one aspect, a crystal having one or more peaks at chemical shifts (δ ± 0.5 ppm) selected from the group consisting of 9.1 ppm, 10.0 ppm, 10.5 ppm, 16.2 ppm, 21.4 ppm, 23.9 ppm, 24.2 ppm, 25.4 ppm, 26.8 ppm, 28.0 ppm, 28.5 ppm, 33.3 ppm, 33.7 ppm, 35.9 ppm, 39.9 ppm, 41.1 ppm, 42.7 ppm, 45.7 ppm, 56.0 ppm, 59.3 ppm, 67.9 ppm, 70.4 ppm, 72.6 ppm, 76.5 ppm, 77.7 ppm, 83.0 ppm, 124.0 ppm, 124.4 ppm, 128.0 ppm, 130.2 ppm, 134.4 ppm, 139.1 ppm, 141.0 ppm, 141.3 ppm, 166.9 ppm, and 170.4 ppm in a solid state ¹³C NMR spectrum, or in another aspect, a crystal having a peak at a chemical shift (δ ± 0.5 ppm) of 72.6 ppm in a solid state ¹³C NMR spectrum, or in another aspect, a crystal having peaks at chemical shifts (δ ± 0.5 ppm) of 72.6 ppm, 134.4 ppm, and 170.4 ppm in a solid state ¹³C NMR spectrum, or in another aspect, a crystal having peaks at chemical shifts (δ ± 0.5 ppm) of 21.4 ppm, 70.4 ppm, 72.6 ppm, 134.4 ppm, and 170.4 ppm in a solid state ¹³C NMR spectrum, or in another aspect, a crystal having peaks at chemical shifts (δ ± 0.5 ppm) of 16.2 ppm, 21.4 ppm, 67.9 ppm, 70.4 ppm, 72.6 ppm, 76.5 ppm, 77.7 ppm, 134.4 ppm, 166.9 ppm, and 170.4 ppm in a solid state ¹³C NMR spectrum, or in still another aspect, a crystal having peaks at chemical shifts (δ ± 0.5 ppm) of 10.0 ppm, 16.2 ppm, 21.4 ppm, 56.0 ppm, 59.3 ppm, 67.9 ppm, 70.4 ppm, 72.6 ppm, 76.5 ppm, 77.7 ppm, 83.0 ppm, 128.0 ppm, 130.2 ppm, 134.4 ppm, 139.1 ppm, 166.9 ppm, and 170.4 ppm in a solid state ¹³C NMR spectrum.

In the crystal of pladienolide D 2-methyltetrahydrofuran solvate, a crystal having a powder X-ray diffraction peak described above may have a peak at a chemical shift in a solid state ¹³C NMR spectrum described above at the same time. The crystal of pladienolide D 2-methyltetrahydrofuran solvate may be, in one aspect, a crystal having diffraction peaks at diffraction angles (2θ ± 0.2°) of 10.7°, 14.4°, and 18.9° in powder X-ray diffraction and having peaks at chemical shifts (δ ± 0.5 ppm) of 72.6 ppm, 134.4 ppm, and 170.4 ppm in a solid state ¹³C NMR spectrum, or in another aspect, a crystal having diffraction peaks at diffraction angles (2θ ± 0.2°) of 10.7°, 14.4°, 17.2°, 18.9°, and 21.2° in powder X-ray diffraction and having peaks at chemical shifts (δ ± 0.5 ppm) of 21.4 ppm, 70.4 ppm, 72.6 ppm, 134.4 ppm, and 170.4 ppm in a solid state ¹³C NMR spectrum, or in still another aspect, a crystal having diffraction peaks at diffraction angles (2θ ± 0.2°) of 10.3°, 10.7°, 11.7°, 13.3°, 14.4°, 17.2°, 18.9°, 21.2°, 22.5°, and 23.4° in powder X-ray diffraction and having peaks at chemical shifts (δ ± 0.5 ppm) of 16.2 ppm, 21.4 ppm, 67.9 ppm, 70.4 ppm, 72.6 ppm, 76.5 ppm, 77.7 ppm, 134.4 ppm, 166.9 ppm, and 170.4 ppm in a solid state ¹³C NMR spectrum.

The diffraction peaks in the powder X-ray diffraction and the chemical shifts in the solid state ¹³C NMR spectrum described above are characteristic peaks in the crystal of pladienolide D toluene solvate, the crystal of pladienolide D chlorobenzene solvate, the crystal of pladienolide D tetrahydrofuran solvate, and the crystal of pladienolide D 2-methyltetrahydrofuran solvate.

In general, since an error may occur in a diffraction angle (2θ) in powder X-ray diffraction within a range of ± 0.2°, the value of the diffraction angle needs to be understood as including a numerical value within a range of about ± 0.2°. Therefore, in a specific compound or a salt thereof or a solvate thereof, not only crystals in which diffraction angles of peaks in powder X-ray diffraction completely coincide with each other but also crystals in which diffraction angles of peaks coincide with each other with an error of about ± 0.2° are included in the present invention. Therefore, in the present description, for example, the phrase "having a diffraction peak at a diffraction angle (2θ ± 0.2°) of 14.4°" means "having a diffraction peak at a diffraction angle (2θ) of 14.2° to 14.6°", and the same applies to other diffraction angles.

In general, the peak intensity or the half width of the diffraction angle (2θ) in powder X-ray diffraction varies for each measurement due to a difference in measurement conditions or a variation in size or shape of each particle of a powder crystal used as a measurement sample even if the crystal forms are identical, and a constant peak intensity or half width is not always shown. Therefore, in the comparison of the powder X-ray diffraction patterns, even if there is a difference in peak intensity or half width at the same diffraction angle (2θ), the difference does not mean that the difference is derived from different crystal forms. Therefore, with respect to the diffraction peak characteristic of the specific crystal of the present invention, it is meant that the crystal having a powder X-ray diffraction pattern having such a difference has a crystal form identical to that of the crystal of the present invention.

In the present description, the phrase "having a powder X-ray pattern substantially identical to the powder X-ray diffraction pattern shown in FIG. 4" means that, in the powder X-ray diffraction pattern having a characteristic diffraction peak, even if the peak intensity or the half width is different from that of the powder X-ray diffraction pattern shown in FIG. 4, when the peak intensity or the half width coincides with that of the powder X-ray diffraction pattern shown in FIG. 4 within an error range of ± 0.2°, the crystal is identical to the crystal exhibiting the powder X-ray diffraction pattern shown in FIG. 4.

In general, since an error may occur in a chemical shift δ in a solid state ¹³C NMR spectrum within a range of ± 0.5 ppm, the value of the chemical shift needs to be understood as including a numerical value within a range of about ± 0.5 ppm. Therefore, not only crystals in which chemical shifts in a solid state ¹³C NMR spectrum completely coincide with each other but also crystals in which chemical shifts coincide with each other with an error of about ± 0.5 ppm are included in the present invention. Therefore, in the present description, for example, the phrase "having a peak at a chemical shift (δ ± 0.5 ppm) of 72.6 ppm" means having a peak at a chemical shift (δ) in a range of 72.1 ppm to 73.1 ppm, and the same applies to the chemical shifts in the other ¹³C solid state NMR spectra.

In general, the peak intensity or the half width at the chemical shift δ in the solid state ¹³C NMR spectrum varies for each measurement due to a difference in measurement conditions or a variation in size or shape of each particle of a powder crystal used as a measurement sample even if the crystal forms are identical, and a constant peak intensity or half width is not always shown. Therefore, in the comparison of the ¹³C solid state NMR spectra, even if there is a difference in peak intensity or half width at the same chemical shift δ, the difference does not mean that the difference is derived from different crystal forms. Therefore, with respect to the peak characteristic of the specific crystal of the present invention, it is meant that the crystal having a solid state ¹³C NMR spectrum having such a difference has a crystal form identical to that of the crystal of the present invention.

In the present description, the phrase "having a solid state ¹³C NMR spectrum substantially identical to the solid state ¹³C NMR spectrum shown in FIG. 5." means that, in the solid state ¹³C NMR spectrum having a characteristic diffraction peak, even if the peak intensity or the half width is different from that of the solid state ¹³C NMR spectrum shown in FIG. 5, when the peak intensity or the half width coincides with that of the solid state ¹³C NMR spectrum shown in FIG. 5 within an error range of ± 0.5 ppm, the crystal is identical to the crystal exhibiting the solid state ¹³C NMR spectrum shown in FIG. 5.

The pladienolide D used for crystallization may be one produced by fermentation or one produced by chemical synthesis, and can also be produced by the above-described method for producing pladienolide D. The pladienolide D used for crystallization may be in any form, may be an anhydride or a solvate, may be amorphous or crystalline (including those formed of multiple crystal polymorphs), or may be a mixture thereof.

The solvent used for crystallization is not particularly limited as long as it is a solvent that forms a solvate of pladienolide D, and examples thereof include alcohol-based solvents such as methanol, ethanol, isopropanol, and 1-propanol; acetonitrile; amide-based solvents such as N,N-dimethylformamide; ester-based solvents such as ethyl acetate and isopropyl acetate; saturated hydrocarbon-based solvents such as hexane and heptane; ketone-based solvents such as acetone and 2-butanone; ether-based solvents such as diethyl ether, diisopropyl ether, t-butyl methyl ether, tetrahydrofuran, and 2-methyltetrahydrofuran; unsaturated hydrocarbon solvents such as toluene and chlorobenzene; and water. These solvents may be used alone, or two or more thereof may be used in combination.

The amount of the solvent used can be appropriately selected with the lower limit being the amount in which pladienolide D is dissolved by heating or the amount in which a suspension can be stirred, and the upper limit being the amount in which the yield of a crystal does not significantly decrease.

The temperature when pladienolide D is dissolved by heating may be appropriately selected according to the solvent, and is preferably in a range of 50°C to a temperature at which a recrystallization solvent starts to reflux, and more preferably 60 to 80°C.

Since rapid cooling may give a crystal (polymorph) having a different aspect, cooling during crystallization may be desirably performed by appropriately adjusting the cooling rate in consideration of the effect on the quality, grain size, and the like of the crystal, and cooling at a rate of, for example, 5 to 40°C/hour is preferable. More preferably, cooling is performed at a rate of, for example, 5 to 25°C/hour.

The final crystallization temperature can be appropriately selected based on the yield, quality, and the like of the crystal, and is preferably -25°C to +30°C.

When a crystal of a solvate of pladienolide D is obtained by crystallization, the crystal of a solvate of pladienolide D can be obtained, for example, by separating the crystallized crystal by a normal filtration operation, washing the crystal separated by filtration with a solvent as necessary, and further drying the crystal. As the solvent used for washing the crystal, a solvent similar to the crystallization solvent can be used, and the crystal can also be washed with a solvent different from the crystallization solvent. Examples thereof include methanol, ethanol, isopropanol, 1-propanol, acetonitrile, ethyl acetate, isopropyl acetate, hexane, heptane, acetone, 2-butanone, diethyl ether, diisopropyl ether, t-butyl methyl ether, tetrahydrofuran, 2-methyltetrahydrofuran, toluene, and chlorobenzene. These solvents may be used alone, or two or more thereof may be used in combination.

The crystal separated by the filtration operation can be appropriately dried by being left in the atmosphere or under a nitrogen stream or by heating.

As the drying time, a time until the amount of the residual solvent falls below a predetermined amount may be appropriately selected according to the production amount, the drying device, the drying temperature, and the like. Drying can be performed under ventilation or under reduced pressure. The degree of pressure reduction may be appropriately selected according to the production amount, the drying device, the drying temperature, and the like. The obtained crystal after drying can also be left in the atmosphere as necessary.

The pladienolide D used in the production of the crystal of a solvate of pladienolide D according to the present disclosure may be one chemically synthesized according to, for example, the production method according to an aspect of the present disclosure, or may be one produced by fermentation. As a method of producing pladienolide D by fermentation, for example, the production method described in Patent Literature 1 may be used, and the method is incorporated herein by reference.

The crystal of a solvate of pladienolide D according to the present disclosure (for example, a crystal of pladienolide D 2-methyltetrahydrofuran solvate) may have high purity. In the crystal of a solvate of pladienolide D according to the present disclosure, the high purity may mean that, for example, the ratio of the area of the absorption peak of pladienolide D to the total area of the absorption peak detected at 240 nm in an analysis using high performance liquid chromatography (HPLC) is 90.00% or more, 93.00% or more, 95.00% or more, or 96.00% or more. Another aspect of the present disclosure may be a composition containing a crystal of a solvate of pladienolide D according to an aspect of the present disclosure, in which the ratio of the area of the absorption peak of pladienolide D to the total area of the absorption peak detected at 240 nm in an analysis using high performance liquid chromatography (HPLC) is 90.00% or more, 93.00% or more, 95.00% or more, or 96.00% or more. In these cases, the analysis conditions of HPLC may be, for example, those used in "Example E. Measurement of purity" described later.

### Examples

Hereinafter, the present invention will be described in detail with reference to examples.

A compound for which the name of literature or the like is described indicates that the compound was produced according to the literature or the like.

Also, abbreviations used herein are conventional abbreviations well known to those skilled in the art. In the present description, the following abbreviations are used.
Ac: acetyl
AcOH: acetic acid
Ac₂O: acetic anhydride
Ag₂CO₃: silver carbonate
Ag₂O: silver oxide(I)
B₂pin₂: bis(pinacolato)diboron
n-Bu₃SnH: tri-n-butyltin
DCM: dichloromethane
DDQ: 2,3-dichloro-5,6-dicyano-p-benzoquinone
DIPEA: N,N-diisopropylethylamine
DMAP: 4-dimethylaminopyridine
DMF: N,N-dimethylformamide
Et₃N: triethylamine
Grubbs-II: (1,3-bis(2,4,6-trimethylphenyl)-2-imidazolidinylidene)dichloro(phenylmethylene)(tricyclohexylphosphine)ruthe nium
Hoveyda-Grubbs-II: (1,3-bis-(2,4,6-trimethylphenyl)-2-imidazolidinylidene)dichloro(o-isopropoxyphenylmethylene)ruthenium
K₂CO₃: potassium carbonate
Me: methyl
MeCN: acetonitrile
MeOH: methanol
MPM: para-methoxybenzyl
MTBE: 2-methoxy-2-methylpropane
PdCl₂(PhCN)₂: dichlorobis(benzonitrile)palladium(II)
PdCl₂(PPh₃)₂: bis(triphenylphosphine)palladium(II) dichloride
Pd₂(dba)₃: tris(dibenzylideneacetone)dipalladium(0)
Pd(dppf)Cl₂: (1,1'-bis(diphenylphosphino)ferrocene)dichloropalladium(II)
Pd(OAc)₂: palladium(II) acetate
PPTS: pyridinium p-toluenesulfonate
TFA: trifluoroacetic acid
TBAF: tetrabutylammonium fluoride
TBDPS: tert-butyldiphenylsilyl
TBS-Cl: tert-butyldimethylchlorosilane
TBS-OTf: tert-butyldimethylsilyl trifluoromethanesulfonate
TES-Cl: triethylchlorosilane
TES-OTf; triethylsilyl trifluoromethanesulfonate
THF: tetrahydrofuran
XPhos: 2-dicyclohexylphosphino-2',4',6'-triisopropylbiphenyl
Zhan Catalyst-1B; 1,3-bis(2,4,6-trimethylphenyl)-4,5-dihydroimidazol-2-ylidene[2-(isopropoxy)-5-(N,N-dimethylaminosulfonyl)phenyl]methyleneruthenium(II) dichloride
n-: normal-
s-: secondary-
t-: tertiary-
tert-: tertiary-
¹H-NMR: proton nuclear magnetic resonance spectrometry

"Room temperature" in the following examples and production examples usually refers to about 10°C to about 35°C. % represents weight percent unless otherwise specified.

In the measurement of the proton nuclear magnetic resonance spectrum, a nuclear magnetic resonance apparatus, model JNM-ECZ500R/S1 manufactured by JEOL Ltd., or a nuclear magnetic resonance apparatus, Avance Neo 700 MHz manufactured by Bruker was used. Chemical shifts of proton nuclear magnetic resonance spectra are recorded in δ unit (ppm) relative to tetramethylsilane in CDCl₃, recorded in δ unit with methanol set at 3.31 ppm (3.30 ppm for a solvate of pladienolide D) in CD₃OD, and recorded in δ unit with acetonitrile set at 1.93 ppm in CD₃CN. Coupling constants are recorded in Hertz (Hz). The abbreviations of the splitting pattern are as follows.
s: singlet, d: doublet, t: triplet, q: quartet, m: multiplet, br.s: broad singlet

As for chromatography, any of Silica Gel 60 (70-230 mesh ASTM) manufactured by Merck, parallel prep {Column: Hi-Flash^{™} Column (Silicagel) manufactured by YAMAZEN, size; any of S (16 × 60 mm), M (20 × 75 mm), L (26 × 100 mm), 2L (26 × 150 mm), and 3L (46 × 130 mm)} manufactured by YAMAZEN, or Isolera 1 {column: Biotage Sfar 20um (Silicagel HC D) manufactured by Biotage, size; any of 5 g (9 mL), 25 g (42 mL), 50 g (80 mL), 100 g (150 mL), 200 g (310 mL), and 350 g (530 mL)} manufactured by Biotage was used as the silica gel.

As the NH silica gel, CHROMATOREX NH-DM2035 manufactured by Fuji Silysia Chemical Ltd., parallel prep {Column: Hi-Flash^{™} Column (Amino) manufactured by YAMAZEN, size; any of S (16 × 60 mm), M (20 × 75 mm), L (26 × 100 mm), 2L (26 × 150 mm), and 3L (46 × 130 mm)} manufactured by YAMAZEN, and Isolera 1 {column: Biotage Sfar 50 µm (Amino D) manufactured by Biotage, size; any of 5 g (9 mL) and 11 g (15 mL)} manufactured by Biotage were used.

Powder X-ray crystal diffraction of crystals produced in the following examples was performed by placing the obtained crystals on a sample stage of a powder X-ray diffractometer and analyzing the crystals under the following conditions.

### (Conditions for transmittance method)

X-ray source: Cu-Kα
Voltage: 45 kV
Current: 200 mA
Optical system: condensing optical system
Solar slit: 2.5°
Detector: D/teX Ultra 250 (one-dimensional semiconductor detector)
Scan speed: 10°/min
Step width: 0.01°
Scan range: 3° to 40°
Film: Mylar film

In the thermal analysis (TG-DTA), a sample was precisely weighed in an aluminum sample pan and measured under the following conditions.

### (Measurement conditions)

Atmosphere: under 100 mL/min nitrogen gas flow
Control: Empty aluminum sample pan
Heating rate: 10°C/min
Sampling interval: 1 sec
Measurement temperature range: room temperature to 200°C, room temperature to 150°C, or room temperature to 160°C

A solid state ¹³C NMR spectrum was measured under the following conditions by sealing about 100 mg of a solid sample in a sample tube.

### (Measurement conditions)

Apparatus used: Avance Neo 400 MHz (manufactured by Bruker) 4 mm-CPMAS probe (manufactured by Bruker)
Measurement nucleus: ¹³C (resonance frequency 100.6228298 MHz)
Measurement temperature: room temperature
Pulse mode: CPTOSS measurement
Rotation speed: 10,000 Hz
Pulse repeating time: 5 sec
Contact time: 2 msec
Number of integrations: 2048 times
Reference material: 10% 15 N glycine (external reference: 176.03 ppm)

As the nomenclature of the compounds shown below, the nomenclature shown in "E-Notebook" version 18 (PerkinElmer, Inc.) was used except for commonly used reagents.

### Example A-1

### Synthesis of (2R,3 S)-2-((2R,3R)-3-((R)-2-hydroxy-2-methylbut-3-yn-1-yl)oxiran-2-yl)pentan-3-ol

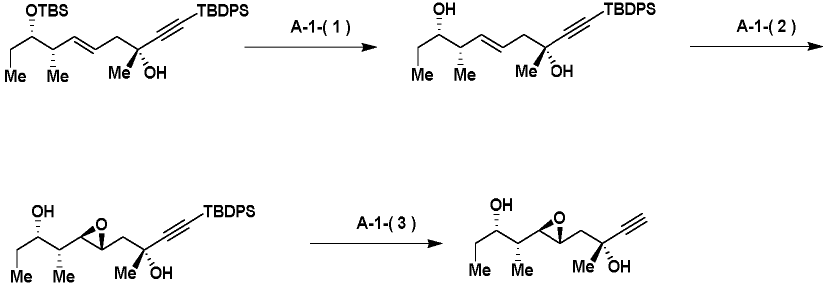

### A-1-(1)

### Synthesis of (3R,7S,8S,E)-1-(t-butyldiphenylsilyl)-3,7-dimethyldec-5-en-1-yne-3,8-diol

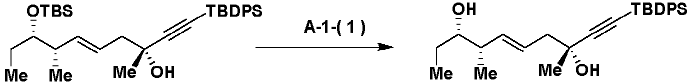

PPTS (3.96 g, 15.8 mmol) was added to a solution of (3R,7S,8S,E)-8-((t-butyldimethylsilyl)oxy)-1-(t-butyldiphenylsilyl)-3,7-dimethyldec-5-en-1-yne-3-ol (CAS No. 2389978-00-9; Angew. Chem. Int. Ed. 2019, 58, 18803-18807) (4.33 g, 7.89 mmol) synthesized according to the method described in the literature in MeOH (86 mL) at 25°C. The reaction mixture was stirred at 50°C for 21 hours. The reaction mixture was allowed to cool to room temperature, ethyl acetate and water were then added to the reaction mixture, and the organic layer was separated. The aqueous layer was subjected to re-extraction with ethyl acetate. The combined organic layer was washed sequentially with 0.5 N hydrochloric acid, water, a saturated aqueous sodium bicarbonate solution, and saturated saline. The organic layer was dried over anhydrous magnesium sulfate, filtered, and then concentrated under reduced pressure. The residue was purified by silica gel column chromatography (10%-50% ethyl acetate/n-heptane) to obtain the title compound (2.97 g).

¹H-NMR (500 MHz, CDCl₃) δ (ppm): 0.93 (t, J = 7.5 Hz, 3H), 1.02 (d, J= 6.9 Hz, 3H), 1.08 (s, 9H), 1.33-1.45 (m, 2H), 1.48-1.53 (m, 2H), 1.59 (s, 3H), 2.25-2.34 (m, 1H), 2.39-2.47 (m, 1H), 2.56 (dd, J = 13.8, 6.3 Hz, 1H), 3.39 (dt, J = 8.7, 4.5 Hz, 1H), 5.58-5.66 (m, 1H), 5.69-5.79 (m, 1H), 7.33-7.44 (m, 6H), 7.75-7.81 (m, 4H).

### A-1-(2)

### Synthesis of (2R,3S)-2-((2R,3R)-3-((R)-4-(t-butyldiphenylsilyl)-2-hydroxy-2-methylbut-3-yn-1-yl)oxiran-2-yl)pentan-3-ol

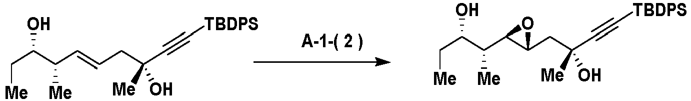

To a mixed solution of (3R,7S,8S,E)-1-(t-butyldiphenylsilyl)-3,7-dimethyldec-5-en-1-yne-3,8-diol (2.97 g, 6.83 mmol) obtained in Example A-1-(1) in a 0.05 M aqueous sodium tetraborate solution (pH = 9; 50 mL) containing MeCN (75 mL) and 0.4 mM disodium ethylenediamine-N,N,N',N'-tetraacetate, 1,2:4,5-di-O-isopropylidene-β-D-erythro-2,3-hexodiulo-2,6-pyranose (Shi epoxidation catalyst; CAS No. 18422-53-2) (5.29 g, 20.5 mmol) was added at 30°C, and the mixture was stirred at room temperature for 15 minutes. The mixture was ice-cooled, and Oxone (registered trademark) powder (CAS No. 70693-62-8) (1.58 g, 2.56 mmol) and K₂CO₃ powder (708 mg, 5.12 mmol) were added 8 times at 15 minute intervals. The reaction mixture was then stirred under ice cooling for 150 minutes. Ethyl acetate and water were added to the reaction liquid, and the organic layer was separated. The aqueous layer was subjected to re-extraction with ethyl acetate. The combined organic layer was dried over anhydrous magnesium sulfate, filtered, and then concentrated under reduced pressure. The residue was purified by silica gel column chromatography (15%-40% ethyl acetate/n-heptane). Fractions containing the desired product were collected and concentrated. Heptane was added to the resulting residue, then the mixture was sonicated and stirred at room temperature for 30 minutes. The resulting solid was collected by filtration, washed with heptane and dried under reduced pressure to obtain the title compound (1.66 g).

¹H-NMR (500 MHz, CDCl₃) δ (ppm): 0.90 (d, J = 6.9 Hz, 3H), 0.93 (t, J = 7.5 Hz, 3H), 1.08 (s, 9H), 1.46-1.51 (m, 2H), 1.58 (br.dd, J = 6.6, 3.2 Hz, 1H), 1.66 (s, 3H), 1.84 (br.s, 1H), 1.96 (dd, J = 14.3, 7.5 Hz, 1H), 2.08-2.14 (m, 1H), 2.87 (dd, J = 6.3, 2.3 Hz, 1H), 3.03 (br.s, 1H), 3.26 (ddd, J = 7.2, 4.9, 2.3 Hz, 1H), 3.58-3.64 (m, 1H), 7.34-7.43 (m, 6H), 7.77 (ddd, J = 7.9, 3.3, 1.4 Hz, 4H).

### A-1-(3)

### Synthesis of (2R,3S)-2-((2R,3R)-3-((R)-2-hydroxy-2-methylbut-3-yn-1-yl)oxiran-2-yl)pentan-3-ol

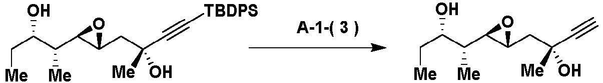

To a solution of (2R,3S)-2-((2R,3R)-3-((R)-4-(t-butyldiphenylsilyl)-2-hydroxy-2-methylbut-3-yn-1-yl)oxiran-2-yl)pentan-3-ol (1.66 g, 3.68 mmol) obtained in Example A-1-(2) in THF (9.2 mL), a THF solution of TBAF (1 M; 9.2 mL, 9.2 mmol) was added at 25° C. Then, the reaction mixture was stirred at room temperature for 21.5 hours. A saturated aqueous ammonium chloride solution, ethyl acetate, and water were added to the reaction liquid, and the organic layer was separated. The aqueous layer was subjected to extraction with ethyl acetate (twice). The combined organic layer was dried over anhydrous magnesium sulfate, filtered, and then concentrated under reduced pressure. The residue was purified by silica gel column chromatography (20%-50% ethyl acetate/n-heptane) to obtain the title compound (726 mg).

¹H-NMR (500 MHz, CDCl₃) δ (ppm): 0.94-1.00 (m, 6H), 1.50-1.56 (m, 5H), 1.57-1.64 (m, 1H), 1.79-1.91 (m, 2H), 1.96-2.03 (m, 1H), 2.52 (s, 1H), 2.84 (dd, J = 6.9, 2.3 Hz, 1H), 2.93 (s, 1H), 3.20 (ddd, J = 7.2, 4.9, 2.3 Hz, 1H), 3.67 (tt, J = 7.4, 3.5 Hz, 1H).

### Example A-2

### Synthesis of (2R,3 S)-2-((2R,3R)-3-((R)-2-hydroxy-2-methylbut-3-en-1-yl)oxiran-2-yl)pentan-3-ol

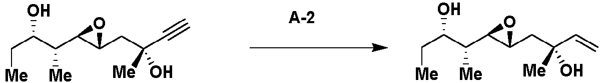

To a solution of (2R,3 S)-2-((2R,3R)-3-((R)-2-hydroxy-2-methylbut-3-yn-1-yl)oxiran-2-yl)pentan-3-ol (200 mg, 0.942 mmol) obtained in Example A-1-(3) in ethyl acetate (4 mL), Lindlar catalyst (20 mg) was added at 25°C. The reaction mixture was stirred under a hydrogen atmosphere at room temperature for 2.5 hours. The catalyst was filtered off and washed with ethyl acetate. The filtrate was concentrated under reduced pressure. The residue was purified by silica gel column chromatography (30%-60% ethyl acetate/n-heptane) to obtain the title compound (183 mg).

¹H-NMR (500 MHz, CDCl₃) δ (ppm): 0.91 (d, J = 7.3 Hz, 3H), 0.97 (t, J = 7.3 Hz, 3H), 1.36 (s, 3H), 1.48-1.54 (m, 2H), 1.63 (td, J = 6.7, 3.1 Hz, 1H), 1.71-1.83 (m, 2H), 2.06 (br.d, J = 4.3 Hz, 1H), 2.31 (s, 1H), 2.76 (dd, J = 6.7, 2.5 Hz, 1H), 2.99 (td, J = 6.1, 2.5 Hz, 1H), 3.66 (br.d, J = 3.1 Hz, 1H), 5.11 (dd, J = 11.0, 1.2 Hz, 1H), 5.31 (dd, J = 17.1, 1.2 Hz, 1H), 5.98 (dd, J = 17.1, 10.4 Hz, 1H).

### Example A-3

### Synthesis of triethyl(((R)-2-methyl-1-((2R,3R)-3-((2S,3S)-3-((triethylsilyl)oxy)pentan-2-yl)oxiran-2-yl)but-3-en-2-yl)oxy)silane

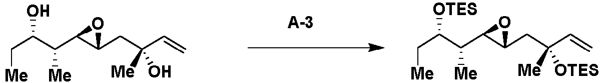

To a solution of (2R,3 S)-2-((2R,3R)-3-((R)-2-hydroxy-2-methylbut-3-en-1-yl)oxiran-2-yl)pentan-3-ol (91 mg, 0.425 mmol) obtained in Example A-2 and 2,6-lutidine (0.20 mL, 1.7 mol) in THF (2 mL), TES-OTf (0.29 mL, 1.3 mmol) was added at 0°C over 3 minutes. The reaction mixture was stirred at the same temperature for 45 minutes. To the reaction mixture, 2,6-lutidine (0.10 mL, 0.86 mmol) was added at 0°C, and subsequently TES-OTf (0.15 mL, 0.66 mmol) was added over 2 minutes. Then, the reaction liquid was stirred for 30 minutes. To the reaction liquid, water (1 mL) was added at 0°C, and the mixture was stirred for 5 minutes. Ethyl acetate and water were added to the mixture, and the organic layer was separated. The organic layer was washed sequentially with 0.5 N hydrochloric acid, saline, a saturated aqueous sodium bicarbonate solution, and saturated saline. The organic layer was dried over anhydrous magnesium sulfate, filtered, and then concentrated under reduced pressure. The residue was purified by silica gel column chromatography (0%-10% ethyl acetate/n-heptane) to obtain the title compound (155 mg).

¹H-NMR (500 MHz, CDCl₃) δ (ppm): 0.57-0.66 (m, 12H), 0.83 (t, J = 7.3 Hz, 3H), 0.89 (d, J = 7.3 Hz, 3H), 0.93-1.01 (m, 18H), 1.27-1.34 (m, 1H), 1.39 (s, 3H), 1.46-1.52 (m, 2H), 1.61-1.66 (m, 1H), 1.80 (dd, J = 14.1, 5.5 Hz, 1H), 2.60 (dd, J = 8.0, 2.5 Hz, 1H), 2.86 (td, J = 5.7, 2.1 Hz, 1H), 3.75 (td, J = 6.4, 3.7 Hz, 1H), 5.02 (dd, J = 10.4, 1.2 Hz, 1H), 5.18 (dd, J = 17.1, 1.2 Hz, 1H), 5.92 (dd, J = 17.1, 11.0 Hz, 1H).

### Example A-4

### Synthesis of triethyl(((R,E)-2-methyl-4-(4,4,5,5,-tetramethyl-1,3,2-dioxaborolan-2-yl)-1-((2R,3R)-3-((2S,3S)-3-((triethylsilyl)oxy)pentan-2-yl)oxiran-2-yl)but-3-en-2-yl)oxy)silane

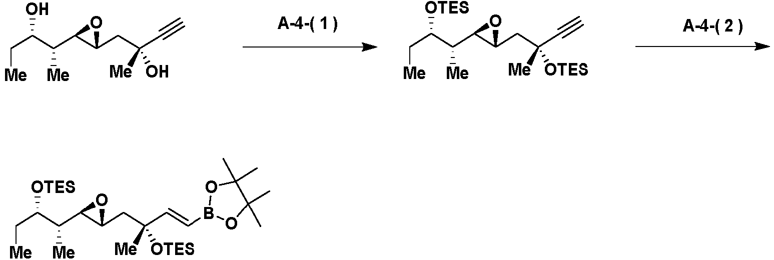

### A-4-(1)

### Synthesis of triethyl(((R)-2-methyl-1-((2R,3R)-3-((2S,3S)-3-((triethylsilyl)oxy)pentan-2-yl)oxiran-2-yl)but-3-yn-2-yl)oxy)silane

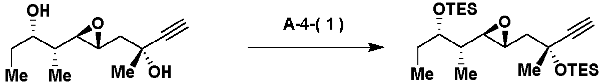

To a solution of (2R,3 S)-2-((2R,3R)-3-((R)-2-hydroxy-2-methylbut-3-yn-1-yl)oxiran-2-yl)pentan-3-ol (56 mg, 0.26 mmol) obtained in Example A-1-(3) and 2,6-lutidine (79 µL, 0.68 mmol) in THF (1.9 mL), TES-OTF(0.14 mL, 0.62 mmol) was added at 0°C over 2 minutes. The reaction mixture was stirred at the same temperature for 30 minutes. To the reaction mixture, 2,6-lutidine (40 µL, 0.34 mmol) was added at 0°C. TES-OTf (70 µL, 0.31 mmol) was then added over 2 minutes. Then, the reaction mixture was stirred for 20 minutes. To the reaction mixture, a saturated aqueous sodium bicarbonate solution (1 mL) was added at 0°C, and the mixture was stirred for 5 minutes. Ethyl acetate and water were added to the mixture, and the organic layer was separated. The organic layer was washed sequentially with 0.5 N hydrochloric acid, water, a saturated aqueous sodium bicarbonate solution, and saturated saline. The organic layer was dried over anhydrous magnesium sulfate, filtered, and then concentrated under reduced pressure. The residue was purified by silica gel column chromatography (0%-10% ethyl acetate/n-heptane) to obtain the title compound (109 mg).

¹H-NMR (500 MHz, CDCl₃) δ (ppm): 0.59-0.65 (m, 6H), 0.66-0.73 (m, 6H), 0.84 (t, J = 7.5 Hz, 3H), 0.93 (d, J = 6.9 Hz, 3H), 0.95-0.99 (m, 18H), 1.27-1.36 (m, 1H), 1.45-1.54 (m, 5H), 1.77 (dd, J = 13.8, 6.3 Hz, 1H), 2.03 (dd, J = 13.8, 5.2 Hz, 1H), 2.45 (s, 1H), 2.71 (dd, J = 8.0, 2.3 Hz, 1H), 2.94-3.00 (m, 1H), 3.77 (td, J = 6.3, 3.4 Hz, 1H).

### A-4-(2)

### Synthesis of triethyl(((R,E)-2-methyl-4-(4,4,5,5,-tetramethyl-1,3,2-dioxaborolan-2-yl)-1-((2R,3R)-3-((2S,3S)-3-((triethylsilyl)oxy)pentan-2-yl)oxiran-2-yl)but-3-en-2-yl)oxy)silane

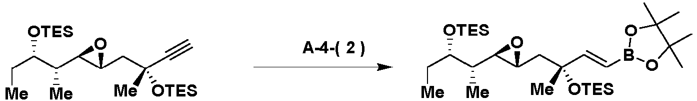

To a solution of triethyl(((R)-2-methyl-1-((2R,3R)-3-((2S,3S)-3-((triethylsilyl)oxy)pentan-2-yl)oxiran-2-yl)but-3-yn-2-yl)oxy)silane (46.8 mg, 106 µmol) obtained in Example A-4-(1) in DCM (1 mL), pinacolborane (CAS No. 25015-63-8) (23 µL, 0.16 mmol) and carbonyl bis(triphenylphosphine)rhodium(I) chloride (14.7 mg, 21 µmol) were added at 25°C. The reaction mixture was stirred under a nitrogen atmosphere at 40°C for 8 hours. The reaction mixture was allowed to cool. The reaction mixture was directly purified by silica gel column chromatography (0%-5% ethyl acetate/n-heptane) to obtain the title compound (32.2 mg).

¹H-NMR (500 MHz, CDCl₃) δ (ppm): 0.61 (q, J = 8.0 Hz, 12H), 0.82 (t, J = 7.3 Hz, 3H), 0.88 (d, J = 6.7 Hz, 3H), 0.96 (td, J = 8.0, 3.7 Hz, 18H), 1.19-1.23 (m, 1H), 1.27 (d, J = 1.2 Hz, 12H), 1.37 (s, 3H), 1.43-1.54 (m, 3H), 1.93 (dd, J = 13.8, 4.6 Hz, 1H), 2.56 (dd, J = 8.3, 2.1 Hz, 1H), 2.84 (ddd, J = 6.9, 4.7, 2.5 Hz, 1H), 3.73 (td, J = 6.4, 3.1 Hz, 1H), 5.64 (d, J = 18.3 Hz, 1H), 6.61 (d, J = 17.7 Hz, 1H).

### Example A-5

### Synthesis of (2R,3 S)-2-((2R,3R)-3-((R)-2-hydroxy-2-methylbut-3-en-1-yl)oxiran-2-yl)pentan-3-yl 3,5-dinitrobenzoate

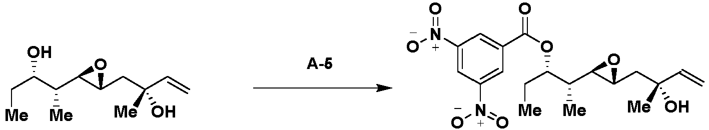

To a solution of (2R,3 S)-2-((2R,3R)-3-((R)-2-hydroxy-2-methylbut-3-en-1-yl)oxiran-2-yl)pentan-3-ol (50 mg, 0.23 mmol) obtained in Example A-2, Et₃N (75 µL, 0.54 mmol) and DMAP (5.7 mg, 47 µmol) in DCM (2 mL), 3,5-dinitrobenzoyl chloride (81 mg, 0.35 mmol) was added at 0°C. The reaction mixture was stirred at 0°C for 10 minutes and at room temperature for 45 minutes. To the reaction mixture, water (1 mL) was added at 0°C, and the mixture was stirred for 10 minutes. Ethyl acetate and 1 N hydrochloric acid were added to the mixture, and the organic layer was separated. The organic layer was washed sequentially with water, a saturated aqueous sodium bicarbonate solution, and saturated saline. The organic layer was dried over anhydrous magnesium sulfate, filtered, and then concentrated under reduced pressure. The residue was purified by silica gel column chromatography (20%-40% ethyl acetate/n-heptane) to obtain the title compound (76 mg).

¹H-NMR (500 MHz, CDCl₃) δ (ppm): 0.97 (t, J = 7.6 Hz, 3H), 1.08 (d, J = 7.3 Hz, 3H), 1.33 (s, 3H), 1.60-1.67 (m, 1H), 1.68-1.76 (m, 1H), 1.83-1.91 (m, 3H), 2.02 (s, 1H), 2.62 (dd, J = 8.0, 1.8 Hz, 1H), 2.90-2.95 (m, 1H), 5.11 (d, J = 10.4 Hz, 1H), 5.26-5.34 (m, 2H), 5.95 (dd, J = 17.1, 11.0 Hz, 1H), 9.17 (d, J = 1.8 Hz, 2H), 9.22-9.25 (m, 1H).

### Example A-6

### Synthesis of (2R,3S)-2-((2R,3R)-3-((R,E)-4-(benzyldimethylsilyl)-2-hydroxy-2-methylbut-3-en-1-yl)oxiran-2-yl)pentan-3-ol

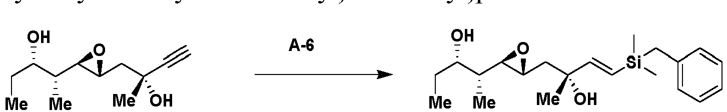

A mixture of Ag₂O (0.7 mg, 2.6 µmol), XPhos (CAS No. 564483-18-7) (2.7 mg, 5.7 µmol), and THF (0.75 mL) was stirred under a nitrogen atmosphere at 50°C for 30 minutes, and then the mixture was allowed to cool. To this mixture, a solution of (2R,3S)-2-((2R,3R)-3-((R)-2-hydroxy-2-methylbut-3-yn-1-yl)oxiran-2-yl)pentan-3-ol (20 mg, 94 µmol) obtained in Example A-1-(3) in THF (1 mL) and benzyldimethylsilane (CAS No. 1631-70-5) (40.2 µL, 0.236 mmol) were added at room temperature. The reaction mixture was stirred under a nitrogen atmosphere at 50°C for 130 minutes. The reaction mixture was allowed to cool to room temperature. The reaction mixture was directly purified by silica gel column chromatography (20%-60% ethyl acetate/n-heptane) to obtain the title compound (32.2 mg).

¹H-NMR (500 MHz, CDCl₃) δ (ppm): 0.06 (s, 3H), 0.07 (s, 3H), 0.89 (d, J = 6.9 Hz, 3H), 0.97 (t, J = 7.5 Hz, 3H), 1.30 (s, 3H), 1.50-1.54 (m, 2H), 1.61-1.71 (m, 2H), 1.74-1.81 (m, 1H), 2.06-2.11 (m, 1H), 2.14 (s, 2H), 2.32 (s, 1H), 2.73 (dd, J = 6.3, 2.3 Hz, 1H), 2.92 (td, J = 6.3, 2.3 Hz, 1H), 3.61-3.70 (m, 1H), 5.87 (d, J = 18.9 Hz, 1H), 6.05 (d, J = 18.9 Hz, 1H), 6.97 (d, J = 7.5 Hz, 2H), 7.03-7.09 (m, 1H), 7.16-7.22 (m, 2H).

### Example A-7

### Synthesis of (2R,3S)-2-((2R,3R)-3-((R,E)-2-hydroxy-2-methyl-4-(tributylstannyl)but-3-en-1-yl)oxiran-2-yl)pentan-3-ol

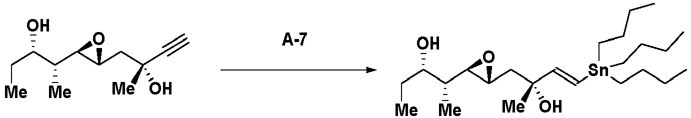

A THF solution (1.5 mL) of (2R,3S)-2-((2R,3R)-3-((R)-2-hydroxy-2-methylbut-3-yn-1-yl)oxiran-2-yl)pentan-3-ol (20.5 mg, 97 µmol) obtained in Example A-1-(3) was cooled in an ice-water bath. PdCl₂(PPh₃)₂ (3.39 mg, 4.84 µmol) and n-Bu₃SnH (31 µL, 116 µmol) were added thereto and the resulting mixture was stirred at room temperature for 3 hours. The reaction mixture was diluted with ethyl acetate and washed with a saturated aqueous sodium bicarbonate solution. The organic layer was separated and dried over anhydrous sodium sulfate, filtered, and then concentrated under reduced pressure. The resulting residue was purified by silica gel column chromatography (0%-25% ethyl acetate/n-heptane) to obtain the title compound (16.3 mg).

¹H-NMR (500 MHz, CDCl₃) δ (ppm): 0.82-0.94 (m, 18H), 0.97 (t, J = 7.5 Hz, 3H), 1.25-1.33 (m, 6H), 1.34 (s, 3H), 1.44-1.54 (m, 8H), 1.62-1.75 (m, 2H), 1.83 (dd, J = 14.0, 6.6 Hz, 1H), 2.20 (br.d, J = 4.6 Hz, 1H), 2.28 (s, 1H), 2.76 (dd, J = 6.3, 2.3 Hz, 1H), 2.99 (td, J = 6.2, 2.6 Hz, 1H), 3.66 (br.dd, J = 7.5, 3.4 Hz, 1H), 6.08 (d, J = 19.5 Hz, 1H), 6.20 (d, J = 19.5 Hz, 1H).

### Example A-8

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-ol

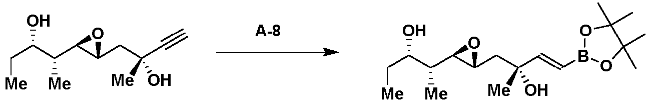

A solution of copper(II) trifluoromethanesulfonate (21.3 mg, 0.059 mmol), B₂pin₂ (359 mg, 1.41 mmol), sodium t-butoxide (11.3 mg, 0.118 mmol), and 2,6-di(1-pyrazolyl)pyridine (24.9 mg, 0.118 mmol) in MeCN (8 mL) and MeOH (150 µL) was stirred at room temperature for 10 minutes (this mixture is referred to as solution B).

(2R,3S)-2-((2R,3R)-3-((R)-2-hydroxy-2-methylbut-3-yn-1-yl)oxiran-2-yl)pentan-3-ol (50 mg, 0.236 mmol) obtained in Example A-1-(3) was dissolved in solution B (1.6 ml). The resulting reaction mixture was stirred at room temperature for 2 hours and then stirred at 50°C for 4 hours. Solution B (1.6 ml) was added to the reaction mixture, and the reaction mixture was stirred at 50°C for 2 hours, and then allowed to cool to room temperature. The reaction mixture was quenched with water. The resulting mixture was filtered. The filtrate was subjected to extraction with ethyl acetate (twice). The combined organic layer was washed with saturated saline, dried over anhydrous sodium sulfate, filtered, and then concentrated under reduced pressure. The residue was purified by silica gel column chromatography (10%-100% ethyl acetate/n-heptane) to obtain the title compound (52.0 mg).

¹H-NMR (500 MHz, CDCl₃) δ (ppm): 0.89 (d, J = 7.3 Hz, 3H), 0.96 (t, J = 7.3 Hz, 3H), 1.24 (s, 12H), 1.35 (s, 3H), 1.48-1.56 (m, 2H), 1.60 (ddd, J = 10.2, 6.9, 3.7 Hz, 1H), 1.71 (br.dd, J = 14.1, 6.1 Hz, 1H)1.85 (dd, J = 14.1, 6.1 Hz, 1H), 1.98 (br.s, 1H), 2.29-2.45 (m, 1H), 2.72 (dd, J = 6.1, 2.5 Hz, 1H), 2.98 (td, J = 6.1, 2.5 Hz, 1H), 3.62-3.67 (m, 1H), 5.70 (d, J = 18.3 Hz, 1H), 6.69 (d, J = 18.3 Hz, 1H).

### Example A-9

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-ol

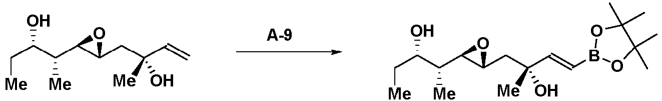

To a solution of (2R,3 S)-2-((2R,3R)-3-((R)-2-hydroxy-2-methylbut-3-en-1-yl)oxiran-2-yl)pentan-3-ol (50 mg, 0.233 mmol) obtained in Example A-2 in 1,2-dichloroethane (4 mL), para-benzoquinone (2.52 mg, 23 µmol), 2-vinyl-4,4,5,5-tetramethyl-1,3,2-dioxaborolane (108 mg, 0.70 mmol), and Zhan Catalyst-1B (34.2 mg, 47 µmol) were added at room temperature. The reaction mixture was stirred under a nitrogen atmosphere at 70°C for 3 hours. To the reaction mixture, 2-vinyl-4,4,5,5-tetramethyl-1,3,2-dioxaborolane (108 mg, 0.70 mmol) was added, and the reaction mixture was stirred at 70°C for 3 hours. Then, Zhan Catalyst-1B (34.2 mg, 47 µmol) was added to the reaction mixture, and the reaction mixture was stirred at 70°C overnight and then allowed to cool. The reaction mixture was filtered. The filtrate was concentrated under reduced pressure. The residue was purified by silica gel column chromatography (10%-50% ethyl acetate/n-heptane) to obtain the title compound (51.3 mg).

The ¹H-NMR spectrum (500 MHz, CDCl₃) of the obtained compound was consistent with the NMR spectrum of the compound obtained in Example A-8.

### Example A-10

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate

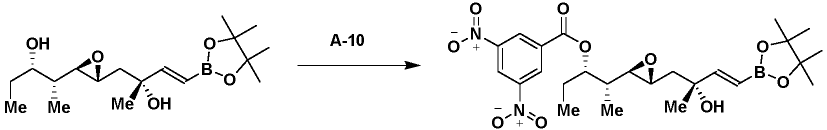

To a solution of (2R,3S)-2-((2R,3R)-3-((RE)-2-hydroxy-2-methyl-4-(4,4,5,5-tetramethyl-1,3,2-dioxaborolan-2-yl)but-3-en-1-yl)oxiran-2-yl)pentan-3-ol (42 mg, 0.123 mmol) obtained in Example A-8 and DMAP (3.02 mg, 25 µmol) in DCM (2 mL), Et₃N (39.6 µL, 0.284 mmol) and 3,5-dinitrobenzoyl chloride (42.7 mg, 0.185 mmol) were added at 0°C. The reaction mixture was stirred at room temperature for 30 minutes. The reaction mixture was quenched with a saturated aqueous ammonium chloride solution. Ethyl acetate was added to the obtained mixture, and the organic layer was separated. The aqueous layer was subjected to extraction with ethyl acetate. The combined organic layer was washed sequentially with a saturated aqueous sodium bicarbonate solution and saturated saline, dried over anhydrous sodium sulfate, filtered, and then concentrated under reduced pressure. The resulting residue was purified by silica gel column chromatography (10%-50% ethyl acetate/n-heptane) to obtain the title compound (43.9 mg).

¹H-NMR (500 MHz, CDCl₃) δ (ppm): 0.97 (t, J = 7.3 Hz, 3H), 1.07 (d, J = 7.3 Hz, 3H), 1.27 (s, 12H), 1.32 (s, 3H), 1.65-1.72 (m, 2H), 1.80-1.89 (m, 3H), 2.59 (dd, J = 8.3, 2.1 Hz, 1H), 2.90 (ddd, J = 6.9, 4.7, 2.5 Hz, 1H), 5.25-5.30 (m, 1H), 5.68 (d, J = 18.3 Hz, 1H), 6.65 (d, J = 18.3 Hz, 1H), 9.17 (d, J = 2.5 Hz, 2H), 9.23 (t, J = 2.1 Hz, 1H).

### Example A-11

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate

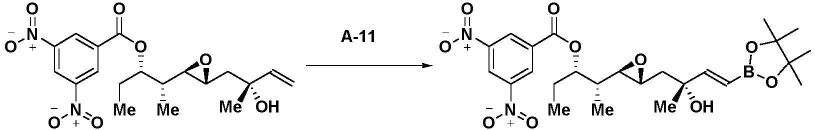

To a solution of (2R,3 S)-2-((2R,3R)-3-((R)-2-hydroxy-2-methylbut-3-en-1-yl)oxiran-2-yl)pentan-3-yl 3,5-dinitrobenzoate (15 mg, 37 µmol) obtained in Example A-5 in 1,2-dichloroethane (2 mL), para-benzoquinone (0.40 mg, 3.7 µmol), 2-vinyl-4,4,5,5-tetramethyl-1,3,2-dioxaborolane (19 µL, 0.11 mmol) and Zhan Catalyst-1B (5.39 mg, 7.35 µmol) were added at room temperature. The reaction mixture was purged with N₂ and then stirred at 70°C for 6 hours. The reaction mixture was concentrated. The residue was purified by silica gel column chromatography (0%-50% ethyl acetate/n-heptane) to obtain the title compound (19.4 mg).

The ¹H-NMR spectrum (500 MHz, CDCl₃) of the obtained compound was consistent with the NMR spectrum of the compound obtained in Example A-10.

### Example A-12

### Synthesis of (2R,3 S)-2-((2R,3R)-3-((R)-2-hydroxy-2-methylbut-3-yn-1-yl)oxiran-2-yl)pentan-3-yl 3,5-dinitrobenzoate

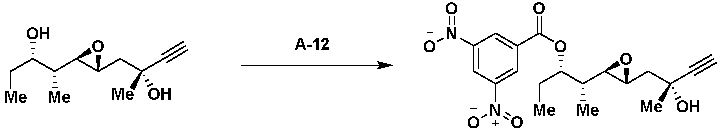

To a solution of (2R,3 S)-2-((2R,3R)-3-((R)-2-hydroxy-2-methylbut-3-yn-1-yl)oxiran-2-yl)pentan-3-ol (50 mg, 0.236 mmol) obtained in Example A-1-(3) and DMAP (5.75 mg, 47 µmol) in DCM (2 mL), Et₃N (76 µL, 0.542 mmol) and 3,5-dinitrobenzoyl chloride (81 mg, 0.353 mmol) were added at 0°C. The reaction mixture was stirred at room temperature for 5 hours. Water and ethyl acetate were added to the reaction mixture, and the organic layer was separated. The organic layer was washed sequentially with 1 N hydrochloric acid, a saturated aqueous sodium bicarbonate solution, and saturated saline. The organic layer was dried over anhydrous sodium sulfate, filtered, and then concentrated under reduced pressure. The resulting residue was purified by silica gel column chromatography (0%-50% ethyl acetate/n-heptane) to obtain the title compound (72.1 mg).

¹H-NMR (500 MHz, CDCl₃) δ (ppm): 0.98 (t, J = 7.3 Hz, 3H), 1.12 (d, J = 6.7 Hz, 3H), 1.54 (s, 3H), 1.74-1.81 (m, 2H), 1.89 (dd, J = 14.7, 7.3 Hz, 2H), 2.00 (dd, J = 14.1, 4.3 Hz, 1H), 2.50 (s, 1H), 2.65 (s, 1H), 2.71 (dd, J = 8.3, 2.1 Hz, 1H), 3.14-3.19 (m, 1H), 5.30 (dt, J = 8.1, 5.1 Hz, 1H), 9.17 (d, J = 2.5 Hz, 2H), 9.24 (t, J = 2.1 Hz, 1H).

### Example A-13

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate

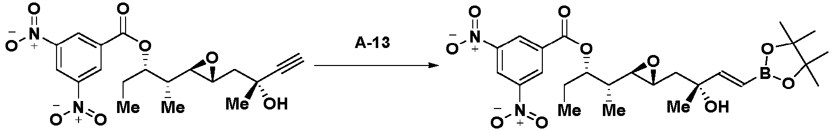

A solution of copper(II) trifluoromethanesulfonate (8.90 mg, 25 µmol), B₂pin₂ (150 mg, 0.591 mmol), sodium t-butoxide (4.73 mg, 49 µmol), and 2,6-di(1-pyrazolyl)pyridine (10.4 mg, 49 µmol) in MeCN (4 mL) and MeOH (60 µL) was stirred at room temperature for 30 minutes (this mixture is referred to as solution A).

(2R,3S)-2-((2R,3R)-3-((R)-2-hydroxy-2-methylbut-3-yn-1-yl)oxiran-2-yl)pentan-3-yl 3,5-dinitrobenzoate (40 mg, 98 µmol) obtained in Example A-12 was dissolved in solution A (810 µL). The resulting reaction mixture was stirred at 50°C for 19 hours. Solution A (810 µL) was added to the reaction mixture, and the reaction mixture was stirred at 50°C for 4 hours. The mixture was allowed to cool to room temperature. Water and ethyl acetate were added to the reaction mixture, and the organic layer was separated. The organic layer was washed sequentially with water and saturated saline. The organic layer was dried over anhydrous sodium sulfate, filtered, and then concentrated under reduced pressure. The resulting residue was purified by silica gel column chromatography (0%-50% ethyl acetate/n-heptane) to obtain the title compound (15.2 mg).

The ¹H-NMR spectrum (500 MHz, CDCl₃) of the obtained compound was consistent with the NMR spectrum of the compound obtained in Example A-10.

### Example A-14

### Synthesis of potassium trifluoro((R,E)-3-hydroxy-4-((2R,3R)-3-((2R,3S)-3-hydroxypentan-2-yl)oxiran-2-yl)-3-methylbut-1-en-1-yl)borate

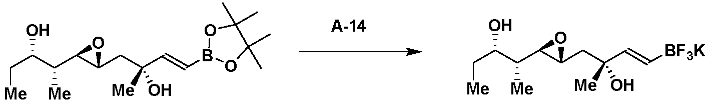

To a solution of (2R,3S)-2-((2R,3R)-3-((RE)-2-hydroxy-2-methyl-4-(4,4,5,5-tetramethyl-1,3,2-dioxaborolan-2-yl)but-3-en-1-yl)oxiran-2-yl)pentan-3-ol (30.7 mg, 0.09 mmol) obtained in Example A-8 in MeOH (0.5 mL), a 4.5 M aqueous potassium hydrogen fluoride solution (140 µL, 0.63 mmol) was added at room temperature. The reaction mixture was stirred at room temperature for 3 hours. A 4.5 M aqueous potassium hydrogen fluoride solution (140 µL, 0.63 mmol) was added to the reaction mixture, and the mixture was further stirred at room temperature for 3 hours. The reaction mixture was concentrated under reduced pressure. Hot acetone was added to the residue and the supernatant was decanted (3 times). The combined supernatant was filtered and the filtrate was concentrated under reduced pressure. The residue was triturated with diethyl ether. The obtained solid was collected by filtration, washed with diethyl ether, and dried under reduced pressure to obtain the title compound (22.2 mg).

¹H-NMR (500 MHz, CDCl₃) δ (ppm): 0.84 (br.d, J = 3.7 Hz, 3H), 0.89-0.97 (m, 3H), 1.32 (br.s, 3H), 1.42-1.61 (m, 3H), 1.64-1.82 (m, 2H), 2.75 (br.s, 1H), 2.90 (br.s, 1H), 3.54-3.72 (m, 2H), 3.91 (br.s, 1H), 5.59 (br.d, J = 16.5 Hz, 1H), 6.02 (br.d, J = 16.5 Hz, 1H).

### Example A-15

### Synthesis of (2R,3 S)-2-((2R,3R)-3-((R)-2-hydroxy-2-methylbut-3-en-1-yl)oxiran-2-yl)pentan-3-ol

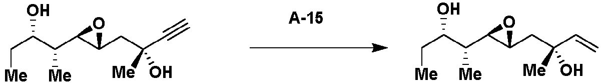

To a solution of (2R,3 S)-2-((2R,3R)-3-((R)-2-hydroxy-2-methylbut-3-yn-1-yl)oxiran-2-yl)pentan-3-ol (680 mg, 3.20 mmol) obtained in Example A-1-(3) in MeOH (13.6 mL), diethylenetriamine (0.348 mL, 3.20 mmol) and 0.3% palladium/boron nitride (34.1 mg, 0.961 µmol; prepared according to Adv. Synth. Catal. 2012, 354, 1264-1268) were added at 25°C. The reaction mixture was stirred under a hydrogen atmosphere at normal pressure at room temperature for 7.25 hours. Ethyl acetate (15 mL) was added to the reaction liquid, and then the catalyst was filtered off through a Millipore filter and washed with ethyl acetate. The filtrate was concentrated under reduced pressure. Ethyl acetate and 5% saline were added to the residue, and the layers were separated. The aqueous layer was subjected to extraction with ethyl acetate (3 times). The combined organic layer was dried over magnesium sulfate, filtered, and then concentrated under reduced pressure. The residue was purified by silica gel column chromatography (30%-60% ethyl acetate/n-heptane) to obtain the title compound (654 mg). The ¹H-NMR spectrum (500 MHz, CDCl₃) of the obtained compound was consistent with the NMR spectrum of the compound obtained in Example A-2.

### Example A-16

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate

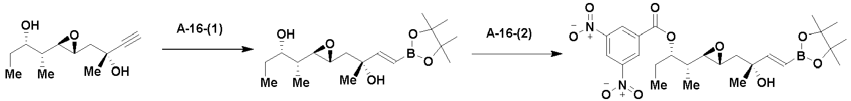

### A-16-(1)

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-ol

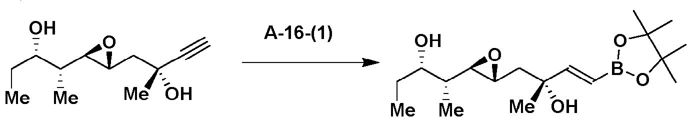

A mixture of (2R,3S)-2-((2R,3R)-3-((R)-2-hydroxy-2-methylbut-3-yn-1-yl)oxiran-2-yl)pentan-3-ol (880 mg, 4.15 mmol) obtained in Example A-1-(3), chloro[1,3-bis(2,4,6-trimethylphenyl)imidazol-2-ylidene]copper(I) (16.7 mg, 0.041 mmol), B2pin2 (1.16 g, 4.56 mmol), and sodium hydroxide (8.29 mg, 0.207 mmol) was purged with nitrogen. To the mixture, cyclopentyl methyl ether (17.6 mL) and MeOH (335 µL) were added. The resulting reaction mixture was stirred at room temperature for 3.4 hours. The reaction mixture was quenched with water and subjected to extraction with ethyl acetate (3 times). The combined organic layer was washed with a saturated aqueous sodium chloride solution, dried over anhydrous sodium sulfate, filtered, and then concentrated under reduced pressure. The resulting residue was purified by silica gel column chromatography (0%-50% ethyl acetate/n-heptane) to obtain the title compound (1.55 g, weight content from NMR: 71.0%). The ¹H-NMR spectrum (500 MHz, CDCl₃) of the mentioned compound as a main component was consistent with the NMR spectrum of the compound obtained in Example A-8.

### A-16-(2)

### Synthesis of (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate

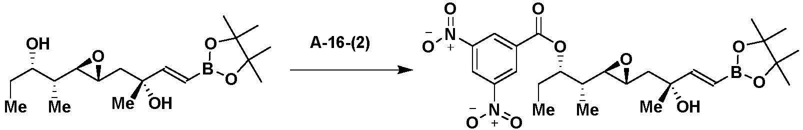

To a solution of (2R,3S)-2-((2R,3R)-3-((RE)-2-hydroxy-2-methyl-4-(4,4,5,5-tetramethyl-1,3,2-dioxaborolan-2-yl)but-3-en-1-yl)oxiran-2-yl)pentan-3-ol (1.55 g, weight content from NMR: 71.0%, 3.23 mmol) obtained in Example A-16-(1), DMAP (79 mg, 647 µmol), and Et₃N (0.901 mL, 6.47 mmol) in DCM (30 mL), 3,5-dinitrobenzoyl chloride (1.04 g, 4.53 mmol) was added at 0°C, and the mixture was stirred at the same temperature for 1 hour. To the reaction mixture, 3,5-dinitrobenzoyl chloride (155 mg, 0.672 mmol) was added, and the mixture was stirred at 0°C for 2 hours. Then, 3,5-dinitrobenzoyl chloride (155 mg, 0.672 mmol) and Et₃N (0.240 mL, 1.72 mmol) were added thereto, and the mixture was stirred at 0°C for 1 hour. The reaction mixture was returned to room temperature, and stirred for 2 hours 40 minutes, then 3,5-dinitrobenzoyl chloride (155 mg, 0.672 mmol) and Et₃N (0.470 mL, 3.37 mmol) were added thereto, and the mixture was stirred at room temperature for 15 minutes. Further, 3,5-dinitrobenzoyl chloride (155 mg, 0.672 mmol) was added thereto, and the mixture was stirred at room temperature for 15 minutes. A saturated aqueous sodium bicarbonate solution was added to the reaction mixture, and the mixture was stirred at room temperature for 20 minutes. Ethyl acetate and water were added to the obtained mixture, and the organic layer was separated. The organic layer was washed sequentially with a saturated aqueous ammonium chloride solution and saturated saline, dried over anhydrous sodium sulfate, filtered, and concentrated under reduced pressure. The resulting residue was purified by silica gel column chromatography (0%-30% ethyl acetate/n-heptane) and concentrated under reduced pressure to obtain a residue (1.39 g) containing the title compound. Ethyl acetate (3.5 mL) and n-heptane (14 mL) were added to this residue, and the residue was dissolved at 100°C. The resulting solution was slowly cooled to room temperature. The precipitated solid was collected by filtration, washed with n-heptane, and dried under reduced pressure to obtain the title compound (920 mg, weight content from NMR: 96.3%) as a solid. The ¹H-NMR spectrum (500 MHz, CDCl₃) of the mentioned compound as a main component was consistent with the NMR spectrum in Example A-10.

### Example A-17

### Synthesis of ((R,E)-4-((2R,3R)-3-((2R,3S)-3-((3,5-dinitrobenzoyl)oxy)pentan-2-yl)oxiran-2-yl)-3-hydroxy-3-methylbut-1-en-1-yl)boronic acid

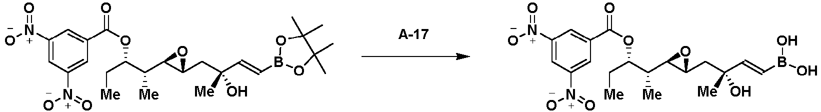

(2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate (24.5 mg, 0.046 mmol) obtained in Example A-10, ammonium acetate (10.6 mg, 0.138 mmol) and sodium periodate (29.4 mg, 0.138 mmol) were dissolved in a mixed solution of acetone (1.0 mL) and water (0.5 mL). The reaction mixture was stirred vigorously at room temperature for 23 hours. To the reaction mixture, ammonium acetate (10.6 mg, 0.138 mmol), sodium periodate (29.4 mg, 0.138 mmol), acetone (1.0 mL), and water (0.5 mL) were added, and the mixture was further stirred for 6 hours. The reaction liquid was filtered to remove a solid and the solid was washed with MTBE. The obtained filtrate was concentrated under reduced pressure. Ethyl acetate and water were added to the residue, and the organic layer was divided. The organic layer was washed with saturated saline and then concentrated under reduced pressure. A solution of the concentrated residue in acetonitrile (0.6 mL) and water (0.3 mL) was stirred at room temperature for 1 hour and then at 70°C for 1 hour. The reaction mixture was slowly cooled to 0°C and water (2.0 mL) was added thereto. The mixture was concentrated under reduced pressure. Toluene was added to the concentrated residue, and the resulting mixture was concentrated again under reduced pressure to obtain the title compound (20.1 mg).

¹H-NMR (500 MHz, DMSO-d6) δ (ppm): 0.89 (t, J = 7.3 Hz, 3H), 1.00 (d, J = 7.3 Hz, 3H), 1.16 (s, 3H), 1.47 (dd, J = 14.1, 6.1 Hz, 1H), 1.61-1.70 (m, 2H), 1.79 (q, J = 7.2 Hz, 2H), 2.63 (dd, J = 8.0, 1.8 Hz, 1H), 2.83 (td, J = 5.7, 2.1 Hz, 1H), 4.65 (s, 1H), 5.14-5.20 (m, 1H), 5.46 (d, J = 17.7 Hz, 1H), 6.45 (d, J = 18.3 Hz, 1H), 7.49 (s, 2H), 8.93 (d, J = 2.5 Hz, 2H), 9.05 (t, J = 2.1 Hz, 1H)

### Example B-1

### Synthesis of (3S,4S,E)-1-iodo-2,4-dimethylhexa-1,5-dien-3-yl (3R,6R,7S)-3-((t-butyldimethylsilyl)oxy)-7-((4-methoxybenzyl)oxy)-6-methyl-6-((triethylsilyl)oxy)non-8-enoate

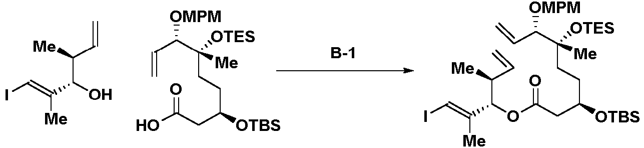

To a solution of (3R,6R,7S)-3-((t-butyldimethylsilyl)oxy)-7-((4-methoxybenzyl)oxy)-6-methyl-6-((triethylsilyl)oxy)non-8-enoic acid (CAS No. 1399679-73-2; Org. Lett. 2012, 14, 4730-4733) (261 mg, 0.46 mmol) synthesized according to the method described in the literature, and Et₃N (90 µL, 0.645 mmol) in THF (10 mL), 2,4,6-trichlorobenzoyl chloride (86 µL, 0.552 mmol) was added under ice cooling, and the reaction mixture was stirred at room temperature for 80 minutes Et₃N (90 µL, 0.645 mmol) and 2,4,6-trichlorobenzoyl chloride (86 µL, 0.552 mmol) were added to the reaction mixture under ice cooling, then the reaction mixture was stirred at room temperature for 2 hours. Et₃N (90 µL, 0.645 mmol) and 2,4,6-trichlorobenzoyl chloride (86 µL, 0.552 mmol) were added to the reaction mixture under ice cooling, then the reaction mixture was stirred at room temperature for 1 hour. Insoluble matter in the reaction mixture was filtered off and the filtrate was concentrated under reduced pressure.

To a solution of the concentrated residue in toluene (4 mL) under ice water, a solution of (3S,4S,E)-1-iodo-2,4-dimethylhexa-1,5-dien-3-ol (CAS No. 952487-46-6; Eur. J. Org. Chem. 2016, 2110-2114) (348 mg, 1.38 mmol) synthesized according to the method described in the literature and DMAP (197 mg, 1.61 mmol) in toluene (6 mL) was added over 5 minutes. The resulting reaction mixture was stirred overnight at room temperature.

MTBE, water, and 0.5 N hydrochloric acid were added to the reaction mixture, and the organic layer was separated. The organic layer was washed sequentially with a saturated aqueous sodium bicarbonate solution and saturated saline. The organic layer was dried over anhydrous sodium sulfate, filtered, and then concentrated under reduced pressure. The resulting residue was purified by silica gel column chromatography (0%-3% ethyl acetate/n-heptane) to obtain a mixture (520 mg) of the title compound and impurities. The reaction mixture was further purified by silica gel column chromatography (0%-3% ethyl acetate/n-heptane) to obtain the title compound (323 mg).

¹H-NMR (500 MHz, CDCl₃) δ (ppm): 0.02 (s, 3H), 0.04 (s, 3H), 0.50-0.61 (m, 6H), 0.86 (s, 9H), 0.88-0.96 (m, 12H), 1.16 (s, 3H), 1.19-1.25 (m, 1H), 1.40-1.53 (m, 2H), 1.69-1.78 (m, 1H), 1.80 (s, 3H), 2.29-2.57 (m, 3H), 3.50 (d, J = 7.6 Hz, 1H), 3.80 (s, 3H), 3.95-4.06 (m, 1H), 4.23 (d, J = 11.7 Hz, 1H), 4.50 (d, J = 11.7 Hz, 1H), 4.97-5.08 (m, 2H), 5.09-5.15 (m, 1H), 5.22 (br.d, J = 17.2 Hz, 1H), 5.31 (d, J = 10.3 Hz, 1H), 5.60-5.73 (m, 1H), 5.76-5.89 (m, 1H), 6.30 (s, 1H), 6.86 (br.d, J = 6.9 Hz, 2H), 7.24 (br.d, J = 6.9 Hz, 2H).

### Example B-2

### Synthesis of (3S,4S,E)-1-iodo-2,4-dimethylhexa-1,5-dien-3-yl(3R,6R,7S)-3-((t-butyldimethylsilyl)oxy)-7-hydroxy-6-methyl-6-((triethylsilyl)oxy)non-8-enoate

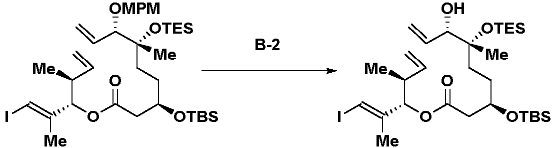

To a solution of (3S,4S,E)-1-iodo-2,4-dimethylhexa-1,5-dien-3-yl (3R,6R,7S)-3-((t-butyldimethylsilyl)oxy)-7-((4-methoxybenzyl)oxy)-6-methyl-6-((triethylsilyl)oxy)non-8-enoate (311 mg, 0.388 mmol) obtained in Example B-1 in DCM (5 mL), 1 M phosphate buffer (pH = 7.0, 0.5 mL) and DDQ (115 mg, 0.505 mmol) were sequentially added. The reaction mixture was stirred at room temperature for 100 minutes. DCM (3 mL), 1 M phosphate buffer (pH = 7.0, 0.3 mL) and DDQ (115 mg, 0.505 mmol) were added to the reaction mixture and the reaction mixture was stirred at room temperature for 1 hour. DCM (1 mL), 1 M phosphate buffer (pH = 7.0, 0.1 mL) and DDQ (25 mg, 0.11 mmol) were then added to the reaction mixture and the reaction mixture was stirred at room temperature for 40 minutes. MTBE, a saturated aqueous sodium bicarbonate solution, and water were added to the reaction mixture, and the organic layer was separated. The organic layer was washed sequentially with water and saturated saline. The organic layer was dried over anhydrous sodium sulfate, filtered, and then concentrated under reduced pressure. The resulting residue was purified by silica gel column chromatography (0%-5% MTBE/n-heptane) to obtain the title compound (249 mg).

¹H-NMR (500 MHz, CDCl₃) δ (ppm): 0.03 (s, 3H), 0.06 (s, 3H), 0.63 (q, J = 7.6 Hz, 6H), 0.87 (s, 9H), 0.90-1.02 (m, 12H), 1.22 (s, 3H), 1.28-1.33 (m, 1H), 1.47-1.54 (m, 1H), 1.55-1.61 (m, 1H), 1.67-1.75 (m, 1H), 1.81 (d, J = 1.4 Hz, 3H), 2.33-2.55 (m, 3H), 2.60 (d, J = 3.4 Hz, 1H), 3.78-3.90 (m, 1H), 4.03 (quin, J = 5.9 Hz, 1H), 4.96-5.10 (m, 2H), 5.13 (d, J = 8.3 Hz, 1H), 5.20 (dd, J = 10.3, 1.4 Hz, 1H), 5.31 (dt, J = 17.2, 1.7 Hz, 1H), 5.57-5.74 (m, 1H), 5.84 (ddd, J = 17.2, 10.7, 6.5 Hz, 1H), 6.32 (s, 1H).

### Example B-3

### Synthesis of (4R,7R,8S,11S,12S,E)-4-((t-butyldimethylsilyl)oxy)-8-hydroxy-12-((E)-1-iodoprop-1-en-2-yl)-7,11-dimethyl-7-((triethylsilyl)oxacyclododec-9-en-2-one

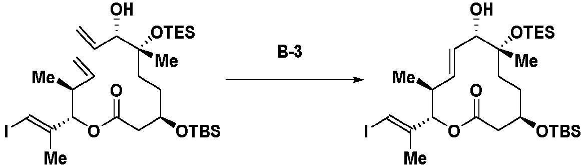

To parabenzoquinone (77 mg, 0.717 mmol), a toluene solution (1,200 mL) of (3S,4S,E)-1-iodo-2,4-dimethylhexa-1,5-dien-3-yl (3R,6R,7S)-3-((t-butyldimethylsilyl)oxy)-7-hydroxy-6-methyl-6-((triethylsilyl)oxy)non-8-enoate (4.88 g, 7.167 mmol) obtained in Example B-2 was added. To this mixture heated to 100°C, a toluene solution (20 mL) of Gmbbs-II (0.61 g, 0.717 mmol) was added dropwise over 70 minutes using a syringe pump. The reaction mixture was cooled to room temperature and the reaction mixture was concentrated under reduced pressure. The resulting residue was purified by silica gel column chromatography (0%-5% ethyl acetate/n-heptane) to obtain the title compound (3.51 g).

¹H-NMR (500 MHz, CDCl₃) δ (ppm): 0.07 (s, 3H), 0.08 (s, 3H), 0.65 (q, J = 7.8 Hz, 6H), 0.89-0.94 (m, 12H), 0.95-1.02 (m, 9H), 1.32 (s, 3H), 1.34-1.43 (m, 2H), 1.45-1.54 (m, 1H), 1.57-1.64 (m, 1H), 1.80 (d, J = 1.2 Hz, 3H), 2.38-2.56 (m, 3H), 2.64 (d, J = 10.9 Hz, 1H), 3.56 (t, J = 10.0 Hz, 1H), 3.76-3.85 (m, 1H), 5.12 (d, J = 10.9 Hz, 1H), 5.36 (dd, J = 14.9, 9.7 Hz, 1H), 5.60 (dd, J = 14.9, 9.7 Hz, 1H), 6.45 (s, 1H).

### Example B-4

### Synthesis of (2S,3S,6S,7R,10R,E)-10-((t-butyldimethylsilyl)oxy)-2-((E)-1-iodoprop-1-en-2-yl)-3,7-dimethyl-12-oxo-7-((triethylsilyl)oxy)oxacyclododec-4-en-6-yl acetate

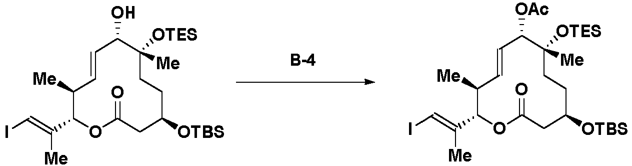

To a solution of (4R,7R,8S,11S,12S,E)-4-((t-butyldimethylsilyl)oxy)-8-hydroxy-12-((E)-1-iodoprop-1-en-2-yl)-7,11-dimethyl-7-((triethylsilyl)oxacyclododec-9-en-2-one (110 mg, 0.169 mmol) obtained in Example B-3 in pyridine (3 mL), Ac₂O (1.45 mL, 15.3 mmol) was added. The resulting reaction mixture was stirred overnight at room temperature. The reaction mixture was concentrated under reduced pressure. The resulting residue was purified by silica gel column chromatography (0%-4% ethyl acetate/n-heptane) to obtain the title compound (113 mg).

¹H-NMR (500 MHz, CDCl₃) δ (ppm): 0.06 (s, 3H), 0.07 (s, 3H), 0.59-0.66 (m, 6H), 0.90 (s, 12H), 0.99 (t, J = 7.6 Hz, 9H), 1.21 (s, 3H), 1.31-1.35 (m, 1H), 1.37-1.49 (m, 2H), 1.58-1.70 (m, 1H), 1.81 (s, 3H), 2.06 (s, 3H), 2.35-2.53 (m, 3H), 3.66-3.89 (m, 1H), 4.97 (d, J = 9.8 Hz, 1H), 5.11 (d, J = 11.0 Hz, 1H), 5.56 (dd, J = 15.3, 9.8 Hz, 1H), 5.66 (dd, J = 15.9, 9.8 Hz, 1H), 6.45 (s, 1H).

### Example B-5

### Synthesis of (4R,7R,8S,11S,12SX)-4-((t-butyldimethylsilyl)oxy)-7,8-dihydroxy-12-((E)-1-iodoprop-1-en-2-yl)-7,11-dimethyloxacyclododec-9-en-2-one

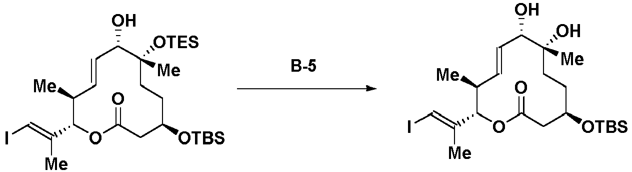

To a solution of (4R,7R,8S,11S,12S,E)-4-((t-butyldimethylsilyl)oxy)-8-hydroxy-12-((E)-1-iodoprop-1-en-2-yl)-7,11-dimethyl-7-((triethylsilyl)oxacyclododec-9-en-2-one (260 mg, 0.398 mmol) obtained in Example B-3 in THF (10 mL), AcOH (30 µL, 0.518 mmol) and a THF solution of TBAF (1 M; 0.518 mL, 0.518 mmol) were sequentially added under ice water. The resulting mixture was stirred at room temperature for 3 hours 30 minutes. The reaction mixture was quenched with a saturated aqueous sodium bicarbonate solution under ice cooling. MTBE was added to the mixture, and the organic layer was separated. The aqueous layer was subjected to extraction with MTBE. The combined organic layer was washed with saturated saline. The organic layer was dried over anhydrous sodium sulfate, filtered, and then concentrated under reduced pressure. The resulting residue was purified by silica gel column chromatography (0%-30% ethyl acetate/n-heptane) to obtain the title compound (208 mg).

¹H-NMR (500 MHz, CDCl₃) δ (ppm): 0.07 (s, 3H), 0.08 (s, 3H), 0.85-0.94 (m, 12H), 1.30 (s, 3H), 1.34-1.47 (m, 3H), 1.58-1.65 (m, 1H), 1.79 (s, 3H), 1.85 (d, J = 4.3 Hz, 1H), 2.30-2.60 (m, 4H), 3.72-3.91 (m, 2H), 5.12 (d, J = 10.4 Hz, 1H), 5.43 (dd, J = 15.3, 9.8 Hz, 1H), 5.73 (dd, J = 15.3, 9.8 Hz, 1H), 6.45 (s, 1H)

### Example B-6

### Synthesis of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclodec-4-en-6-yl acetate

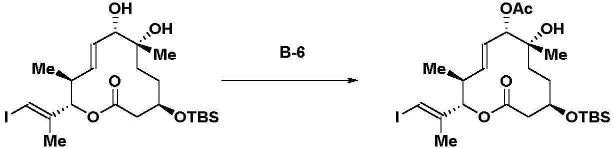

To a solution of (4R,7R,8S,11S,12S,E)-4-((t-butyldimethylsilyl)oxy)-7,8-dihydroxy-12-((E)-1-iodoprop-1-en-2-yl)-7,11-dimethyloxacyclododec-9-en-2-one (144 mg, 0.268 mmol) obtained in Example B-5 and DMAP (6.54 mg, 54 µmol) in DCM (4 mL), Et₃N (0.112 mL, 0.803 mmol) and Ac₂O (27 µL, 0.281 mmol) were sequentially added under ice cooling. The reaction mixture was stirred at the same temperature for 1 hour. The reaction mixture was quenched with a saturated aqueous sodium bicarbonate solution. MTBE was added to the obtained mixture, and the organic layer was separated. The organic layer was washed with saturated saline. The organic layer was dried over anhydrous sodium sulfate, filtered, and then concentrated under reduced pressure. The resulting residue was purified by silica gel column chromatography (0%-20% ethyl acetate/n-heptane) to obtain the title compound (149 mg).

¹H-NMR (500 MHz, CDCl₃) δ (ppm): 0.06 (s, 3H), 0.08 (s, 3H), 0.80-0.96 (m, 12H), 1.21 (s, 3H), 1.30-1.48 (m, 3H), 1.61-1.72 (m, 1H), 1.78 (d, J = 1.2 Hz, 3H), 2.08 (s, 1H), 2.09 (s, 3H), 2.35-2.43 (m, 1H), 2.43-2.56 (m, 2H), 3.84 (dq, J = 8.3, 4.2 Hz, 1H), 5.06 (d, J = 9.8 Hz, 1H), 5.09 (d, J = 10.4 Hz, 1H), 5.60 (dd, J = 15.3, 9.8 Hz, 1H), 5.68 (dd, J = 15.3, 9.8 Hz, 1H), 6.46 (d, J = 1.2 Hz, 1H).

### Example B-7

### Synthesis of (4R,7R,8S,11S,12S,E)-4,7,8-trihydroxy-12-((E)-1-iodoprop-1-en-2-yl)-7,11-dimethyloxacyclododec-9-en-2-one

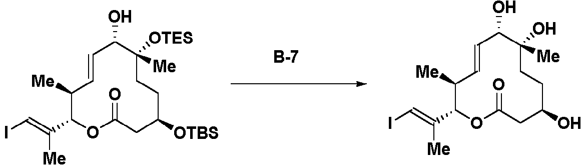

To a solution of (4R,7R,8S,11S,12S,E)-4-((t-butyldimethylsilyl)oxy)-8-hydroxy-12-((E)-1-iodoprop-1-en-2-yl)-7,11-dimethyl-7-((triethylsilyl)oxacyclododec-9-en-2-one (600 mg, 0.919 mmol) obtained in Example B-3 in THF (6 mL), a THF solution of TBAF (1 M; 5.51 mL, 5.51 mmol) was added under ice water. The resulting mixture was stirred overnight at room temperature. The reaction mixture was quenched with a saturated aqueous ammonium chloride solution. Ethyl acetate and saturated saline were added to the obtained mixture, and the organic layer was separated. The aqueous layer was subjected to extraction with ethyl acetate twice. The combined organic layer was dried over anhydrous sodium sulfate, filtered, and then concentrated under reduced pressure. The resulting residue was purified by silica gel column chromatography (0%-80% ethyl acetate/n-heptane) to obtain the title compound (312 mg).

¹H-NMR (500 MHz, CDCl₃) δ (ppm): 0.92 (d, J = 6.7 Hz, 3H), 1.15-1.27 (m, 1H), 1.31 (s, 3H), 1.35-1.52 (m, 2H), 1.69 (tt, J = 13.3, 3.8 Hz, 1H), 1.80-1.87 (m, 4H), 2.36 (s, 1H), 2.46-2.59 (m, 2H), 2.61-2.69 (m, 1H), 3.39 (d, J = 11.0 Hz, 1H), 3.69-3.78 (m, 1H), 3.81 (dd, J = 9.8, 4.3 Hz, 1H), 5.30 (d, J = 10.4 Hz, 1H), 5.39 (dd, J = 15.0, 10.1 Hz, 1H), 5.75 (dd, J = 15.3, 9.8 Hz, 1H), 6.48 (d, J = 1.2 Hz, 1H).

### Example B-8

### Synthesis of (2S,3 S,6S,7R,10R,E)-7,10-dihydroxy-2-((E)-1-iodoprop-1-en-2-yl)-3,7-dimethyl-12-oxooxacyclodec-4-en-6-yl acetate

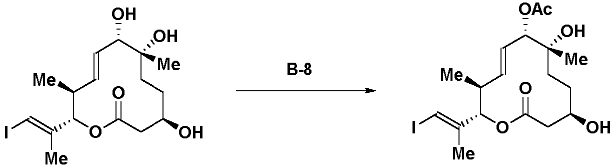

To a solution of (4R,7R,8S,11S,12S,E)-4,7,8-trihydroxy-12-((E)-1-iodoprop-1-en-2-yl)-7,11-dimethyloxacyclododec-9-en-2-one (220 mg, 0.519 mmol) obtained in Example B-7 and DMAP (12.7 mg, 0.104 mmol) in DCM (5.5 mL), Et₃N (0.216 mL, 1.56 mmol) and Ac₂O (51 µL, 0.544 mmol) were sequentially added under ice cooling. The reaction mixture was stirred at the same temperature for 1 hour. The reaction liquid was quenched with a saturated aqueous sodium bicarbonate solution. Ethyl acetate was added to the obtained mixture, and the organic layer was separated. The organic layer was washed three times with water. The combined aqueous layer was subjected to re-extraction with ethyl acetate. The ethyl acetate layer obtained by re-extraction was washed sequentially with water and saturated saline. All combined organic layer was dried over anhydrous sodium sulfate, filtered, and then concentrated under reduced pressure. The resulting residue was purified by silica gel column chromatography (30%-80% ethyl acetate/n-heptane) to obtain the title compound (200 mg).

¹H-NMR (500 MHz, CDCl₃) δ (ppm): 0.90 (d, J = 6.8 Hz, 3H), 1.21 (s, 3H), 1.23-1.33 (m, 2H), 1.38 (td, J = 13.2, 3.9 Hz, 1H), 1.65-1.74 (m, 1H), 1.83 (d, J = 1.0 Hz, 3H), 2.07 (s, 1H), 2.09 (s, 3H), 2.44-2.69 (m, 3H), 3.40 (d, J = 10.7 Hz, 1H), 3.71-3.81 (m, 1H), 5.07 (d, J = 9.3 Hz, 1H), 5.31 (d, J = 10.7 Hz, 1H), 5.58 (dd, J = 15.1, 9.3 Hz, 1H), 5.69 (dd, J = 15.1, 9.3 Hz, 1H), 6.48 (d, J = 1.5 Hz, 1H).

### Example B-9

### Synthesis of (2S,3S,6S,7R,10R,E)-7,10-dihydroxy-3,7-dimethyl-12-oxo-2-((E)-penta-2,4-dien-2-yl)oxacyclododec-4-en-6-yl acetate

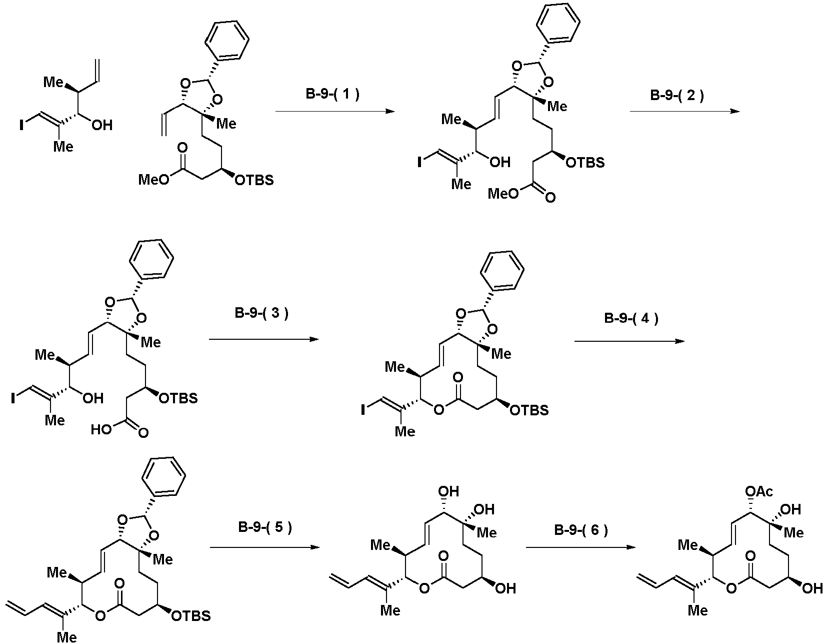

### B-9-(1)

### Synthesis of methyl (R)-3-((t-butyldimethylsilyl)oxy)-5-((2S,4R,5S)-5-((1E,3S,4S,5E)-4-hydroxy-6-iodo-3,5-dimethylhexa-1,5-dien-1-yl)-4-methyl-2-phenyl-1,3-dioxolan-4-yl)pentanoate

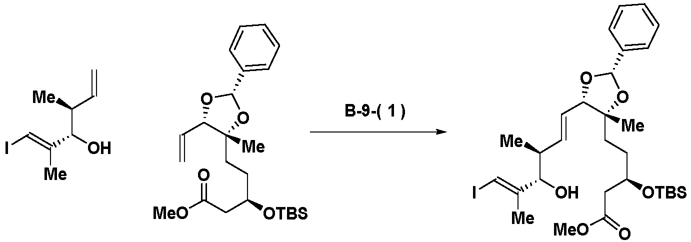

A solution of Hoveyda-Grubbs-111 (24 mg, 38 µmol) in toluene (7.8 mL) was prepared. This is referred to as liquid C.

Liquid C (2.6 mL) was added to a solution of methyl (R)-3-((t-butyldimethylsilyl)oxy)-5-((2S,4R,5S)-4-methyl-2-phenyl-5-vinyl-1,3-dioxolan-4-yl)pentanoate (CAS No. 934497-57-1; Angew. Chem. Int. Ed. 2007, 46, 4350-4355) (178 mg, 0.410 mmol) synthesized according to the method described in the literature, and (3S,4S,E)-1-iodo-2,4-dimethylhexa-1,5-dien-3-ol (CAS No. 952487-46-6; Eur. J. Org. Chem. 2016, 2110-2114) (206 mg, 0.819 mmol) synthesized according to the method described in the literature in toluene (13 mL). The reaction mixture was stirred at 110°C for 4 hours under nitrogen bubbling. Liquid C (1.3 mL) was added to the reaction mixture, and the mixture was stirred for 2 hours under the same conditions. Liquid C (1.3 mL) was then added to the reaction mixture, and the mixture was stirred for 4 hours under the same conditions. Liquid C (2.6 mL) was further added to the reaction mixture, and the mixture was stirred at 100°C for 13.5 hours under nitrogen bubbling. The reaction mixture was cooled to room temperature. The reaction mixture was directly purified by silica gel column chromatography (NH silica gel, 0%-30% ethyl acetate/n-heptane) to obtain the title compound (234 mg).

¹H-NMR (500 MHz, CDCl₃) δ (ppm): 0.01 (s, 3H), 0.03 (s, 3H), 0.85 (s, 9H), 0.98 (d, J = 7.3 Hz, 3H), 1.32 (s, 3H), 1.56-1.75 (m, 4H), 1.81 (d, J = 1.2 Hz, 3H), 1.86 (d, J = 3.7 Hz, 1H), 2.32-2.38 (m, 1H), 2.40-2.46 (m, 2H), 3.64 (s, 3H), 3.93 (dd, J = 7.0, 4.0 Hz, 1H), 4.07-4.13 (m, 1H), 4.27 (d, J = 7.3 Hz, 1H), 5.62 (dd, J = 15.6, 7.6 Hz, 1H), 5.80 (dd, J = 15.3, 8.0 Hz, 1H), 5.90 (s, 1H), 6.26 (s, 1H), 7.34-7.39 (m, 3H), 7.47-7.49 (m, 2H).

### B-9-(2)

### Synthesis of (R)-3-((t-butyldimethylsilyl)oxy)-5-((2S,4R,5S)-5-((1E,3S,4S,5E)-4-hydroxy-6-iodo-3,5-dimethylhexa-1,5-dien-1-yl)-4-methyl-2-phenyl-1,3-dioxolan-4-yl)pentanoic acid

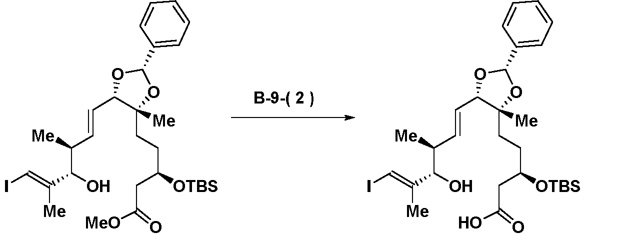

Lithium hydroxide (149 mg, 3.55 mmol) was added to a solution of methyl (R)-3-((t-butyldimethylsilyl)oxy)-5-((2S,4R,5S)-5-((1E,3S,4S,5E)-4-hydroxy-6-iodo-3,5-dimethylhexa-1,5-dien-1-yl)-4-methyl-2-phenyl-1,3-dioxolan-4-yl)pentanoate (234 mg, 0.355 mmol) obtained in Example B-9-(1) in THF (5 mL) and water (2.5 mL). The reaction mixture was sonicated to dissolve lithium hydroxide. The mixture was stirred at room temperature for 3 days. To the reaction mixture, 0.5 N hydrochloric acid and ethyl acetate were added, and the organic layer was separated. The aqueous layer was subjected to re-extraction with ethyl acetate. The combined organic layer was washed with saturated saline, dried over anhydrous magnesium sulfate, and then filtered. The filtrate was concentrated under reduced pressure to obtain the title compound (251 mg). This crude product was used in the subsequent reaction without further purification.

¹H-NMR (500 MHz, CDCl₃) δ (ppm): 0.07 (s, 6H), 0.88 (s, 9H), 0.96 (d, J = 6.7 Hz, 3H), 1.32 (s, 3H), 1.35-1.38 (m, 1H), 1.59-1.66 (m, 3H), 1.73-1.80 (m, 1H), 1.82 (d, J = 1.2 Hz, 3H), 2.39-2.46 (m, 1H), 2.48 (d, J = 4.9 Hz, 2H), 3.93 (d, J = 7.3 Hz, 1H), 4.03 (quin, J = 5.7 Hz, 1H), 4.28 (d, J = 7.3 Hz, 1H), 5.59 (dd, J = 15.3, 7.3 Hz, 1H), 5.77 (dd, J = 15.9, 8.0 Hz, 1H), 5.91 (s, 1H), 6.25 (s, 1H), 7.34-7.39 (m, 3H), 7.45-7.49 (m, 2H).

### B-9-(3)

### Synthesis of (2S,3aS,6S,7S,11R,13aR,E)-11-((t-butyldimethylsilyl)oxy)-7-((E)-1-iodoprop-1-en-2-yl)-6,13a-dimethyl-2-phenyl-3a,6,7,10,11,12,13,13a-octahydro-9H-[1,3]dioxolo[4,5-f][1]oxacyclododecin-9-one

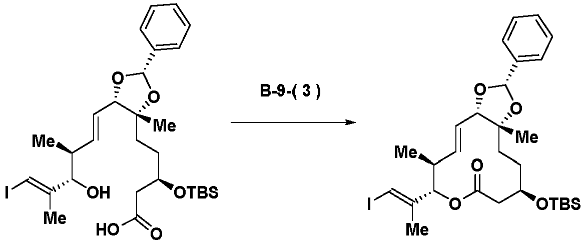

To a solution of 2-methyl-6-nitrobenzoic anhydride (351 mg, 1.02 mmol) and DMAP (249 mg, 2.04 mmol) in toluene (40 mL) stirred at 120°C, a solution of (R)-3-((t-butyldimethylsilyl)oxy)-5-((2S,4R,5S)-5-((1E,3S,4S,5E)-4-hydroxy-6-iodo-3,5-dimethylhexa-1,5-dien-1-yl)-4-methyl-2-phenyl-1,3-dioxolan-4-yl)pentanoic acid (219 mg, 0.340 mmol) obtained in Example B-9-(2) in toluene (10 mL) was added dropwise over 5 hours using a syringe pump. After completion of the dropwise addition, the reaction mixture was stirred at the same temperature for 1 hour. The reaction mixture was cooled to room temperature. The reaction mixture was directly purified twice by silica gel column chromatography (silica gel, 0%-15% ethyl acetate/n-heptane) to obtain the title compound (188 mg).

¹H-NMR (500 MHz, CDCl₃) δ (ppm): 0.08 (s, 3H), 0.09 (s, 3H), 0.89 (s, 9H), 0.89-0.90 (m, 3H), 1.32-1.37 (m, 1H), 1.38 (s, 3H), 1.40-1.47 (m, 1H), 1.63 (br.t, J = 11.6 Hz, 1H), 1.81 (d, J = 1.2 Hz, 3H), 2.04 (td, J = 12.7, 7.6 Hz, 1H), 2.33 (dd, J = 14.4, 10.1 Hz, 1H), 2.53-2.63 (m, 2H), 3.93-3, 99 (m, 1H), 4.18 (d, J = 9.8 Hz, 1H), 5.10 (d, J = 10.4 Hz, 1H), 5.38 (dd, J = 15.0, 9.5 Hz, 1H), 5.65 (dd, J = 15.3, 9.8 Hz, 1H), 5.91 (s, 1H), 6.47 (s, 1H), 7.36-7.40 (m, 3H), 7.48-7.51 (m, 2H).

### B-9-(4)

### Synthesis of (2S,3aS,6S,7S,11R,13aR,E)-11-((t-butyldimethylsilyl)oxy)-6,13a-dimethyl-7-((E)-penta-2,4-dien-2-yl)-2-phenyl-3a,6,7,10,11,12,13,13a-octahydro-9H-[1,3]dioxolo[4,5-f][1]oxacyclododecin-9-one (CAS No. 934497-98-0)

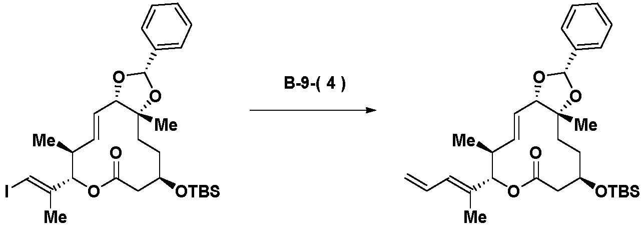

To a solution of (2S,3aS,6S,7S,11R,13aR,E)-11-((t-butyldimethylsilyl)oxy)-7-((E)-1-iodoprop-1-en-2-yl)-6,13a-dimethyl-2-phenyl-3a,6,7,10,11,12,13,13a-octahydro-9H-[1,3]dioxolo[4,5-f][1]oxacyclododecin-9-one (70.8 mg, 0.113 mmol) obtained in Example B-9-(3) and 2-vinyl-4,4,5,5-tetramethyl-1,3,2-dioxaborolane (70.0 mg, 0.452 mmol) in THF (2 mL) and water (0.2 mL), Ag₂O (131 mg, 0.565 mmol) and Pd(dppf)Cl₂ (16.5 mg, 23 µmol) were added. The reaction mixture was stirred at room temperature for 1 hour. Insoluble matter in the reaction mixture was filtered off through Celite^{™} and washed with ethyl acetate. The filtrate was concentrated under reduced pressure. The residue was purified by silica gel column chromatography (silica gel, 0%-10% ethyl acetate/n-heptane) to obtain the title compound (57.4 mg).

¹H-NMR (500 MHz, CDCl₃) δ (ppm): 0.07 (s, 3H), 0.09 (s, 3H), 0.88 (s, 9H), 0.90 (s, 3H), 1.26-1.33 (m, 1H), 1.39 (s, 3H), 1.41 (br.d, J = 8.0 Hz, 1H), 1.61-1.67 (m, 1H), 1.74 (s, 3H), 2.01-2.09 (m, 1H), 2.31 (dd, J = 14.7, 10.4 Hz, 1H), 2.54-2.64 (m, 2H), 3.94-4.02 (m, 1H), 4.19 (d, J = 9.2 Hz, 1H), 4.98 (d, J = 10.4 Hz, 1H), 5.17 (dd, J = 10.4, 1.2 Hz, 1H), 5.27 (dd, J = 16.8, 1.5 Hz, 1H), 5.42 (dd, J = 15.3, 9.8 Hz, 1H), 5.64 (dd, J = 15.0, 9.5 Hz, 1H), 5.91 (s, 1H), 6.13 (d, J = 11.0 Hz, 1H), 6.54 (dt, J = 16.7, 10.6 Hz, 1H), 7.35-7.41 (m, 3H), 7.48-7.53 (m, 2H).

### B-9-(5)

### Synthesis of (4R,7R,8S,11S,12S,E)-4,7,8-trihydroxy-7,11-dimethyl-12-((E)-penta-2,4-dien-2-yl)oxacyclododec-9-en-2-one (CAS No. 934497-99-1)

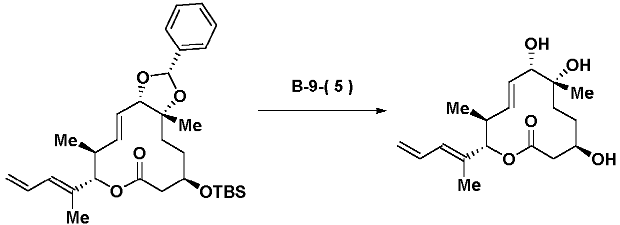

PPTS (180 mg, 0.717 mmol) was added to a solution of (2S,3aS,6S,7S,11R,13aR,E)-11-((t-butyldimethylsilyl)oxy)-6,13a-dimethyl-7-((E)-penta-2,4-dien-2-yl)-2-phenyl-3a,6,7,10,11,12,13,13a-octahydro-9H-[1,3]dioxolo[4,5-f][1]oxacyclododecin-9-one (40.4 mg, 77 µmol) obtained in Example B-9-(4) in MeOH (2 mL). The reaction mixture was stirred at room temperature for 1 day. To the reaction mixture, MeOH (6 mL) was added, and the reaction mixture was stirred at room temperature for 2 days. MeOH (6 mL) was further added to the reaction mixture, and the mixture was stirred at room temperature for 4 days. The reaction liquid was concentrated under reduced pressure, and the resulting residue was purified by silica gel column chromatography (silica gel, 0%-100% ethyl acetate/n-heptane) to obtain the title compound (17.5 mg).

¹H-NMR (500 MHz, CDCl₃) δ (ppm)0.92 (dd, J = 6.7, 1.2 Hz, 3H), 1.31 (d, J = 1.2 Hz, 3H), 1.40 (td, J = 13.5, 3.7 Hz, 1H), 1.49 (br.t, J = 13.5 Hz, 1H), 1.65-1.73 (m, 1H), 1.76 (s, 3H), 1.82 (br.d, J = 3.1 Hz, 1H), 2.38 (s, 1H), 2.50-2.58 (m, 2H), 2.60-2.66 (m, 1H), 3.53 (d, J = 10.4 Hz, 1H), 3.72-3.78 (m, 1H), 3.82 (br.dd, J = 9.8, 2.5 Hz, 1H), 5.19 (br.t, J = 11.6 Hz, 2H), 5.26 (s, 1H), 5.30 (s, 1H), 5.40-5.48 (m, 1H), 5.70-5.77 (m, 1H), 6.12 (br.d, J = 11.0 Hz, 1H), 6.51-6.61 (m, 1H).

### B-9-(6)

### Synthesis of (2S,3S,6S,7R,10R,E)-7,10-dihydroxy-3,7-dimethyl-12-oxo-2-((E)-penta-2,4-dien-2-yl)oxacyclododec-4-en-6-yl acetate (CAS No. 934498-00-7)

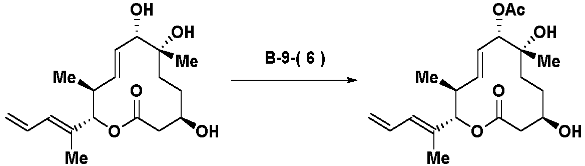

Ac₂O (2.3 µL, 25 µmol) was added to a solution of (4R,7R,8S,11S,12S,E)-4,7,8-trihydroxy-7,11-dimethyl-12-((E)-penta-2,4-dien-2-yl)oxacyclododec-9-en-2-one (8.0 mg, 25 µmol) obtained in Example B-9-(5), triethylamine (3.4 µL, 25 µmol), and DMAP (0.6 mg, 5 µmol) in DCM (1 ml). The reaction mixture was stirred at room temperature for 1 day. The reaction liquid was concentrated under reduced pressure, and the residue was purified by silica gel column chromatography (silica gel, 0%-100% ethyl acetate/n-heptane) to obtain the title compound (5.4 mg).

¹H-NMR (500 MHz, CDCl₃) δ (ppm): 0.91 (d, J = 6.7 Hz, 3H), 1.22 (s, 3H), 1.29-1.47 (m, 3H), 1.51-1.56 (m, 1H), 1.67-1.75 (m, 1H), 1.76 (s, 3H), 2.10 (s, 3H), 2.49-2.58 (m, 2H), 2.64 (dd, J = 15.0, 3.4 Hz, 1H), 3.53 (d, J= 11.0 Hz, 1H), 3.76 (ddt, J = 10.9, 7.3, 3.4 Hz, 1H), 5.10 (d, J = 9.2 Hz, 1H), 5.16-5.23 (m, 2H), 5.23-5.36 (m, 1H), 5.62 (dd, J = 15.3, 9.8, 1H), 5.68 (dd, J = 15.3, 9.2 Hz, 1H), 6.13 (d, J = 11.0 Hz, 1H), 6.56 (dt, J = 17.0, 10.5 Hz, 1H).

Pladienolide D can be synthesized by a cross-metathesis reaction of the obtained compound with the compound of Example A-2, in accordance with the method described in the literature (Angew. Chem. Int. Ed. 2007, 46, 4350-4355).

### Example C-1

### Synthesis of pladienolide D

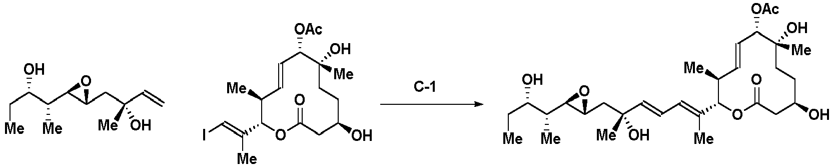

A solution of (2S,3S,6S,7R,10R,E)-7,10-dihydroxy-2-((E)-1-iodoprop-1-en-2-yl)-3,7-dimethyl-12-oxooxacyclodec-4-en-6-yl acetate (5.37 mg, 12 µmol) obtained in Example B-8 and (2R,3S)-2-((2R,3R)-3-((R)-2-hydroxy-2-methylbut-3-en-1-yl)oxiran-2-yl)pentan-3-ol (4.94 mg, 23 µmol) obtained in Example A-2 in DMF (0.8 mL) was degassed and then replaced with a nitrogen atmosphere. To this mixture, Pd(OAc)₂ (0.65 mg, 2.9 µmol) and Ag₂CO₃ (4.13 mg, 15 µmol) were added at room temperature. The resulting reaction mixture was stirred at room temperature for 15 minutes and then at 60°C for 6 hours. After the reaction mixture was allowed to cool, insoluble matter in the reaction mixture was filtered off through a Millipore filter. The filtrate was concentrated under reduced pressure. The residue was purified by silica gel column chromatography (0%-5% MeOH/ethyl acetate) to obtain the title compound (4.7 mg).

¹H-NMR (500 MHz, CD₃OD) δ (ppm): 0.88 (d, J = 6.9 Hz, 3H), 0.90 (d, J = 6.9 Hz, 3H), 0.94 (t, J = 7.5 Hz, 3H), 1.19 (s, 3H), 1.25-1.30 (m, 1H), 1.34 (s, 3H), 1.37-1.71 (m, 7H), 1.78 (d, J = 1.2 Hz, 3H), 1.87 (dd, J = 14.0, 5.4 Hz, 1H), 2.06 (s, 3H), 2.50-2.55 (m, 2H), 2.55-2.62 (m, 1H), 2.67 (dd, J = 8.0, 2.3 Hz, 1H), 2.90 (td, J = 5.7, 2.3 Hz, 1H), 3.53 (dt, J = 8.7, 4.5 Hz, 1H), 3.79 (br.dd, J = 9.7, 4.0 Hz, 1H), 5.03-5.09 (m, 2H), 5.57 (dd, J = 15.5, 9.7 Hz, 1H), 5.70 (dd, J = 14.9, 9.7 Hz, 1H), 5.87 (d, J = 14.9 Hz, 1H), 6.14 (dd, J = 10.9, 1.2 Hz, 1H), 6.53 (dd, J = 15.5, 10.9 Hz, 1H).

### Example C-2

### Synthesis of pladienolide D

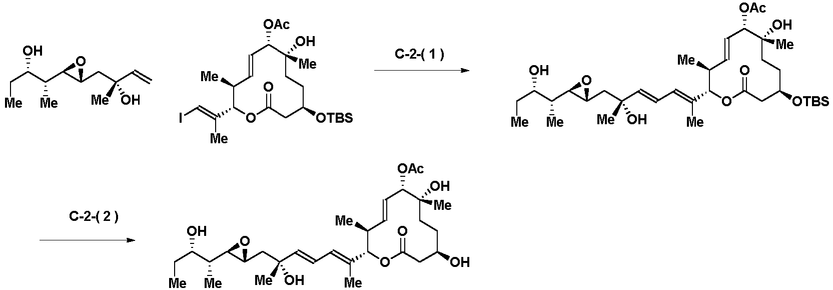

### C-2-(1)

### Synthesis of (2S,3S,6S,7R,10R,E)-10-((t-butyldimethylsilyl)oxy)-7-hydroxy-2-((R,2E,4E)-6-hydroxy-7-((2R,3R)-3-((2R,3S)-3-hydroxypentan-2-yl)oxiran-2-yl)-6-methylhepta-2,4-dien-2-yl)-3,7-dimethyl-12-oxooxacyclododec-4-en-6-yl acetate

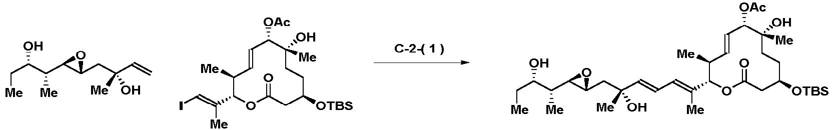

A DMF solution (1 mL) of (2R,3S)-2-((2R,3R)-3-((R)-2-hydroxy-2-methylbut-3-en-1-yl)oxiran-2-yl)pentan-3-ol (10.8 mg, 50 µmol) obtained in Example A-2 and (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclodec-4-en-6-yl acetate (14.5 mg, 25 µmol) obtained in Example B-6 was degassed and then replaced with a nitrogen atmosphere. To this mixture, Pd(OAc)₂ (1.4 mg, 6.25 µmol) and Ag₂CO₃ (8.96 mg, 33 µmol) were added. The resulting reaction mixture was stirred at 80°C for 90 minutes. Ethyl acetate and water were added to the reaction mixture. Insoluble matter in the mixture was filtered off. Then, the organic layer of the filtrate was separated. The aqueous layer was subjected to extraction with ethyl acetate. The combined organic layer was washed with a 10% aqueous sodium chloride solution (twice), dried over anhydrous sodium sulfate, filtered, and then concentrated under reduced pressure. The resulting residue was purified by silica gel column chromatography (0%-70% ethyl acetate/n-heptane) to obtain the title compound (12.5 mg).

¹H-NMR (500 MHz, CDCl₃) δ (ppm): 0.06 (s, 3H), 0.07 (s, 3H), 0.88-0.91 (m, 15H), 0.96 (t, J = 7.3 Hz, 3H), 1.21 (s, 3H), 1.28-1.33 (m, 1H), 1.36 (s, 3H), 1.37-1.54 (m, 5H), 1.58-1.65 (m, 1H), 1.72-1.77 (m, 4H), 1.80-1.86 (m, 1H), 2.02 (br.s, 1H), 2.09 (s, 1H), 2.10 (s, 3H), 2.32 (br.s, 1H), 2.36-2.42 (m, 1H), 2.42-2.60 (m, 2H), 2.75 (dd, J = 6.1, 2.5 Hz, 1H), 2.98 (td, J = 6.1, 2.5 Hz, 1H), 3.65 (br.s, 1H), 3.79-3.93 (m, 1H), 4.96 (d, J = 10.4 Hz, 1H), 5.07 (d, J = 8.6 Hz, 1H), 5.56-5.74 (m, 2H), 5.81 (d, J = 15.3 Hz, 1H), 6.14 (d, J = 11.0 Hz, 1H), 6.50 (dd, J = 15.0, 10.7 Hz, 1H).

### C-2-(2)

### Synthesis of pladienolide D

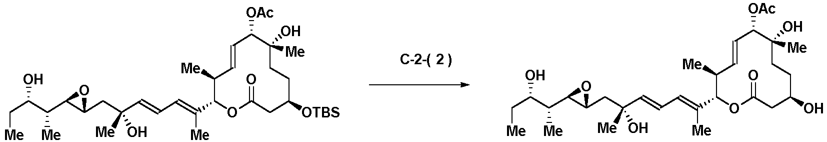

To a THF solution (0.6 mL) of (2S,3S,6S,7R,10R,E)-10-((t-butyldimethylsilyl)oxy)-7-hydroxy-2-((R,2E,4E)-6-hydroxy-7-((2R,3R)-3-((2R,3S)-3-hydroxypentan-2-yl)oxiran-2-yl)-6-methylhepta-2,4-dien-2-yl)-3,7-dimethyl-12-oxooxacyclododec-4-en-6-yl acetate (10.5 mg, 16 µmol) obtained in Example C-2-(1), a THF solution of TBAF (1 M; 47 µL, 47 µmol) was added. The resulting reaction mixture was stirred overnight at room temperature. Ethyl acetate, a saturated aqueous ammonium chloride solution, and water were added to the ice-cooled reaction mixture, and the organic layer was separated. The aqueous layer was subjected to extraction with ethyl acetate. The combined organic layer was dried over anhydrous sodium sulfate, filtered, and then concentrated under reduced pressure. The resulting residue was purified by silica gel column chromatography (0%-100% ethyl acetate/n-heptane) to obtain the title compound (7.5 mg).

The ¹H-NMR spectrum (500 MHz, CD₃OD) of the obtained compound was consistent with the NMR spectrum in Example C-1.

### Example C-3

### Synthesis of pladienolide D

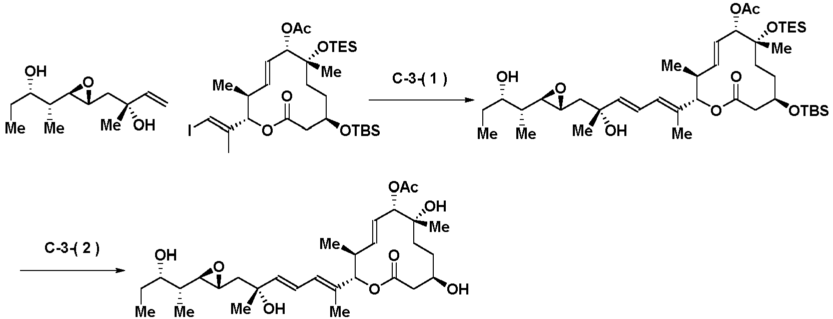

### C-3-(1)

### Synthesis of (2S,3S,6S,7R,10R,E)-10((t-butyldimethylsilyl)oxy)-2-((R,2E,4E)-6-hydroxy-7-((2R,3R)-3-((2R,3S)-3-hydroxypentan-2-yl)oxiran-2-yl)-6-methylhepta-2,4-dien-2-yl)-3,7-dimethyl-12-oxo-7-((triethylsilyl)oxy)oxacyclododec-4-en-6-yl acetate

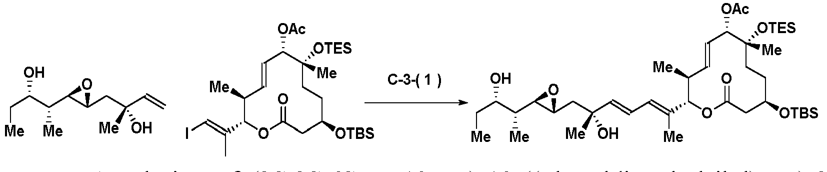

A solution of (2S,3S,6S,7R,10R,E)-10-((t-butyldimethylsilyl)oxy)-2-((E)-1-iodoprop-1-en-2-yl)-3,7-dimethyl-12-oxo-7-((triethylsilyl)oxy)oxacyclododec-4-en-6-yl acetate (12.5 mg, 18 µmol) obtained in Example B-4 and (2R,3S)-2-((2R,3R)-3-((R)-2-hydroxy-2-methylbut-3-en-1-yl)oxiran-2-yl)pentan-3-ol (7.71 mg, 36 µmol) obtained in Example A-2 in DMF (1 mL) was degassed and replaced with a nitrogen atmosphere. To this mixture, Ag₂CO₃ (7.4 mg, 27 µmol) and Pd(OAc)₂ (1.0 mg, 4.5 µmol) were added at room temperature. The resulting reaction mixture was stirred at room temperature for 20 minutes and then at 80°C for 2 hours. The reaction mixture was allowed to cool and concentrated under reduced pressure. The residue was suspended in ethyl acetate, and insoluble matter was filtered off through a Millipore filter. The filtrate was concentrated under reduced pressure. The residue was purified by silica gel column chromatography (25%-50% ethyl acetate/n-heptane) to obtain the title compound (10.1 mg).

¹H-NMR (500 MHz, CDCl₃) δ (ppm): 0.06 (s, 3H), 0.07 (s, 3H), 0.60-0.66 (m, 6H), 0.87-0.91 (m, 15H), 0.94-1.02 (m, 12H), 1.18-1.23 (m, 3H), 1.24-1.35 (m, 2H), 1.36 (s, 3H), 1.40-1.47 (m, 2H), 1.49-1.56 (m, 2H), 1.59-1.68 (m, 2H), 1.73 (br.d, J = 6.1 Hz, 1H), 1.75 (s, 3H), 1.79-1.87 (m, 1H), 2.06 (s, 3H), 2.30-2.55 (m, 4H), 2.75 (dd, J = 6.4, 2.1 Hz, 1H), 2.98 (td, J = 6.1, 2.5 Hz, 1H), 3.65 (br.s, 1H), 3.76-3.86 (m, 1H), 4.95-5.01 (m, 2H), 5.59 (dd, J = 15.3, 9.2 Hz, 1H), 5.66 (dd, J = 15.3, 9.2 Hz, 1H), 5.81 (d, J = 15.3 Hz, 1H), 6.14 (d, J = 11.0 Hz, 1H)6.50 (dd, J = 15.3, 11.0 Hz, 1H).

### C-3-(2)

### Synthesis of pladienolide D

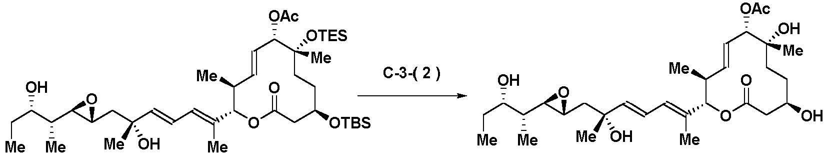

To a solution of (2S,3S,6S,7R,10R,E)-10((t-butyldimethylsilyl)oxy)-2-((R,2E,4E)-6-hydroxy-7-((2R,3R)-3-((2R,3S)-3-hydroxypentan-2-yl)oxiran-2-yl)-6-methylhepta-2,4-dien-2-yl)-3,7-dimethyl-12-oxo-7-((triethylsilyl)oxy)oxacyclododec-4-en-6-yl acetate (10.0 mg, 13 µmol) obtained in Example C-3-(1) in THF (0.3 mL), a THF solution of TBAF (1 M; 64 µL, 64 µmol) was added at 25°C. The reaction mixture was stirred at room temperature for 15 hours. A saturated aqueous ammonium chloride solution was added to the reaction mixture, then ethyl acetate and water were added thereto, and the organic layer was separated. The organic layer was dried over magnesium sulfate, filtered, and then concentrated under reduced pressure. The residue was purified by silica gel column chromatography (0%-5% MeOH/ethyl acetate) to obtain the title compound (6.2 mg).

The ¹H-NMR spectrum (500 MHz, CD₃OD) of the obtained compound was consistent with the NMR spectrum in Example C-1.

### Example C-4

### Synthesis of pladienolide D

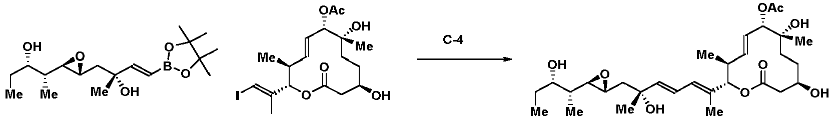

To a solution of (2S,3S,6S,7R,10R,E)-7,10-dihydroxy-2-((E)-1-iodoprop-1-en-2-yl)-3,7-dimethyl-12-oxooxacyclododec-4-en-6-yl acetate (10 mg, 21 µmol) obtained in Example B-8 and (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-ol (8.03 mg, 24 µmol) obtained in Example A-8 in THF (1 mL) and water (0.1 mL), Ag₂O (24.9 mg, 0.107 mmol), Pd₂(dba)₃ (3.93 mg, 4.29 µmol), and Pd(dppf)Cl₂ (3.14 mg, 4.29 µmol) were added, and the mixture was stirred overnight at room temperature under a nitrogen atmosphere. The reaction mixture was filtered. The filtrate was concentrated under reduced pressure. The resulting residue was purified by silica gel column chromatography (0%-100% ethyl acetate/n-heptane) to obtain the title compound (3.0 mg).

The ¹H-NMR spectrum (500 MHz, CD₃OD) of the obtained compound was consistent with the NMR spectrum in Example C-1.

### Example C-5

### Synthesis of (2S,3S,6S,7R,10R,E)-10-((t-butyldimethylsilyl)oxy)-7-hydroxy-2-((R,2E,4E)-6-hydroxy-7-((2R,3R)-3-((2R,3S)-3-hydroxypentan-2-yl)oxiran-2-yl)-6-methylhepta-2,4-dien-2-yl)-3,7-dimethyl-12-oxooxacyclododec-4-en-6-yl acetate

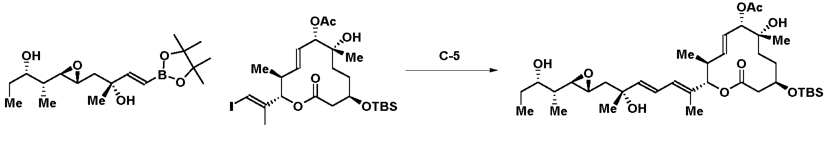

To a solution of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclododec-4-en-6-yl acetate (10 mg, 17 µmol) obtained in Example B-6 and (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-ol (6.45 mg, 19 µmol) obtained in Example A-8 in THF (1 mL) and water (0.1 mL), Ag₂O (20.0 mg, 86 µmol), Pd₂(dba)₃ (3.15 mg, 3.45 µmol), and Pd(dppf)Cl₂ (2.52 mg, 3.45 µmol) were added. The reaction mixture was stirred overnight at room temperature under a nitrogen atmosphere. The reaction mixture was filtered. The filtrate was concentrated under reduced pressure. The resulting residue was purified by silica gel column chromatography (0%-100% ethyl acetate/n-heptane) to obtain the title compound (7.4 mg).

The ¹H-NMR spectrum (500 MHz, CDCl₃) of the obtained compound was consistent with the NMR spectrum in Example C-2-(1).

According to the method of Example C-2-(2), pladienolide D can be synthesized from the obtained compound.

### Example C-6

### Synthesis of pladienolide D

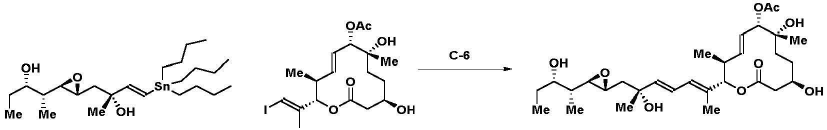

To a DMF solution (1 mL) of (2S,3S,6S,7R,10R,E)-7,10-dihydroxy-2-((E)-1-iodoprop-1-en-2-yl)-3,7-dimethyl-12-oxooxacyclodec-4-en-6-yl acetate (12.5 mg, 27 µmol) obtained in Example B-8, a THF solution (1 mL) of (2R,3S)-2-((2R,3R)-3-((R,E)-2-hydroxy-2-methyl-4-(tributylstannyl)but-3-en-1-yl)oxiran-2-yl)pentan-3-ol (16.2 mg, 32 µmol) obtained in Example A-7, PdCl₂(PhCN)₂ (3.08 mg, 8.04 µmol), and DIPEA (24 µL, 139 µmol) were added. The resulting mixture was stirred at 50°C for 5 hours 30 minutes. The reaction mixture was cooled in an ice-water bath. Ethyl acetate and water were added to the reaction mixture, and the organic layer was separated. Ethyl acetate and saturated saline were added to the aqueous layer, then the mixture was filtered, and the organic layer of the filtrate was separated. The combined organic layer was dried over anhydrous sodium sulfate, filtered, and then concentrated under reduced pressure. The resulting residue was purified by silica gel column chromatography (0%-100% ethyl acetate/n-heptane) to obtain the title compound (4.39 mg).

The ¹H-NMR spectrum (500 MHz, CD₃OD) of the obtained compound was consistent with the NMR spectrum in Example C-1.

### Example C-7

### Synthesis of pladienolide D

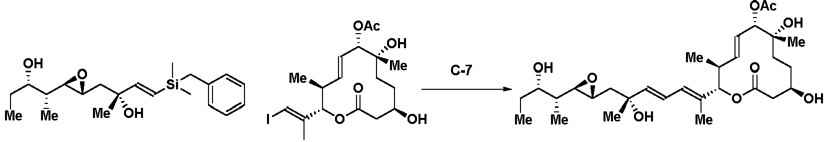

To a solution of (2R,3S)-2-((2R,3R)-3-((R,E)-4-(benzyldimethylsilyl)-2-hydroxy-2-methylbut-3-en-1-yl)oxiran-2-yl)pentan-3-ol (4.7 mg, 13 µmol) obtained in Example A-6 in 1,4-dioxane (0.5 mL), a THF solution of TBAF (1 M; 28 µL, 28 nmol) was added at 25°C, and the mixture was stirred at room temperature for 30 minutes. To this mixture, a solution of (2S,3S,6S,7R,10R,E)-7,10-dihydroxy-2-((E)-1-iodoprop-1-en-2-yl)-3,7-dimethyl-12-oxooxacyclodec-4-en-6-yl acetate (5.3 mg, 11 µmol) obtained in Example B-8 in 1,4-dioxane (0.5 mL) and Pd₂(dba)₃ (1.04 mg, 1.14 µmol) were added. The reaction mixture was stirred at room temperature for 20 hours. The reaction mixture was concentrated under reduced pressure. The residue was purified by silica gel column chromatography (0%-2% MeOH/ethyl acetate) to obtain the title compound (2.9 mg).

The ¹H-NMR spectrum (500 MHz, CD₃OD) of the obtained compound was consistent with the NMR spectrum in Example C-1.

### Example C-8

### Synthesis of pladienolide D

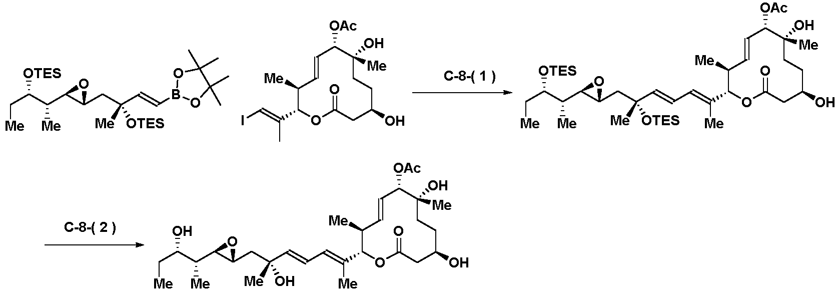

### C-8-(1)

### Synthesis of (2S,3S,6S,7R,10R,E)-7,10-dihydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

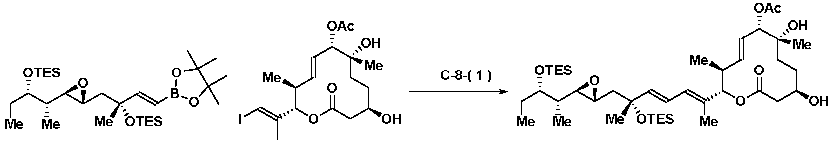

To a mixture of (2S,3S,6S,7R,10R,E)-7,10-dihydroxy-2-((E)-1-iodoprop-1-en-2-yl)-3,7-dimethyl-12-oxooxacyclodec-4-en-6-yl acetate (5.03 mg, 10.8 µmol) obtained in Example B-8, Pd₂(dba)₃ (1.98 mg, 2.16 µmol), and Ag₂O (12.5 mg, 54 µmol), a solution of triethyl(((R,E)-2-methyl-4-(4,4,5,5-tetramethyl-1,3,2-dioxaborolan-2-yl)-1-((2R,3R)-3-((2S,3S)-3-((triethylsilyl)oxy)pentan-2-yl)oxiran-2-yl)but-3-en-2-yl)oxy)silane (8.22 mg, 14 µmol) obtained in Example A-4-(2) in THF (1 mL) was added. Water (0.1 mL) was added to the mixture, and then the reaction mixture was stirred at room temperature under a nitrogen atmosphere for 22 hours. After MeCN was added to the reaction mixture, insoluble matter in the mixture was filtered off through a Millipore filter. The filtrate was concentrated under reduced pressure. The residue was purified by silica gel column chromatography (50%-75% ethyl acetate/n-heptane) to obtain the title compound (3.3 mg).

¹H-NMR (500 MHz, CDCl₃) δ (ppm): 0.57-0.65 (m, 12H), 0.79-0.85 (m, 6H), 0.88 (d, J = 6.3 Hz, 3H), 0.96 (td, J = 8.0, 3.4 Hz, 18H), 1.21 (s, 3H), 1.28-1.38 (m, 3H), 1.40 (s, 3H), 1.42-1.54 (m, 4H), 1.59 (br.d, J = 6.3 Hz, 1H), 1.65-1.72 (m, 1H), 1.74 (d, J = 1.2 Hz, 3H), 1.90 (dd, J = 13.8, 5.2 Hz, 1H), 2.10 (s, 3H), 2.44-2.67 (m, 4H), 2.80-2.86 (m, 1H), 3.53 (d, J = 10.9 Hz, 1H), 3.70-3.77 (m, 2H), 5.09 (d, J = 9.2 Hz, 1H), 5.18 (d, J = 10.3 Hz, 1H), 5.61 (dd, J = 14.9, 9.2 Hz, 1H), 5.67 (dd, J = 14.9, 9.2 Hz, 1H), 5.76 (d, J = 14.9 Hz, 1H), 6.09 (d, J = 10.9 Hz, 1H), 6.43 (dd, J = 15.2, 11.2 Hz, 1H).

### C-8-(2)

### Synthesis of pladienolide D

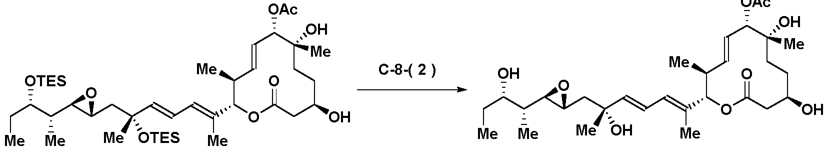

To a solution of (2S,3S,6S,7R,10R,E)-7,10-dihydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate (3.3 mg, 4.2 µmol) obtained in Example C-8-(1) in THF (0.5 mL), a THF solution of TBAF (1 M; 17 µL, 17 µmol) was added at 25°C. The reaction mixture was stirred at room temperature for 1 hour. Then, to the reaction mixture, a THF solution of TBAF (1 M; 17 µL, 17 µmol) was added, and the mixture was stirred for 2.5 hours. A saturated aqueous ammonium chloride solution was added to the reaction mixture, then ethyl acetate and water were added thereto, and the organic layer was separated. The organic layer was dried over anhydrous magnesium sulfate, filtered, and then concentrated under reduced pressure. The residue was purified by silica gel column chromatography (0%-5% MeOH/ethyl acetate) to obtain the title compound (2.3 mg).

The ¹H-NMR spectrum (500 MHz, CD₃OD) of the obtained compound was consistent with the NMR spectrum in Example C-1.

### Example C-9

### Synthesis of (2S,3S,6S,7R,10R,E)-7,10-dihydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

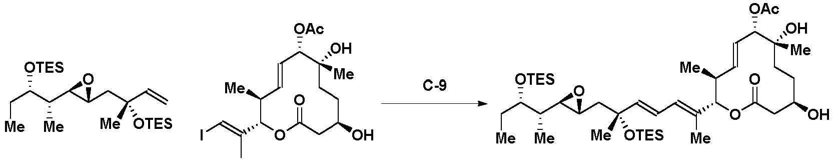

A solution of (2S,3S,6S,7R,10R,E)-7,10-dihydroxy-2-((E)-1-iodoprop-1-en-2-yl)-3,7-dimethyl-12-oxooxacyclodec-4-en-6-yl acetate (6.45 mg, 14 µmol) obtained in Example B-8 and triethyl(((R)-2-methyl-1-((2R,3R)-3-((2S,3S)-3-((triethylsilyl)oxy)pentan-2-yl)oxiran-2-yl)but-3-en-2-yl)oxy)silane (12.3 mg, 28 µmol) obtained in Example A-3 in DMF (1 ml) was degassed and replaced with a nitrogen atmosphere. To this mixture, Ag₂CO₃ (4.96 mg, 18 µmol) and Pd(OAc)₂ (0.78 mg, 3.5 µmol) were added. The resulting reaction mixture was stirred at room temperature for 15 minutes, at 80°C for 2.5 hours, and then at 60°C for 15 hours. Then, to the reaction mixture, Ag₂CO₃ (4.96 mg, 18 µmol) and Pd(OAc)₂ (0.78 mg, 3.5 µmol) were added, and then the reaction mixture was stirred at 80°C for 2.5 hours. The reaction mixture was allowed to cool and concentrated under reduced pressure. The residue was suspended in ethyl acetate, and insoluble matter was filtered off through a Millipore filter. The filtrate was concentrated under reduced pressure. The residue was purified by silica gel column chromatography (50%-70% ethyl acetate/n-heptane) to obtain the title compound (4.4 mg).

The ¹H-NMR spectrum (500 MHz, CDCl₃) of the obtained compound was consistent with the NMR spectrum in Example C-8-(1).

According to the method of Example C-8-(2), pladienolide D can be synthesized from the obtained compound.

### Example C-10

### Synthesis of pladienolide D

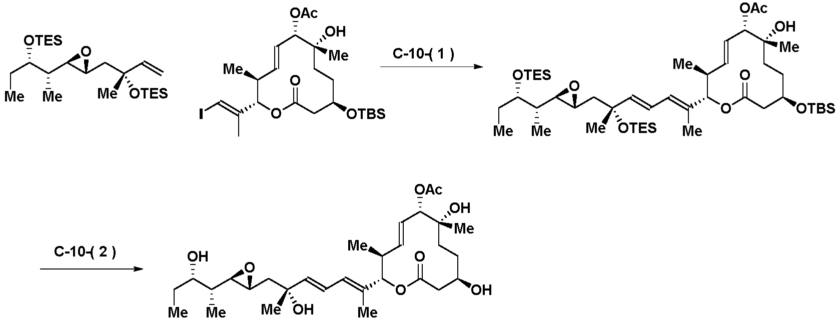

### C-10-(1)

### Synthesis of (2S,3S,6S,7R,10R,E)-10-((t-butyldimethylsilyl)oxy)-7-hydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

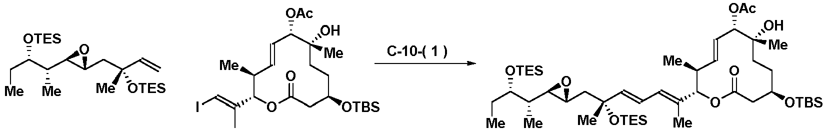

A solution of triethyl(((R)-2-methyl-1-((2R,3R)-3-((2S,3S)-3-((triethylsilyl)oxy)pentan-2-yl)oxiran-2-yl)but-3-en-2-yl)oxy)silane (19.6 mg, 44 µmol) obtained in Example A-3 and (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclodec-4-en-6-yl acetate (17 mg, 29 µmol) obtained in Example B-6 in DMF (1 mL) was degassed and then replaced with a nitrogen atmosphere. To this mixture, Pd(OAc)₂ (1.64 mg, 7.32 µmol) and Ag₂CO₃ (10.5 mg, 38 µmol) were sequentially added. The resulting reaction mixture was stirred at 80°C for 3 hours. Then, to the reaction mixture, Pd(OAc)₂ (1.64 mg, 7.32 µmol) and Ag₂CO₃ (10.5 mg, 38 µmol) were added, and the mixture was stirred at 80°C for 4 hours. To the ice-cooled reaction mixture, ethyl acetate and water were added. Insoluble matter in the mixture was filtered off. Then, the organic layer of the filtrate was separated. The organic layer was washed with a 10% aqueous sodium chloride solution (twice), dried over anhydrous sodium sulfate, filtered, and then concentrated under reduced pressure. The resulting residue was purified by silica gel column chromatography (0%-20% ethyl acetate/n-heptane) to obtain the title compound (8.6 mg).

¹H-NMR (500 MHz, CDCl₃) δ (ppm): 0.06 (s, 3H), 0.07 (s, 3H), 0.61 (q, J = 8.0 Hz, 12H), 0.79-0.85 (m, 6H), 0.88 (br.d, J = 6.7 Hz, 3H), 0.90 (s, 9H), 0.96 (t, J = 7.6 Hz, 18H), 1.21 (s, 3H), 1.38 (s, 3H), 1.40-1.55 (m, 6H), 1.57-1.60 (m, 1H), 1.67 (br.s, 1H), 1.70 (s, 3H), 1.89 (dd, J = 14.1, 4.9 Hz, 1H), 2.09 (d, J = 1.2 Hz, 4H), 2.33-2.54 (m, 3H), 2.56 (br.d, J = 8.0 Hz, 1H), 2.83 (br.t, J = 5.5 Hz, 1H), 3.69-3.79 (m, 1H), 3.84 (br.d, J = 3.7 Hz, 1H), 4.97 (d, J = 10.4 Hz, 1H), 5.08 (d, J = 8.0 Hz, 1H), 5.60-5.68 (m, 2H), 5.71 (d, J = 14.7 Hz, 1H), 6.10 (br.d, J = 11.0 Hz, 1H), 6.41 (dd, J = 15.3, 11.0 Hz, 1H).

### C-10-(2)

### Synthesis of pladienolide D

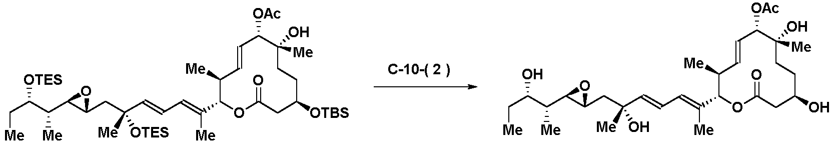

To a solution of (2S,3S,6S,7R,10R,E)-10-((t-butyldimethylsilyl)oxy)-7-hydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate (18.9 mg, 21 µmol) obtained in Example C-10-(1) in THF (1 mL), a THF solution of TBAF (1 M; 0.19 mL, 0.19 mmol) was added. The resulting reaction mixture was stirred overnight at room temperature. A 10% aqueous ammonium chloride solution and ethyl acetate were added to the ice-cooled reaction mixture, and then the organic layer was separated. The aqueous layer was subjected to extraction with ethyl acetate. The combined organic layer was dried over anhydrous sodium sulfate, filtered, and then concentrated under reduced pressure. The resulting residue was purified by silica gel column chromatography (0%-100% ethyl acetate/n-heptane) to obtain the title compound (11.2 mg).

The ¹H-NMR spectrum (500 MHz, CD₃OD) of the obtained compound was consistent with the NMR spectrum in Example C-1.

### Example C-11

### Synthesis of pladienolide D

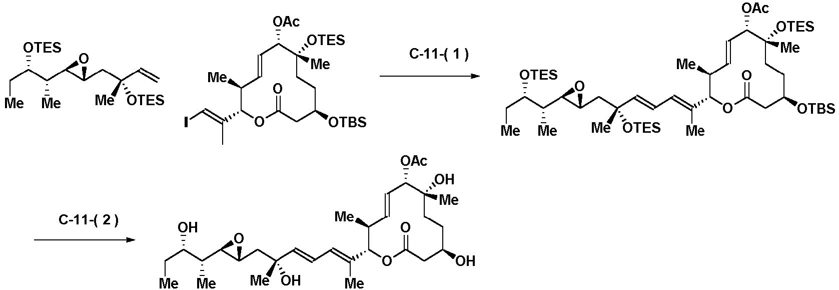

### C-11-(1)

### Synthesis of (2S,3S,6S,7R,10R,E)-10-((t-butyldimethylsilyl)oxy)-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxo-7-((triethylsilyl)oxy)oxacyclododec-4-en-6-yl acetate

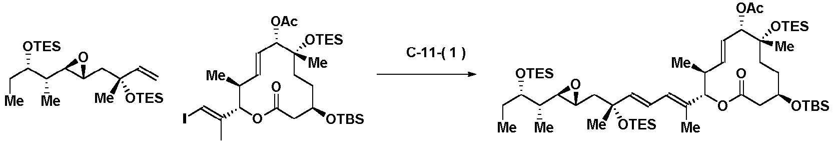

To a solution of (2S,3S,6S,7R,10R,E)-10-((t-butyldimethylsilyl)oxy)-2-((E)-1-iodoprop-1-en-2-yl)-3,7-dimethyl-12-oxo-7-((triethylsilyl)oxy)oxacyclododec-4-en-6-yl acetate (20 mg, 29 µmol) obtained in Example B-4 and triethyl(((R)-2-methyl-1-((2R,3R)-3-((2S,3S)-3-((triethylsilyl)oxy)pentan-2-yl)oxiran-2-yl)but-3-en-2-yl)oxy)silane (19.1 mg, 43 µmol) obtained in Example A-3 in DMF (2 mL), Ag₂CO₃ (10.3 mg, 37 µmol) and Pd(OAc)₂ (1.62 mg, 7.20 µmol) were sequentially added. The reaction mixture was stirred under a nitrogen atmosphere at room temperature for 15 minutes and at 80°C for 5 hours. Then, to the reaction mixture, Ag₂CO₃ (10.3 mg, 37 µmol) and Pd(OAc)₂ (1.62 mg, 7.20 µmol) were added, and the reaction mixture was stirred overnight at 80°C. The reaction mixture was allowed to cool to room temperature. Insoluble matter in the mixture was filtered off and washed with ethyl acetate. The filtrate was washed with water. The organic layer was dried over anhydrous sodium sulfate, filtered, and then concentrated under reduced pressure. The residue was purified by silica gel column chromatography (0%-10% ethyl acetate/n-heptane) to obtain the title compound (10.1 mg).

¹H-NMR (500 MHz, CDCl₃) δ (ppm): 0.06 (s, 3H), 0.07 (s, 3H), 0.58-0.66 (m, 18H), 0.81-0.85 (m, 6H), 0.87 (d, J = 6.7 Hz, 3H), 0.90 (s, 9H), 0.94-1.03 (m, 27H), 1.21 (s, 3H), 1.28-1.36 (m, 2H), 1.39 (s, 3H), 1.43-1.53 (m, 4H), 1.58-1.69 (m, 2H), 1.73 (s, 3H), 1.89 (dd, J = 13.5, 4.9 Hz, 1H), 2.06 (s, 3H), 2.35-2.52 (m, 3H), 2.57 (dd, J = 8.0, 1.8 Hz, 1H), 2.81-2.86 (m, 1H), 3.71-3.76 (m, 1H), 3.77-3.83 (m, 1H), 4.96-5.02 (m, 2H), 5.59 (dd, J = 15.3, 9.8 Hz, 1H), 5.65 (dd, J = 15.3, 8.6 Hz, 1H), 5.72 (d, J = 15.3 Hz, 1H), 6.11 (br.d, J = 11.0 Hz, 1H), 6.41 (dd, J = 15.3, 11.0 Hz, 1H).

### C-11-(2)

### Synthesis of pladienolide D

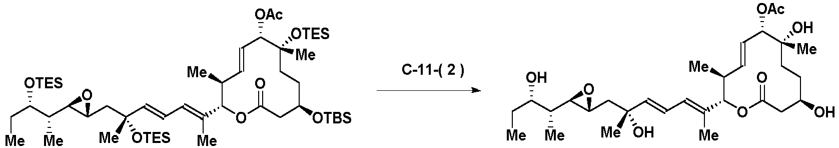

To a solution of (2S,3S,6S,7R,10R,E)-10-((t-butyldimethylsilyl)oxy)-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxo-7-((triethylsilyl)oxy)oxacyclododec-4-en-6-yl acetate (10.1 mg, 10.0 µmol) obtained in Example C-11-(1) in THF (0.50 mL), a THF solution of TBAF (1 M; 0.12 mL, 0.12 mmol) was added. The reaction mixture was stirred overnight at room temperature. The reaction mixture was concentrated under reduced pressure. The resulting residue was purified by silica gel column chromatography (66%-100% ethyl acetate/n-heptane) to obtain the title compound (5.0 mg).

The ¹H-NMR spectrum (500 MHz, CD₃OD) of the obtained compound was consistent with the NMR spectrum in Example C-1.

### Example C-12

### Synthesis of (2S,3S,6S,7R,10R,E)-7,10-dihydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

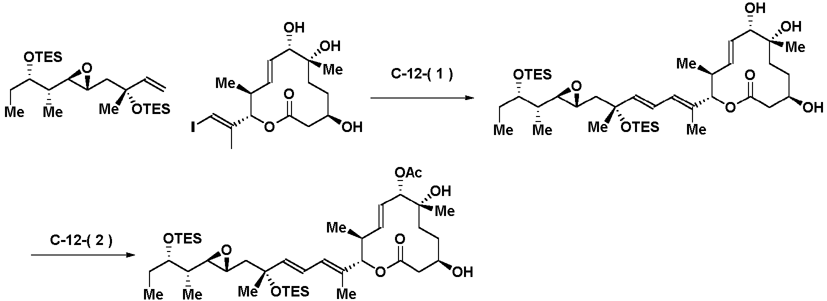

### C-12-(1)

### Synthesis of (4R,7R,8S,11S,12S,E)-4,7,8-trihydroxy-7,11-dimethyl-12-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)oxacyclododec-9-en-2-one

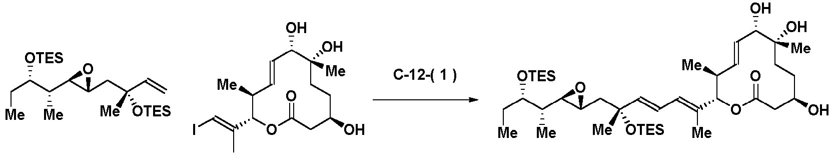

A solution of (4R,7R,8S,11S,12S,E)-4,7,8-trihydroxy-12-((E)-1-iodoprop-1-en-2-yl)-7,11-dimethyloxacyclododec-9-en-2-one (19.0 mg, 45 µmol) obtained in Example B-7 and triethyl(((R)-2-methyl-1-((2R,3R)-3-((2S,3S)-3-((triethylsilyl)oxy)pentan-2-yl)oxiran-2-yl)but-3-en-2-yl)oxy)silane (29.7 mg, 67 µmol) obtained in Example A-3 in DMF (1 ml) was degassed and replaced with a nitrogen atmosphere. To this mixture, Ag₂CO₃ (16.1 mg, 58 µmol) and Pd(OAc)₂ (2.51 mg, 11 µmol) were added. The resulting reaction mixture was stirred at room temperature for 20 minutes and then at 80°C for 4 hours 20 minutes. The reaction mixture was allowed to cool and concentrated under reduced pressure. The residue was suspended in ethyl acetate, and insoluble matter was filtered off through a Millipore filter. The filtrate was concentrated under reduced pressure. The residue was purified by silica gel column chromatography (50%-75% ethyl acetate/n-heptane) to obtain the title compound (18.4 mg).

¹H-NMR (500 MHz, CDCl₃) δ (ppm): 0.58-0.67 (m, 12H), 0.79-0.85 (m, 6H), 0.90 (d, J = 6.7 Hz, 3H), 0.97 (td, J = 8.0, 4.3 Hz, 18H), 1.18-1.30 (m, 3H), 1.30-1.34 (m, 3H), 1.39-1.41 (m, 3H), 1.42-1.54 (m, 4H), 1.63-1.73 (m, 1H), 1.75 (s, 3H), 1.83 (br.d, J = 4.3 Hz, 1H), 1.91 (dd, J = 14.1, 4.9 Hz, 1H), 2.38 (s, 1H), 2.48-2.58 (m, 3H), 2.59-2.66 (m, 1H), 2.84 (ddd, J = 6.6, 4.7, 2.1 Hz, 1H), 3.54 (d, J = 11.0 Hz, 1H), 3.69-3.78 (m, 2H), 3.82 (dd, J = 9.8, 3.7 Hz, 1H), 5.17 (d, J = 10.4 Hz, 1H), 5.43 (dd, J = 15.0, 10.1 Hz, 1H), 5.69-5.79 (m, 2H), 6.08 (dd, J = 11.0, 1.2 Hz, 1H), 6.43 (dd, J = 15.3, 11.0 Hz, 1H).

### C-12-(2)

### Synthesis of (2S,3S,6S,7R,10R,E)-7,10-dihydroxy-3,7-dimethyl-2-((R,2E,4E)-6-methyl-6-((triethylsilyl)oxy)-7-((2R,3R)-3-((25,3S)-3-((triethylsilyl)oxy)pentan-2-yl)oxiran-2-yl)hepta-2,4-dien-2-yl)-12-oxooxacyclododec-4-en-6-yl acetate

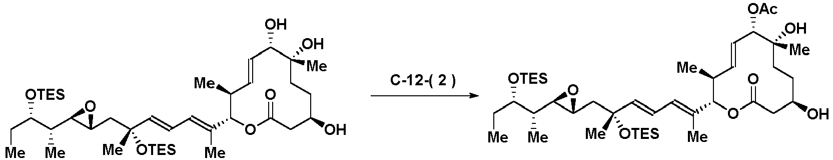

To a solution of (4R,7R,8S,11S,12S,E)-4,7,8-trihydroxy-7,11-dimethyl-12-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)oxacyclododec-9-en-2-one (16.5 mg, 22 µmol) obtained in Example C-12-(1), DMAP (0.55 mg, 4.5 µmol), and Et₃N (9.3 µL, 67 µmol) in DCM (1 mL), a DCM solution of Ac₂O (1 M; 22 µL, 22 µmol) was added under ice cooling. The reaction solution was stirred at the same temperature for 30 minutes. A DCM solution of Ac₂O (1 M; 2 µL, 2 µmol) was then added to the reaction mixture, and the resulting reaction mixture was stirred under ice cooling for 30 minutes. A saturated aqueous sodium bicarbonate solution, ethyl acetate, and water were added to the reaction mixture, and the organic layer was separated. The resulting organic layer was washed with saturated saline, dried over anhydrous magnesium sulfate, filtered, and then concentrated under reduced pressure. The residue was purified by silica gel column chromatography (46%-65% ethyl acetate/n-heptane) to obtain the title compound (14.9 mg).

The ¹H-NMR spectrum (500 MHz, CDCl₃) of the obtained compound was consistent with the NMR spectrum in Example C-8-(1).

According to the method of Example C-8-(2), pladienolide D can be synthesized from the obtained compound.

### Example C-13

### Synthesis of (2S,3S,6S,7R,10R,E)-10-((t-butyldimethylsilyl)oxy)-7-hydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

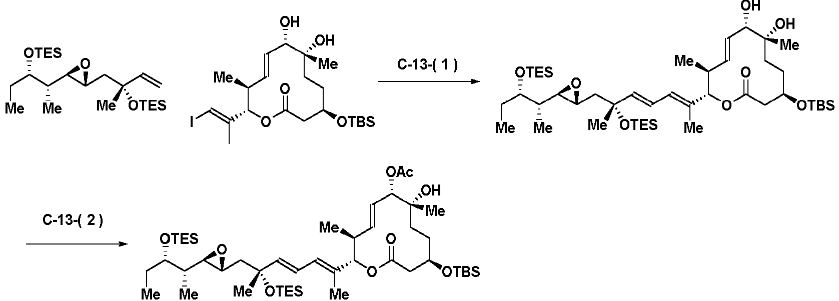

### C-13-(1)

### Synthesis of (4R,7R,8S,11S,12SX)-4-((t-butyldimethylsilyl)oxy)-7,8-dihydroxy-7,11-dimethyl-12-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)oxacyclododec-9-en-2-one

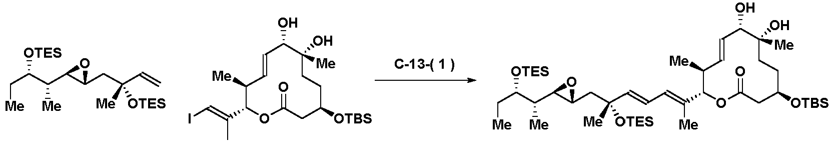

A solution of triethyl(((R)-2-methyl-1-((2R,3R)-3-((2S,3S)-3-((triethylsilyl)oxy)pentan-2-yl)oxiran-2-yl)but-3-en-2-yl)oxy)silane (30 mg, 68 µmol) obtained in Example A-3 and (4R,7R,8S,11S,12S,E)-4-((t-butyldimethylsilyl)oxy)-7,8-dihydroxy-12-((E)-1-iodoprop-1-en-2-yl)-7,11-dimethyloxacyclododec-9-en-2-one (24.4 mg, 45 µmol) obtained in Example B-5 in DMF (1 mL) was degassed and then replaced with a nitrogen atmosphere. To this mixture, Pd(OAc)₂ (2.54 mg, 11 µmol) and Ag₂CO₃ (16.2 mg, 59 µmol) were sequentially added. The resulting reaction mixture was stirred at 80°C for 3 hours. Ethyl acetate and water were added to the reaction mixture, and insoluble matter in the mixture was filtered off. Then, the organic layer of the filtrate was separated. The organic layer was washed with a 10% aqueous sodium chloride solution (twice), dried over anhydrous sodium sulfate, filtered, and then concentrated under reduced pressure. The resulting residue was purified by silica gel column chromatography (0%-30% ethyl acetate/n-heptane) to obtain the title compound (18.7 mg).

¹H-NMR (500 MHz, CDCl₃) δ (ppm): 0.07 (s, 3H), 0.08 (s, 3H), 0.56-0.65 (m, 12H), 0.78-0.85 (m, 6H), 0.89 (d, J = 6, 7 Hz, 3H), 0.90 (s, 9H), 0.96 (td, J = 8.0, 1.8 Hz, 18H), 1.23 (td, J = 7, 6, 3.7 Hz, 1H), 1.30 (s, 3H), 1.39 (s, 3H), 1.40-1.54 (m, 6H), 1.58-1.65 (m, 1H), 1.66-1.77 (m, 3H), 1.82 (d, J = 4.3 Hz, 1H), 1.90 (dd, J = 13.8, 4.6 Hz, 1H), 2.35-2.60 (m, 5H), 2.77-2.86 (m, 1H), 3.73 (td, J = 6.4, 3.1 Hz, 1H), 3.81 (dt, J = 9.8, 4.9 Hz, 2H), 5.00 (d, J = 10.4 Hz, 1H), 5.47 (dd, J = 15.0, 10.1 Hz, 1H), 5.66-5.77 (m, 2H), 6.10 (d, J = 11.0 Hz, 1H), 6.42 (dd, J = 15.0, 11.3 Hz, 1H).

### C-13-(2)

### Synthesis of (2S,3S,6S,7R,10R,E)-10-((t-butyldimethylsilyl)oxy)-7-hydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

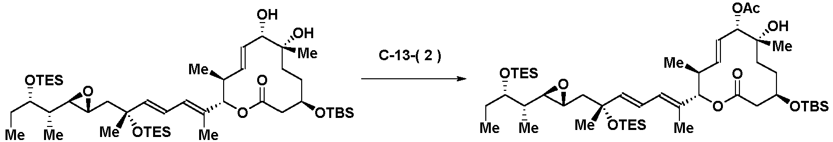

To a DCM solution (1 mL) of (4R,7R,8S,11S,12S,E)-4-((t-butyldimethylsilyl)oxy)-7,8-dihydroxy-7,11-dimethyl-12-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)oxacyclododec-9-en-2-one (18.7 mg, 22 µmol) obtained in Example C-13-(1) and DMAP (0.535 mg, 4.4 µmol), Et₃N (9.2 µL, 66 µmol) and Ac₂O (2.2 µL, 23 µmol) were sequentially added under ice cooling. The reaction mixture was stirred at the same temperature for 1 hour. To the reaction mixture, Ac₂O (2.2 µL, 23 µmol) was added, and the mixture was stirred for 30 minutes. To the reaction mixture, Ac₂O (0.55 µL, 5.8 µmol) was added, and the mixture was stirred for 40 minutes. Then, to the reaction mixture, Ac₂O (0.55 µL, 5.8 µmol) was added, and the mixture was stirred for 15 minutes. The reaction mixture was quenched with a saturated aqueous sodium bicarbonate solution. Ethyl acetate was added to the obtained mixture, and the organic layer was separated. The organic layer was washed with saturated saline. The organic layer was dried over anhydrous sodium sulfate, filtered, and then concentrated under reduced pressure. The resulting residue was purified by silica gel column chromatography (0%-20% ethyl acetate/n-heptane) to obtain the title compound (18.9 mg).

The ¹H-NMR spectrum (500 MHz, CDCl₃) of the obtained compound was consistent with the NMR spectrum in Example C-10-(1).

According to the method of Example C-10-(2), pladienolide D can be synthesized from the obtained compound.

### Example C-14

### Synthesis of (2S,3S,6S,7R,10R,E)-10-((t-butyldimethylsilyl)oxy)-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxo-7-((triethylsilyl)oxy)oxacyclododec-4-en-6-yl acetate

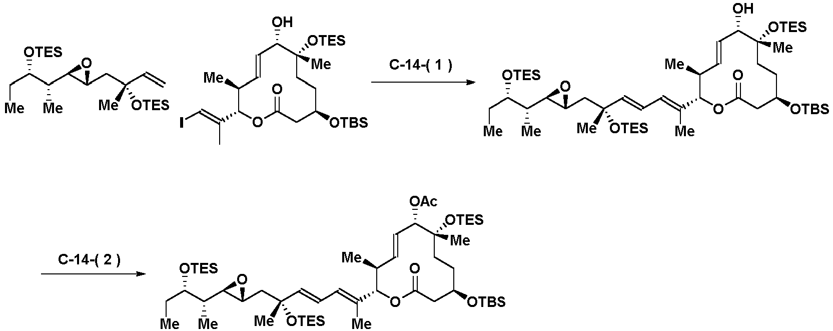

### C-14-(1)

### Synthesis of (4R,7R,8S,11S,12S,E)-4-((t-butyldimethylsilyl)oxy)-8-hydroxy-7,11-dimethyl-12-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-7-((triethylsilyl)oxy)oxacyclododec-9-en-2-one

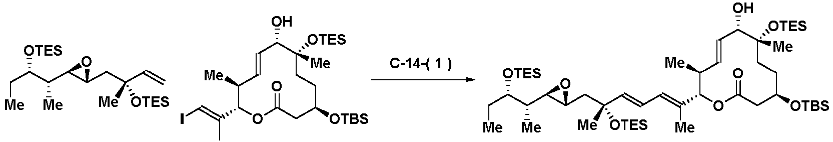

To a solution of (4R,7R,8S,11S,12S,E)-4-((t-butyldimethylsilyl)oxy)-8-hydroxy-12-((E)-1-iodoprop-1-en-2-yl)-7,11-dimethyl-7-((triethylsilyl)oxy)oxacyclododec-9-en-2-one (30 mg, 46 µmol) obtained in Example B-3 and triethyl(((R)-2-methyl-1-((2R,3R)-3-((2S,3S)-3-((triethylsilyl)oxy)pentan-2-yl)oxiran-2-yl)but-3-en-2-yl)oxy)silane (30.5 mg, 69 µmol) obtained in Example A-3 in DMF (2 mL), Ag₂CO₃ (16.5 mg, 60 µmol) and Pd(OAc)₂ (2.58 mg, 11 µmol) were sequentially added. The reaction mixture was stirred under a nitrogen atmosphere at room temperature for 15 minutes and at 80°C for 3 hours. The reaction mixture was allowed to cool to room temperature. Insoluble matter in the mixture was filtered off and washed with ethyl acetate. The filtrate was washed with water. The organic layer was dried over anhydrous sodium sulfate, filtered, and then concentrated under reduced pressure. The resulting residue was purified by silica gel column chromatography (0%-10% ethyl acetate/n-heptane) to obtain the title compound (15 mg).

¹H-NMR (500 MHz, CDCl₃) δ (ppm): 0.07 (s, 3H), 0.08 (s, 3H), 0.58-0.66 (m, 18H), 0.80-0.85 (m, 6H), 0.89-0.91 (m, 12H), 0.94-1.02 (m, 27H), 1.32 (s, 3H), 1.34-1.38 (m, 1H), 1.39 (s, 3H), 1.41-1.44 (m, 1H), 1.45-1.55 (m, 4H), 1.58-1.66 (m, 2H), 1.72 (d, J = 1.2 Hz, 3H), 1.89 (dd, J = 13.5, 4.9 Hz, 1H), 2.38-2.51 (m, 3H), 2.56 (dd, J = 8.3, 2.1 Hz, 1H), 2.66 (d, J= 10.4 Hz, 1H), 2.84 (br.t, J = 4.6 Hz, 1H), 3.57 (t, J = 10.1 Hz, 1H), 3.73 (dt, J = 6.6, 3.1 Hz, 1H), 3.81 (dt, J = 5.2, 2.9 Hz, 1H), 5.01 (d, J = 11.0 Hz, 1H), 5.40 (dd, J = 15.3, 9.8 Hz, 1H), 5.58 (dd, J = 15.0, 9.5 Hz, 1H), 5.72 (d, J = 15.3 Hz, 1H), 6.10 (d, J = 11.0 Hz, 1H), 6.42 (dd, J = 15.3, 11.0 Hz, 1H).

### C-14-(2)

### Synthesis of (2S,3S,6S,7R,10R,E)-10-((t-butyldimethylsilyl)oxy)-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxo-7-((triethylsilyl)oxy)oxacyclododec-4-en-6-yl acetate

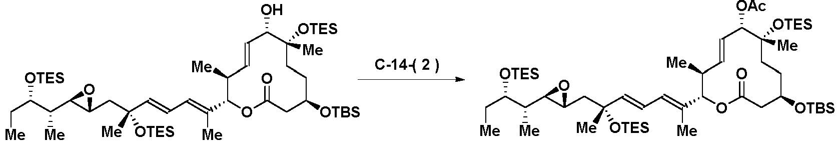

To a solution of (4R,7R,8S,11S,12S,E)-4-((t-butyldimethylsilyl)oxy)-8-hydroxy-7,11-dimethyl-12-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-7-((triethylsilyl)oxy)oxacyclododec-9-en-2-one (15 mg, 16 µmol) obtained in Example C-14-(1) in pyridine (1 mL), Ac₂O (0.5 mL) was added at room temperature. The reaction mixture was stirred overnight at room temperature. The reaction mixture was concentrated under reduced pressure. The resulting residue was purified by silica gel column chromatography (0%-10% ethyl acetate/n-heptane) to obtain the title compound (16.1 mg).

The ¹H-NMR spectrum (500 MHz, CDCl₃) of the obtained compound was consistent with the NMR spectrum in Example C-11-(1).

According to the method of Example C-11-(2), pladienolide D can be synthesized from the obtained compound.

### Example C-15

### Synthesis of (2S,3S,6S,7R,10R,E)-10-((t-butyldimethylsilyl)oxy)-7-hydroxy-3,7-dimethyl-2-((R,2E,4E)-6-methyl-6-((triethylsilyl)oxy)-7-((2R,3R)-3-((2S,3S)-3-((triethylsilyl)oxy)pentan-2-yl)oxiran-2-yl)hepta-2,4-dien-2-yl)-12-oxooxacyclododec-4-en-6-yl acetate

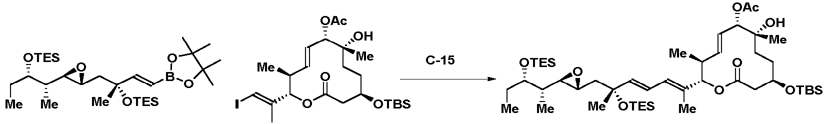

To a solution of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclodec-4-en-6-yl acetate (5.8 mg, 10 µmol) obtained in Example B-6, triethyl(((R,E)-2-methyl-4-(4,4,5,5,-tetramethyl-1,3,2-dioxaborolan-2-yl)-1-((2R,3R)-3-((2S,3S)-3-((triethylsilyl)oxy)pentan-2-yl)oxiran-2-yl)but-3-en-2-yl)oxy)silane (8.4 mg, 15 µmol) obtained in Example A-4-(2), and Pd(dppf)Cl₂ (1.46 mg, 2.0 µmol) in THF (0.9 mL), Ag₂O (11.6 mg, 50 µmol) and water (0.1 ml) were sequentially added at 25°C. The reaction mixture was stirred at room temperature under a nitrogen atmosphere for 130 minutes. After MeCN was added to the reaction mixture, the mixture was filtered through a Millipore filter. The filtrate was concentrated under reduced pressure. The residue was purified by silica gel column chromatography (15%-37% ethyl acetate/n-heptane) to obtain the title compound (8.5 mg).

The ¹H-NMR (500 MHz, CDCl₃) spectrum of the obtained compound was consistent with the NMR spectrum in Example C-10-(1).

According to the method of Example C-10-(2), pladienolide D can be synthesized from the obtained compound.

### Example C-16

### Synthesis of pladienolide D

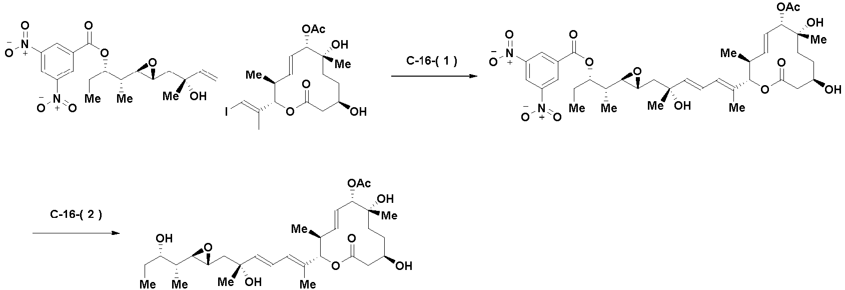

### C-16-(1)

### Synthesis of (2R,3S)-2-((2R,3R)-3-((R,3E,5E)-6-((2S,3S,6S,7R,10R,E)-6-acetoxy-7,10-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

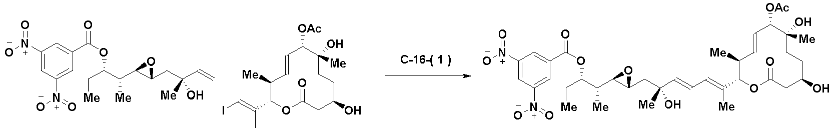

To a solution of (2S,3S,6S,7R,10R,E)-7,10-dihydroxy-2-((E)-1-iodoprop-1-en-2-yl)-3,7-dimethyl-12-oxooxacyclododec-4-en-6-yl acetate (50 mg, 0.107 mmol) obtained in Example B-8 and (2R,3S)-2-((2R,3R)-3-((R)-2-hydroxy-2-methylbut-3-en-1-yl)oxiran-2-yl)pentan-3-yl 3,5-dinitrobenzoate (56.9 mg, 0.139 mmol) obtained in Example A-5 in DMF (3 mL), Ag₂CO₃ (38.4 mg, 0.139 mmol) and Pd(OAc)₂ (6.02 mg, 27 µmol) were added at room temperature. The reaction mixture was stirred under a nitrogen atmosphere at room temperature for 15 minutes and at 80°C for 4 hours. Insoluble matter in the mixture was filtered off and washed with ethyl acetate. The filtrate was washed with water. The organic layer was dried over anhydrous sodium sulfate, filtered, and then concentrated under reduced pressure. The resulting residue was purified by silica gel column chromatography (66%-100% ethyl acetate/n-heptane) to obtain the title compound (57 mg).

¹H-NMR (500 MHz, CDCl₃) δ (ppm): 0.89 (d, J = 6.7 Hz, 3H), 0.97 (t, J = 7.3 Hz, 3H), 1.06 (d, J = 6.7 Hz, 3H), 1.21 (s, 3H), 1.35 (s, 3H), 1.36-1.42 (m, 1H), 1.50-1.56 (m, 2H), 1.63-1.73 (m, 3H), 1.76 (s, 3H), 1.84 (d, J = 3.1 Hz, 1H), 1.87-1.91 (m, 2H), 2.08 (d, J = 4.9 Hz, 2H), 2.10 (s, 3H), 2.48-2.58 (m, 2H), 2.58-2.65 (m, 2H), 2.91 (ddd, J = 7.2, 4.4, 2.5 Hz, 1H), 3.49 (d, J = 11.0 Hz, 1H), 3.76-3.80 (m, 1H), 5.08 (d, J = 9.2 Hz, 1H), 5.16 (d, J = 10.4 Hz, 1H), 5.26-5.31 (m, 1H), 5.61 (dd, J = 15.3, 9.8 Hz, 1H), 5.68 (dd, J = 15.3, 9.2 Hz, 1H), 5.83 (d, J = 15.3 Hz, 1H), 6.11 (d, J = 10.4 Hz, 1H), 6.52 (dd, J = 15.3, 11.0 Hz, 1H), 9.17 (d, J = 1.8 Hz, 2H), 9.24 (t, J = 2.1 Hz, 1H).

### C-16-(2)

### Synthesis of pladienolide D

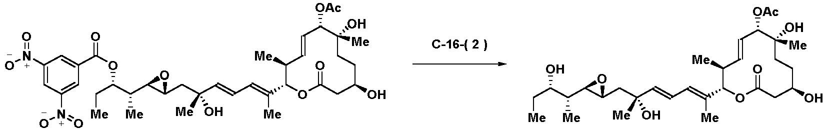

To a solution of (2R,3S)-2-((2R,3R)-3-((R,3E,5E)-6-((2S,3S,6S,7R,10R,E)-6-acetoxy-7,10-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate (3.0 mg, 4.02 µmol) obtained in Example C-16-(1) in THF (0.1 mL), a THF solution of TBAF (1 M; 20 µL, 20 µmol) was added at room temperature. The reaction mixture was stirred at room temperature for 16 hours. A saturated aqueous ammonium chloride solution, ethyl acetate, and water were added to the reaction liquid under ice cooling. The organic layer was separated. The aqueous layer was subjected to extraction with ethyl acetate. The combined organic layer was dried over anhydrous sodium sulfate, filtered, and then concentrated under reduced pressure. The resulting residue was purified by silica gel column chromatography (0%-100% ethyl acetate/n-heptane) to obtain the title compound (2.3 mg).

The ¹H-NMR spectrum (500 MHz, CD₃OD) of the obtained compound was consistent with the NMR spectrum in Example C-1.

### Example C-17

### Synthesis of pladienolide D

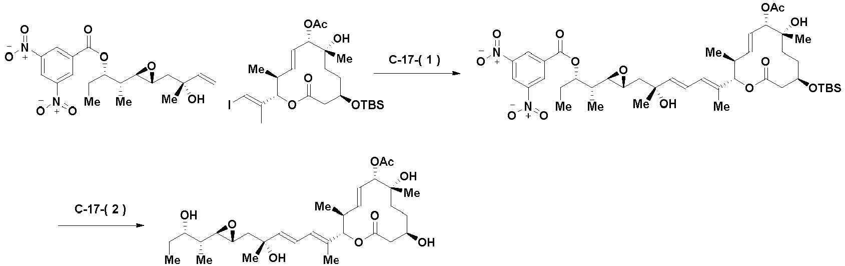

### C-17-(1)

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

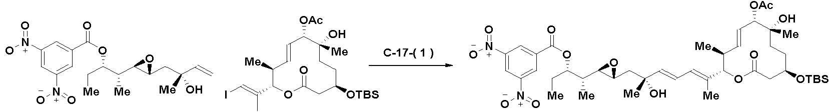

A solution of (2R,3S)-2-((2R,3R)-3-((R)-2-hydroxy-2-methylbut-3-en-1-yl)oxiran-2-yl)pentan-3-yl 3,5-dinitrobenzoate (58.0 mg, 142 µmol) obtained in Example A-5 and (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclodec-4-en-6-yl acetate (55 mg, 95 µmol) obtained in Example B-6 in DMF (2 mL) was degassed and then replaced with a nitrogen atmosphere. To this mixture, Pd(OAc)₂ (5.32 mg, 24 µmol) and Ag₂CO₃ (34 mg, 0.123 mmol) were sequentially added, and the resulting mixture was stirred at 80°C for 2 hours. To the ice-cooled reaction mixture, ethyl acetate and water were added. Insoluble matter in the mixture was filtered off. Then, the organic layer of the filtrate was separated. The organic layer was washed with a 10% aqueous sodium chloride solution (twice), dried over anhydrous sodium sulfate, filtered, and then concentrated under reduced pressure. The resulting residue was purified by silica gel column chromatography (0%-50% ethyl acetate/n-heptane) to obtain the title compound (55.4 mg).

¹H-NMR (500 MHz, CDCl₃) δ (ppm): 0.06 (s, 3H), 0.07 (s, 3H), 0.88-0.92 (m, 12H), 0.97 (t, J = 7.3 Hz, 3H), 1.06 (d, J = 6.7 Hz, 3H), 1.21 (s, 3H), 1.34 (s, 3H), 1.36-1.48 (m, 3H), 1.63-1.74 (m, 6H), 1.80-1.94 (m, 3H), 2.02 (s, 1H), 2.09 (br.s, 1H), 2.10 (s, 3H), 2.38 (dd, J = 14.1, 4.9 Hz, 1H), 2.46 (dd, J = 13.4, 3.1 Hz, 1H), 2.48-2.56 (m, 1H), 2.60 (dd, J = 8.0, 1.8 Hz, 1H), 2.91 (ddd, J = 6.9, 4.4, 2.1 Hz, 1H), 3.84 (br.d, J = 3.7 Hz, 1H), 4.94 (d, J = 11.0 Hz, 1H), 5.07 (d, J = 8.6 Hz, 1H), 5.28 (dt, J = 8.1, 5.1 Hz, 1H), 5.57-5.72 (m, 2H), 5.80 (d, J = 15.3 Hz, 1H), 6.13 (br.d, J = 10.4 Hz, 1H), 6.49 (dd, J = 15.3, 11.0 Hz, 1H), 9.17 (d, J = 2.5 Hz, 2H), 9.24 (t, J = 1.8 Hz, 1H).

### C-17-(2)

### Synthesis of pladienolide D

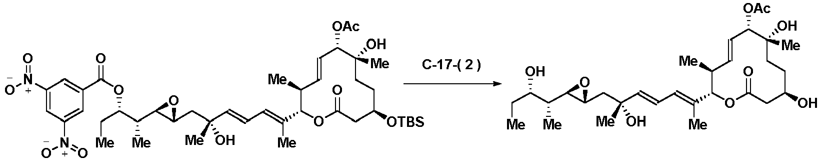

To a THF solution (0.45 mL) of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate (15.1 mg, 18 µmol) obtained in Example C-17-(1), a THF solution of TBAF (1 M; 105 µL, 105 µmol) was added. The resulting mixture was stirred overnight at room temperature. A 10% aqueous ammonium chloride solution and ethyl acetate were added to the ice-cooled reaction mixture, and then the organic layer was separated. The aqueous layer was subjected to extraction with ethyl acetate. The combined organic layer was dried over anhydrous sodium sulfate, filtered, and then concentrated under reduced pressure. The resulting residue was purified by silica gel column chromatography (0%-100% ethyl acetate/n-heptane) to obtain the title compound (8.6 mg).

The ¹H-NMR spectrum (500 MHz, CD₃OD) of the obtained compound was consistent with the NMR spectrum in Example C-1.

### Example C-18

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

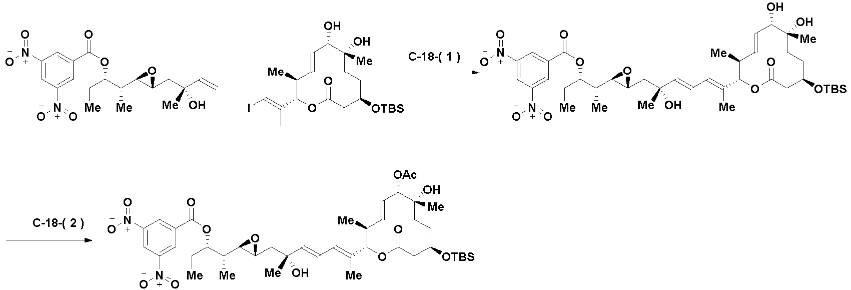

### C-18-(1)

### Synthesis of (2R,3S)-2-((2R,3R)-3-((R,3E,5E)-6-((2S,3S,6S,7R,10R,E)-10-((t-butyldimethylsilyl)oxy)-6,7-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

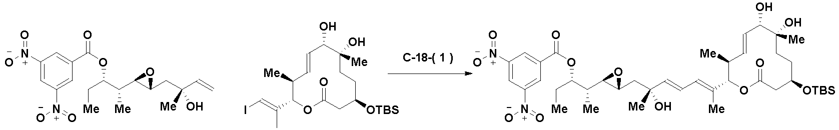

A DMF solution (1 mL) of (2R,3S)-2-((2R,3R)-3-((R)-2-hydroxy-2-methylbut-3-en-1-yl)oxiran-2-yl)pentan-3-yl 3,5-dinitrobenzoate (22.8 mg, 56 µmol) obtained in Example A-5 and (4R,7R,8S,11S,12S,E)-4-((t-butyldimethylsilyl)oxy)-7,8-dihydroxy-12-((E)-1-iodoprop-1-en-2-yl)-7,11-dimethyloxacyclododec-9-en-2-one (20 mg, 37 µmol) obtained in Example B-5 was degassed and replaced with a nitrogen atmosphere. To this mixture, Pd(OAc)₂ (2.1 mg, 9.3 µmol) and Ag₂CO₃ (13.3 mg, 48 µmol) were added. The resulting reaction mixture was stirred at room temperature for 15 minutes and then at 80°C for 2 hours. The reaction mixture was allowed to cool, and then concentrated under reduced pressure, and the resulting residue was suspended in ethyl acetate, and insoluble matter was filtered off through a Millipore filter. The filtrate was concentrated under reduced pressure. The residue was purified by silica gel column chromatography (30%-65% ethyl acetate/n-heptane) to obtain the title compound (19.9 mg).

¹H-NMR (500 MHz, CDCl₃) δ (ppm): 0.06 (s, 3H), 0.07 (br.s, 3H), 0.87-0.91 (m, 12H), 0.97 (br.t, J = 7.3 Hz, 3H), 1.06 (d, J = 7.3 Hz, 3H), 1.30 (s, 3H), 1.34 (s, 3H), 1.35-1.50 (m, 4H), 1.58-1.71 (m, 3H), 1.72 (s, 3H), 1.78-1.94 (m, 4H), 2.34-2.41 (m, 2H), 2.41-2.55 (m, 2H), 2.60 (dd, J = 8.3, 2.1 Hz, 1H), 2.91 (td, J = 4.7, 2.1 Hz, 1H), 3.74-3.87 (m, 2H), 4.97 (d, J = 10.4 Hz, 1H), 5.24-5.32 (m, 1H), 5.46 (dd, J = 15.0, 10.1 Hz, 1H), 5.71 (dd, J = 15.0, 9.5 Hz, 1H), 5.80 (d, J = 15.3 Hz, 1H), 6.12 (br.d, J = 10.4 Hz, 1H), 6.50 (dd, J = 15.3, 11.0 Hz, 1H), 9.17 (d, J = 2.5 Hz, 2H), 9.20-9.25 (m, 1H).

### C-18-(2)

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

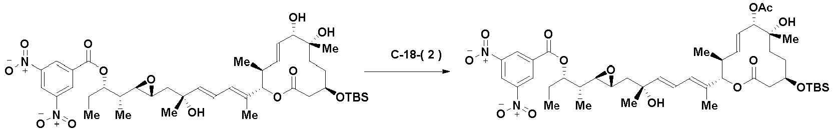

To a solution of (2R,3S)-2-((2R,3R)-3-((R,3E,5E)-6-((2S,3S,6S,7R,10R,E)-10-((t-butyldimethylsilyl)oxy)-6,7-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate (17.0 mg, 21 µmol) obtained in Example C-18-(1), DMAP (0.51 mg, 4.2 µmol) and Et₃N (8.7 µL, 62 µmol) in DCM (1 ml), a DCM solution of Ac₂O (1 M; 21 µL, 21 µmol) was added under ice-cooling, and the mixture was stirred at 0°C for 40 minutes. A saturated aqueous sodium bicarbonate solution, ethyl acetate, and water were added to the reaction mixture, and the organic layer was separated. The resulting organic layer was washed with saturated saline, dried over anhydrous magnesium sulfate, filtered, and then concentrated under reduced pressure. The residue was purified by silica gel column chromatography (30%-65% ethyl acetate/n-heptane) to obtain the title compound (14.7 mg).

The ¹H-NMR spectrum (500 MHz, CDCl₃) of the obtained compound was consistent with the NMR spectrum in Example C-17-(1).

According to the method of Example C-17-(2), pladienolide D can be synthesized from the obtained compound.

### Example C-19

### Synthesis of pladienolide D

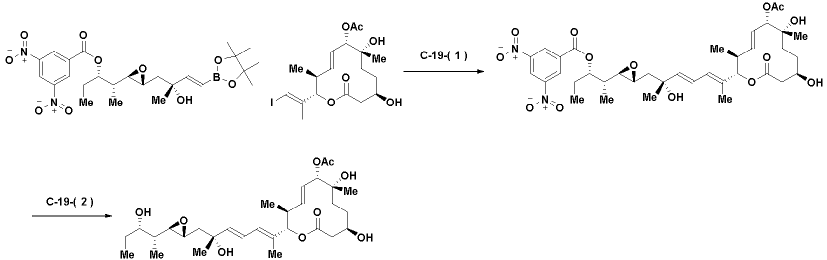

### C-19-(1)

### Synthesis of (2R,3S)-2-((2R,3R)-3-((R,3E,5E)-6-((2S,3S,6S,7R,10RE)-6-acetoxy-7,10-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

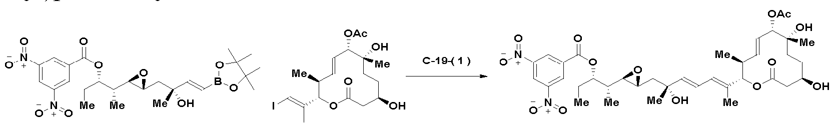

To a solution of (2S,3S,6S,7R,10R,E)-7,10-dihydroxy-2-((E)-1-iodoprop-1-en-2-yl)-3,7-dimethyl-12-oxooxacyclododec-4-en-6-yl acetate (10 mg, 21 µmol) obtained in Example B-8 and (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate (12.6 mg, 24 µmol) obtained in Example A-11 in THF (1 mL) and water (0.1 mL), Ag₂O (24.9 mg, 0.107 mmol), Pd₂(dba)₃ (3.93 mg, 4.29 µmol), and Pd(dppf)Cl₂ (3.14 mg, 4.29 µmol) were added. The reaction mixture was stirred overnight at room temperature under a nitrogen atmosphere. The reaction mixture was filtered. The filtrate was concentrated under reduced pressure. The resulting residue was purified by silica gel column chromatography (0%-100% ethyl acetate/n-heptane) to obtain the title compound (10.4 mg).

The ¹H-NMR spectrum (500 MHz, CDCl₃) of the obtained compound was consistent with the NMR spectrum in Example C-16-(1).

### C-19-(2)

### Synthesis of pladienolide D

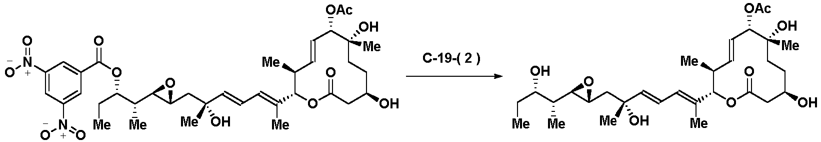

To a solution of (2R,3S)-2-((2R,3R)-3-((R,3E,5E)-6-((2S,3S,6S,7R,10R,E)-6-acetoxy-7,10-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate (4.8 mg, 6.4 µmol) obtained in Example C-19-(1) in THF (1 mL), a 0.1 N aqueous lithium hydroxide solution (64 µL, 6.4 µmol) was added at 0° C. The reaction mixture was stirred at 0°C for 30 minutes and then stirred at room temperature for 2 hours. A saturated aqueous ammonium chloride solution, DCM, and water were added to the reaction liquid, and the organic layer was separated. The aqueous layer was subjected to extraction with DCM (twice). The combined organic layer was dried over anhydrous sodium sulfate, filtered, and then concentrated under reduced pressure. The resulting residue was purified by silica gel column chromatography (0%-100% ethyl acetate/n-heptane) to obtain the title compound (3.0 mg).

The ¹H-NMR spectrum (500 MHz, CD₃OD) of the obtained compound was consistent with the NMR spectrum in Example C-1.

### Example C-20

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

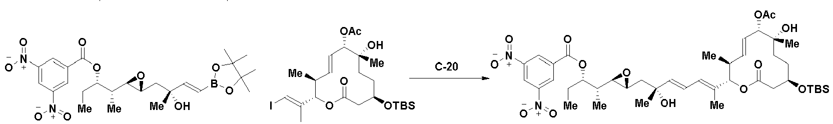

To a solution of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclododec-4-en-6-yl acetate (10 mg, 17 µmol) obtained in Example B-6 and (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate (13 mg, 24 µmol) obtained in Example A-10 in THF (1 mL) and water (0.1 mL), Ag₂O (20.0 mg, 86 µmol) and Pd(dppf)Cl₂ (2.52 mg, 3.45 µmol) were added at room temperature. The reaction mixture was stirred under a nitrogen atmosphere at room temperature for 4 hours. Insoluble matter was filtered off from the reaction mixture with a filter paper and washed with ethyl acetate. The filtrate was concentrated. The resulting residue was purified by silica gel column chromatography (0%-50% ethyl acetate/n-heptane) to obtain the title compound (13.3 mg).

The ¹H-NMR spectrum (500 MHz, CDCl₃) of the obtained compound was consistent with the NMR spectrum in Example C-17-(1).

According to the method of Example C-17-(2), pladienolide D can be synthesized from the obtained compound.

### Example C-21

### Synthesis of (2R,3S)-2-((2R,3R)-3-((R,3E,5E)-6-((2S,3S,6S,7R,10R,E)-10-((t-butyldimethylsilyl)oxy)-6,7-dihydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

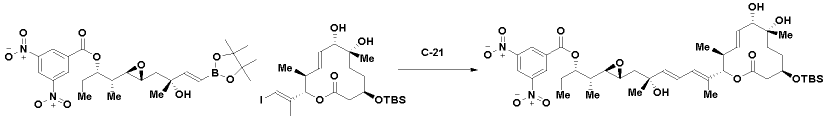

To a solution of (4R,7R,8S,11S,12S,E)-4-((t-butyldimethylsilyl)oxy)-7,8-dihydroxy-12-((E)-1-iodoprop-1-en-2-yl)-7,11-dimethyloxacyclododec-9-en-2-one (13 mg, 24 µmol) obtained in Example B-5 and (2R,3S)-2-((2R,3R)-3-((R,E)-2-hydroxy-2-methyl-4-(4,4,5 5-tetramethyl-1,3,2-dioxaborolan-2-yl)but-3-en-1-yl)oxiran-2-yl)pentan-3-yl 3,5-dinitrobenzoate (18 mg, 34 µmol) obtained in Example A-11 in THF (1 mL), Pd(dppf)Cl₂ (3.53 mg, 4.83 µmol), Ag₂O (28 mg, 0.121 mmol), and water (0.15 mL, 8.33 mmol) were sequentially added. The resulting reaction mixture was stirred at room temperature for 110 minutes. Ethyl acetate and water were added to the ice-cooled reaction mixture, and the organic layer was separated. The organic layer was washed with saturated saline, dried over anhydrous sodium sulfate, filtered, and then concentrated under reduced pressure. The resulting residue was purified with (0%-60% ethyl acetate/n-heptane) to obtain the title compound (18.9 mg).

The ¹H-NMR (500 MHz, CDCl₃) spectrum of the obtained compound was consistent with the NMR spectrum in Example C-18-(1).

Pladienolide D can be synthesized from the obtained compound according to the method of Example C-18-(2), followed by the method of Example C-17-(2).

### Example C-22

### Synthesis of (2S,3S,6S,7R,10R,E)-10-((t-butyldimethylsilyl)oxy)-7-hydroxy-2-((R,2E,4E)-6-hydroxy-7-((2R,3R)-3-((2R,3S)-3-hydroxypentan-2-yl)oxiran-2-yl)-6-methylhepta-2,4-dien-2-yl)-3,7-dimethyl-12-oxooxacyclododec-4-en-6-yl acetate

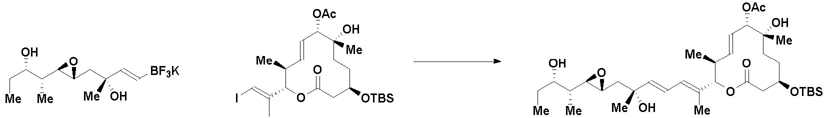

To a solution of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclododec-4-en-6-yl acetate (6.5 mg, 11 µmol) obtained in Example B-6 and potassium trifluoro((R,E)-3-hydroxy-4-((2R,3R)-3-((2R,3S)-3-hydroxypentan-2-yl)oxiran-2-yl)-3-methylbut-1-en-1-yl)borate (5.38 mg, 17 µmol) obtained in Example A-14 in THF (1 mL) and water (0.1 mL), Ag₂O (13.0 mg, 56 µmol) and Pd(dppf)Cl₂ (1.64 mg, 2.24 µmol) were added. The reaction mixture was stirred under nitrogen atmosphere at room temperature for 4 hours. The reaction mixture was filtered through a glass microfiber filter paper. The filtrate was concentrated under reduced pressure. The resulting residue was purified by silica gel column chromatography (0%-50% ethyl acetate/n-heptane) to obtain the title compound (5.3 mg).

The ¹H-NMR spectrum (500 MHz, CDCl₃) of the obtained compound was consistent with the NMR spectrum in Example C-2-(1).

According to the method of Example C-2-(2), pladienolide D can be synthesized from the obtained compound.

### Example C-23

### Synthesis of pladienolide D

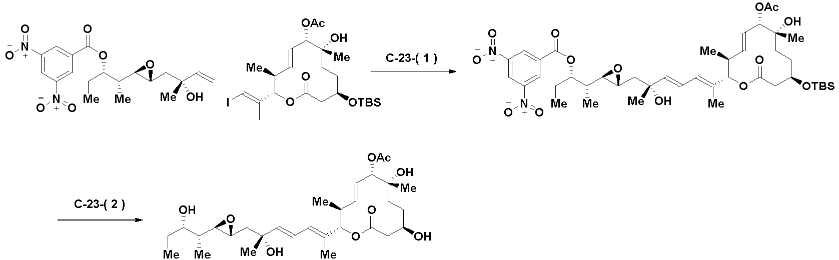

### C-23-(1)

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

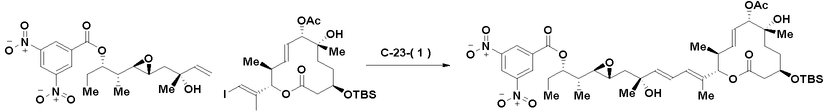

A solution of (2R,3S)-2-((2R,3R)-3-((R)-2-hydroxy-2-methylbut-3-en-1-yl)oxiran-2-yl)pentan-3-yl 3,5-dinitrobenzoate (158 mg, 388 µmol) obtained in Example A-5 and (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclodec-4-en-6-yl acetate (150 mg, 258 µmol) obtained in Example B-6 in DMF (3 mL) was replaced with a nitrogen atmosphere. To this mixture, Pd(OAc)2 (14.5 mg, 65 µmol) and Ag₂CO₃ (93 mg, 336 µmol) were sequentially added and the resulting mixture was stirred under a nitrogen atmosphere at 60°C for 24 hours and then at 80°C for 2 hours. To the reaction mixture, ethyl acetate and water were added at room temperature. Insoluble matter in the mixture was filtered off through a glass fiber filter paper, and the filtrate was separated into layers. A 5% aqueous sodium chloride solution was added to the organic layer obtained by layer separation, the mixture was filtered through celite, and the organic layer was separated from the filtrate and washed with a 5% aqueous sodium chloride solution. The aqueous layer obtained by layer separation was subjected to extraction with ethyl acetate, and the organic layer was washed with a 5% aqueous sodium chloride solution. The combined organic layer was dried over anhydrous magnesium sulfate, filtered, and then concentrated under reduced pressure. The resulting residue was purified by silica gel column chromatography (35%-65% ethyl acetate/n-heptane) to obtain the title compound (142 mg). The ¹H-NMR spectrum (500 MHz, CDCl₃) of the obtained compound was consistent with the NMR spectrum of the compound obtained in Example C-17-(1).

### C-23-(2)

### Synthesis of pladienolide D

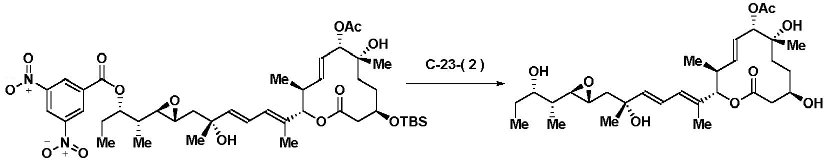

To a solution of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate (100 mg, 116 µmol) obtained in C-23-(1) in THF (2 ml), a THF solution of TBAF (1 M; 0.70 mL,700 µmol) was added. The resulting mixture was stirred at room temperature for 20 hours. The ice-cooled reaction mixture was quenched with a saturated aqueous ammonium chloride solution, and ethyl acetate and water were added to the resulting mixture, then the organic layer was separated. The aqueous layer was subjected to extraction with ethyl acetate. The combined organic layer was dried over anhydrous sodium sulfate, filtered, and then concentrated under reduced pressure. The resulting residue was purified by silica gel column chromatography (50%-100% ethyl acetate/n-heptane) to obtain the title compound (49.4 mg). The ¹H-NMR spectrum (500 MHz, CD₃OD) of the obtained compound was consistent with the NMR spectrum in Example C-1.

### Example C-24

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

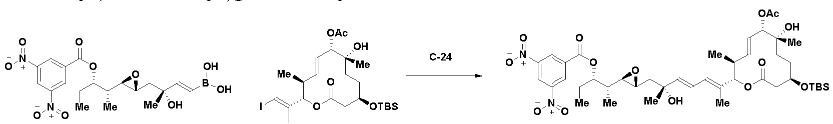

To a solution of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclododec-4-en-6-yl acetate (10 mg, 17 µmol) obtained in Example B-6 and ((R,E)-4-((2R,3R)-3-((2R,3S)-3-((3,5-dinitrobenzoyl)oxy)pentan-2-yl)oxiran-2-yl)-3-hydroxy-3-methylbut-1-en-1-yl)boronic acid (9.35 mg, 21 µmol) obtained in Example A-17 in THF (1 mL) and water (0.1 mL),Ag₂O (4.0 mg, 17 µmol) and Pd(dppf)Cl₂ (2.52 mg, 3.45 µmol) were added. The reaction mixture was stirred under a nitrogen atmosphere at room temperature for 3 hours. The reaction mixture was filtered. The filtrate was concentrated under reduced pressure. The resulting residue was purified by silica gel column chromatography (0%-50% ethyl acetate/n-heptane) to obtain the title compound (14.8 mg).

The ¹H-NMR (500 MHz, CDCl₃) spectrum of the obtained compound was consistent with the NMR spectrum in Example C-17-(1). This compound can be led to pladienolide D by the method of Example C-17-(2).

### Example D-1

### Powderization of pladienolide D

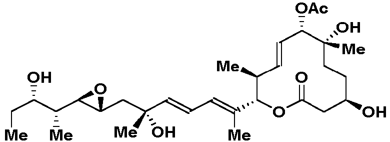

Ethyl acetate and water were added to a mixed solution of pladienolide D obtained by fermentation according to the method described in Patent Literature 1 in n-butyl acetate and DMF (content: 38.1% w/w; 11.3 g, 7.79 mmol), and the organic layer was separated. The organic layer was washed sequentially with water and a 5% aqueous sodium chloride solution. The resulting organic layer was dried over anhydrous sodium sulfate, filtered, and then concentrated under reduced pressure to obtain the title compound (4.64 g; containing 9.16 wt% of n-butyl acetate as a residual solvent) as a pale yellow powder. The ¹H-NMR spectrum (500 MHz, CD₃OD) of the obtained title compound was consistent with the NMR spectrum in Example C-1.

### Example D-2

### Production of pladienolide D toluene solvate

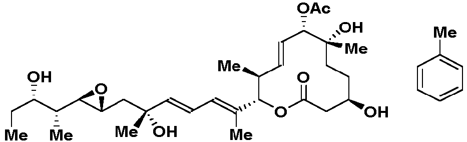

In a 4 mL vial, the pladienolide D (200 mg) obtained in Example D-1 was weighed, and toluene (1 mL) was added. Stirring was performed with a stirrer at room temperature for 12 days. The lid of the vial was loosened and stirring was performed with a stirrer for 2 days while the solvent was splashed. The resultant was dried under reduced pressure at room temperature for 1 day to obtain a crystal of the mentioned toluene solvate.

¹H-NMR (700 MHz, CD₃OD) δ (ppm): 0.88 (3H, d, J = 6.8 Hz), 0.89 (3H, d, J = 7.0 Hz), 0.93 (3H, t, J = 7.4 Hz), 1.18 (3H, s), 1.22-1.27 (1H, m), 1.33 (3H, s), 1.35-1.40 (1H, m), 1.44-1.59 (3H, m), 1.60-1.69 (3H, m), 1.77 (3H, d, J = 0.6 Hz), 1.85 (1H, dd, J = 13.9, 5.6 Hz), 2.05 (3H, s), 2.31 (1.5H, s), 2.46-2.54 (2H, m), 2.54-2.60 (1H, m), 2.66 (1H, dd, J = 8.1, 2.1 Hz), 2.89 (1H, td, J = 5.6, 2.1 Hz), 3.52 (1H, dt, J = 8.5, 4.2 Hz), 3.74-3.81 (1H, m), 4.99-5.11 (2H, m), 5.56 (1H, dd, J = 15.0, 10.0 Hz), 5.69 (1H, dd, J = 15.0, 9.7 Hz), 5.86 (1H, d, J = 15.3 Hz), 6.13 (1H, d, J = 10.8 Hz), 6.52 (1H, dd, J = 15.3, 10.8 Hz), 7.08-7.12 (0.5H, m), 7.14 (1H, d, J = 7.6 Hz), 7.17-7.22 (1H, m).

### Example D-3

### Production of pladienolide D chlorobenzene solvate

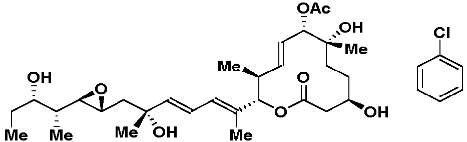

In a 4 mL vial, the pladienolide D (200 mg) obtained in Example D-1 was weighed, and chlorobenzene (0.5 mL) was added. Stirring was performed with a stirrer at room temperature for 1 day. The lid of the vial was removed and stirring was performed with a stirrer for 2 days while the solvent was splashed. The resultant was dried under reduced pressure at room temperature for 1 day to obtain a crystal of the mentioned chlorobenzene solvate.

¹H-NMR (700 MHz, CD₃OD) δ (ppm): 0.88 (3H, d, J = 6.8 Hz), 0.89 (3H, d, J = 7.2 Hz), 0.93 (3H, t, J = 7.4 Hz), 1.18 (3H, s), 1.22-1.25 (1H, m), 1.33 (3H, s), 1.35-1.42 (1H, m), 1.43-1.60 (3H, m), 1.60-1.69 (3H, m), 1.77 (3H, d, J = 1.1 Hz), 1.86 (1H, dd, J = 14.0, 5.5 Hz), 2.05 (3H, s), 2.48-2.54 (2H, m), 2.54-2.60 (1H, m), 2.66 (1H, dd, J = 7.8, 2.4 Hz), 2.89 (1H, td, J = 5.5, 2.4 Hz), 3.52 (1H, dt, J = 8.5, 4.4 Hz), 3.74-3.80 (1H, m), 5.00-5.08 (2H, m), 5.56 (1H, dd, J = 15.3, 10.0 Hz), 5.69 (1H, dd, J = 15.3, 9.7 Hz), 5.86 (1H, d, J = 15.3 Hz), 6.13 (1H, brd, J = 11.1 Hz), 6.52 (1H, dd, J = 15.3, 11.1 Hz), 7.25-7.37 (3H, m).

### Example D-4

### Production of pladienolide D tetrahydrofuran solvate

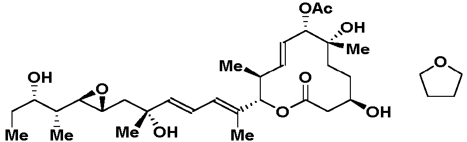

In a 4 mL vial, the pladienolide D (200 mg) obtained in Example D-1 was weighed, and tetrahydrofuran (0.2 mL) and n-heptane (0.8 mL) were added. Stirring was performed with a stirrer at room temperature for 1 day. The lid of the vial was removed and stirring was performed with a stirrer for 1 day while the solvent was splashed. The resultant was dried under reduced pressure at room temperature for 1 day to obtain a crystal of the mentioned tetrahydrofuran solvate.

¹H-NMR (700 MHz, CD₃CN) δ (ppm): 0.80 (3H, d, J = 6.9 Hz), 0.83 (3H, d, J = 6.8 Hz), 0.88 (3H, t, J = 7.2 Hz), 1.11 (3H, s), 1.21-1.27 (1H, m), 1.26-1.31 (1H, m), 1.27 (3H, s), 1.28-1.34 (1H, m), 1.37-1.48 (2H, m), 1.46-1.52 (1H, m), 1.50-1.56 (1H, m), 1.56 (1H, dd, J = 14.0, 6.8 Hz), 1.74 (3H, d, J = 1.1 Hz), 1.76-1.81 (3H, m), 1.82 (1H, dd, J = 14.0, 5.5 Hz), 2.01 (3H, s), 2.46 (1H, dd, J = 14.6, 4.0 Hz), 2.49 (1H, dd, J = 14.6, 3.4 Hz), 2.54-2.59 (1H, m), 2.60 (1H, dd, J = 7.4, 2.3 Hz), 2.79-2.84 (1H, m), 2.81 (1H, d, J = 5.5 Hz), 2.99 (1H, s), 3.22 (1H, s), 3.25 (1H, d, J = 8.7 Hz), 3.45-3.50 (1H, m), 3.58-3.66 (3H, m), 3.66-3.73 (1H, m), 4.98 (1H, d, J = 9.7 Hz), 5.03 (1H, d, J = 10.6 Hz), 5.51 (1H, dd, J = 15.2, 9.9 Hz), 5.64 (1H, dd, J = 15.2, 9.7 Hz), 5.86 (1H, d, J = 15.2 Hz), 6.10 (1H, d, J = 10.6 Hz), 6.46 (1H, dd, J = 15.2, 10.6 Hz).

### Example D-5

### Production of pladienolide D 2-methyltetrahydrofuran solvate

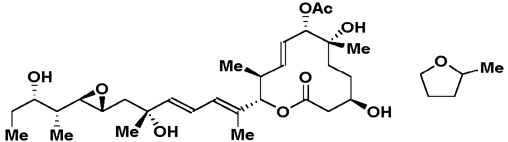

The pladienolide D (300 mg) obtained in Example D-1 was dissolved in 2-methyltetrahydrofuran (1.2 mL), and n-heptane (0.3 mL) was added dropwise thereto at room temperature, then the mixture was stirred at room temperature for 10 minutes. The resulting mixture was stirred at 40°C for 25 minutes, at room temperature for 2 hours, and further at 0°C for 21 hours. The precipitated crystal was collected by filtration, washed with a 2 : 1 mixed solution of n-heptane and 2-methyltetrahydrofuran, and dried under an air stream to obtain a crystal of the mentioned tetrahydrofuran solvate (157 mg).

¹H-NMR (500 MHz, CD₃OD) δ (ppm): 0.89 (d, J = 6.9 Hz, 3H), 0.90 (d, J = 7.5 Hz, 3H), 0.94 (t, J = 7.5 Hz, 3H), 1.19 (s, 3H), 1.21 (d, J = 6.3 Hz, 3H), 1.23-1.28 (m, 1H), 1.34 (s, 3H), 1.36-1.39 (m, 1H), 1.39-1.70 (m, 7H), 1.78 (s, 3 H), 1.87 (dd, J = 14.3, 5.7 Hz, 1H), 1.89-1.98 (m, 2H), 1.99-2.04 (m, 1H), 2.06 (s, 3H), 2.51-2.54 (m, 2H), 2.55-2.61 (m, 1H), 2.67 (dd, J = 7.5, 2.3 Hz, 1H), 2.90 (td, J = 6.0, 2.3 Hz, 1H), 3.53 (dt, J = 8.3, 4.4 Hz, 1H), 3.70 (td, J = 8.0, 6.3 Hz, 1H), 3.79 (br dd, J = 9.7, 3.4 Hz, 1H), 3.84-3.90 (m, 1H), 3.95 (dt, J = 7.7, 6.2 Hz, 1H), 5.05 (dd, J = 10.0, 6.6 Hz, 2H), 5.57 (dd, J = 15.2, 10.0 Hz, 1H), 5.67-5.74 (m, 1H), 5.87 (d, J = 15.5 Hz, 1H), 6.14 (d, J = 10.9 Hz, 1H), 6.53 (dd, J = 15.2, 11.2 Hz, 1H).

### Example D-6

### Production of pladienolide D 2-methyltetrahydrofuran solvate

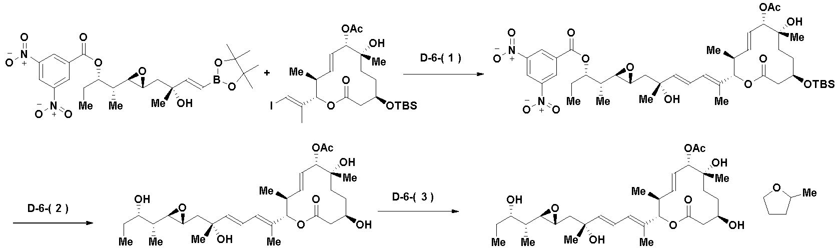

### D-6-(1)

### Synthesis of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate

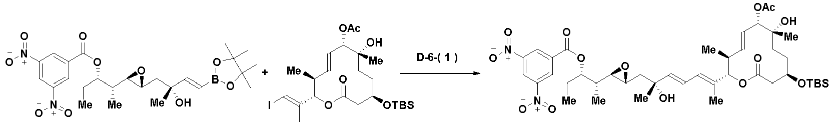

To a solution of (2S,3S,6S,7R,10R,E)-10-((t-butyldimethylsilyl)oxy)-7-hydroxy-2-((E)-1-iodoprop-1-en-2-yl)-3,7-dimethyl-12-oxooxacyclododec-4-en-6-yl acetate (802 mg, 1.38 mmol) obtained in Example B-6 and (2R,3S)-2-((2R,3R)-3-((R,E)-2-hydroxy-2-methyl-4-(4,4,5,5-tetramethyl-1,3,2-dioxaborolan-2-yl)but-3-en-1-yl)oxiran-2-yl)pentan-3-yl 3,5-dinitrobenzoate (920 mg, weight content from NMR: 96.3%, 1.66 mmol) obtained in Example A-16-(2) in THF (80 mL) and water (8 mL), Ag₂O (1.60 g, 6.91 mmol) and Pd(dppf)Cl₂ (202 mg, 0.276 mmol) were added, and the mixture was stirred at room temperature for 2 hours. The reaction liquid was filtered, and the filter cake was washed with ethyl acetate, then the filtrate was concentrated. Water and ethyl acetate were added to the residue, and the organic layer was separated. The aqueous layer was subjected to re-extraction with ethyl acetate. The combined organic layer was washed with a saturated aqueous sodium chloride solution. The organic layer was dried over anhydrous sodium sulfate, filtered, and then concentrated under reduced pressure. The resultant was purified by silica gel column chromatography (0%-50% ethyl acetate/n-heptane) to obtain the title compound (1.40 g, crude weight). The ¹H-NMR spectrum of the mentioned compound as a main component was consistent with the NMR spectrum of the compound obtained in Example C-17-(1).

### D-6-(2)

### Synthesis of pladienolide D

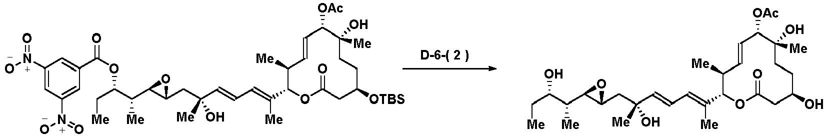

To a solution of (2R,3S)-2-((2R,3R)-3-((R,3E,5E)-6-((2S,3S,6S,7R,10R,E)-6-acetoxy-10-((t-butyldimethylsilyl)oxy)-7-hydroxy-3,7-dimethyl-12-oxooxacyclododec-4-en-2-yl)-2-hydroxy-2-methylhepta-3,5-dien-1-yl)oxiran-2-yl)pentan-3-yl 3,5-dinitrobenzoate (1.40 g, crude weight) obtained in Example D-6-(1) in THF (14 ml), a THF solution of TBAF (1 M; 9.76 mL, 9.76 mmol) was added at room temperature. The resulting mixture was stirred at room temperature for 16 hours. Subsequently, to the reaction mixture, a THF solution of TBAF (1 M; 9.76 mL, 9.76 mmol) was added, and the reaction mixture was stirred for an additional 25 hours. The reaction mixture was quenched with a saturated aqueous ammonium chloride solution. Ethyl acetate and water were added to the obtained mixture, and the organic layer was separated. The aqueous layer was subjected to extraction with ethyl acetate. The combined organic layer was washed with a saturated aqueous sodium chloride solution, dried over anhydrous sodium sulfate, filtered, and then concentrated under reduced pressure. The residue was dissolved in ethyl acetate, and the resultant washed sequentially with a saturated aqueous ammonium chloride solution (twice) and a saturated aqueous sodium bicarbonate solution (twice). The combined aqueous layer was subjected to extraction with ethyl acetate (twice). The combined ethyl acetate layer was washed with saturated saline, dried over anhydrous sodium sulfate, filtered, and then concentrated under reduced pressure. The resulting residue was purified by silica gel column chromatography (ethyl acetate), and then re-purified by NH silica gel column chromatography (0%-100% MeOH/ethyl acetate) to obtain the title compound (504 mg, weight content from NMR: 96.3%). The ¹H-NMR spectrum (500 MHz, CD₃OD) of the mentioned compound was consistent with the NMR spectrum in Example C-1.

### D-6-(3)

### Production of pladienolide D 2-methyltetrahydrofuran solvate

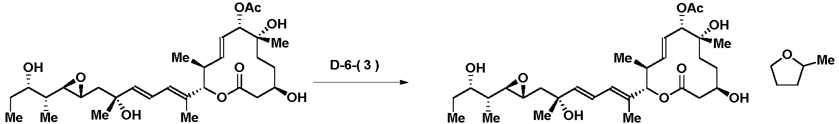

To a solution of the pladienolide D (98.7 mg, weight content from NMR: 96.3%, 172 µmol) obtained in Example D-6-(2) in 2-methyltetrahydrofuran (0.5 mL), n-heptane (0.5 mL) was added in 5 portions of 0.1 ml at room temperature over 10 minutes, and the mixture was stirred for 30 minutes. Then, to the mixture, a seed crystal of the pladienolide D 2-methyltetrahydrofuran solvate obtained in Example D-5 was added, and the mixture was stirred at room temperature for 15 minutes. A crystal adhering to the wall was dropped with ultrasonic waves, and further the mixture was stirred at room temperature for 45 minutes. Thereafter, the mixture was gradually cooled to 0°C over 40 minutes, and further stirred overnight at 0°C. The precipitated crystal was collected by filtration, washed with a 2 : 1 mixed solution (2 mL) of n-heptane and 2-methyl-THF, and dried under reduced pressure to obtain the title compound (98.9 mg).

¹H-NMR (700 MHz, CD₃OD) δ (ppm): 0.87 (3H, d, J = 6.6 Hz), 0.89 (3H, d, J = 7.0 Hz), 0.93 (3H, t, J = 7.5 Hz), 1.18 (3H, brs), 1.20 (3H, d, J = 6.2 Hz), 1.21-1.26 (1H, m), 1.33 (3H, s)1.34-1.39 (1H, m), 1.41-1.46 (1H, m), 1.46-1.51 (1H, m), 1.51-1.55 (1H, m), 1.54-1.59 (1H, m), 1.60-1.66 (1H, m), 1.60-1.64 (1H, m), 1.65 (1H, dd, J = 14.1, 6.6 Hz), 1.77 (3H, s), 1.86 (1H, dd, J = 14.1, 5.7 Hz), 1.88-1.97 (2H, m), 2.00-2.04 (1H, m), 2.05 (3H, s), 2.50-2.53 (2H, m), 2.54-2.60 (1H, m), 2.66 (1H, dd, J = 7.9, 2.2 Hz), 2.89 (1H, ddd, J = 6.6, 5.7, 2.2 Hz), 3.52 (1H, dt, J = 8.4, 4.4 Hz), 3.69 (1H, td, J = 8.3, 6.6 Hz), 3.75-3.80 (1H, m), 3.86 (1H, td, J = 7.9, 6.6 Hz), 3.92-3.97 (1H, m), 5.04 (1H, d, J = 9.6 Hz), 5.06 (1H, d, J = 10.2 Hz), 5.56 (1H, dd, J = 15.4, 10.1 Hz), 5.69 (1H, dd, J = 15.4, 9.6 Hz), 5.86 (1H, d, J = 15.4 Hz), 6.13 (1H, d, J = 10.6 Hz), 6.52 (1H, dd, J = 15.4, 10.6 Hz).

### Example E. Measurement of purity

The pladienolide D obtained in Example D-6-(2) and the pladienolide D 2-methyltetrahydrofuran solvate obtained in Example D-6-(3) were dissolved in water/acetonitrile (1/1, v/v) to prepare solutions having a concentration of 0.5 mg/mL. Measurement was performed under the following high performance liquid chromatography (HPLC) conditions, and HPLC purity was evaluated. The results are shown in Table 2.

### [HPLC conditions]

Column: Sun Armor C18, 4.6 mm × 250 mm, 3 µm
Mobile phase A: water/acetonitrile (77/23, v/v)
Mobile phase B: Acetonitrile
Flow rate: 0.9 mL/min
Column temperature: 30°C
Detection wavelength: 240 nm
Injection amount: 10 µL
Gradient conditions

**[Table 1]**

| Time (min) | Mobile phase B (%) |
|---|---|
| 0 | 0 |
| 110 | 0 |
| 120 | 15 |
| 130 | 80 |
| 135 | 80 |
| 135.01 | 0 |
| 150 | 0 |

**[Table 2]**

| Retention time (min) | HPLC (area%) | |
|---|---|---|
| | Example D-6-(2) | Example D-6-(3) |
| 38.5 | 2.42 | 0.22 |
| 75.5 | 89.92 | 96.75 |
| 81.5 | 1.53 | 0.81 |
| 86.8 | 3.36 | 1.40 |
| 93.8 | 1.02 | 0.41 |

The retention time of the pladienolide D and the pladienolide D 2-methyltetrahydrofuran solvate is 75.5 minutes. In the pladienolide D 2-methyltetrahydrofuran solvate obtained in Example D-6-(3), the proportion of impurities was reduced by crystallization and washing, and improvement of the purity was verified.

## Claims

1. A method of producing pladienolide D represented by formula (1): 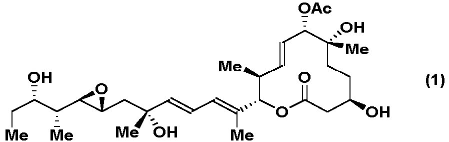 or a salt thereof, or a solvate thereof,
the method comprising the following steps:
step 2-1) allowing a compound represented by formula (A2): 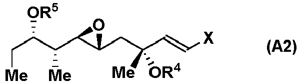
wherein X is hydrogen or optionally substituted boryl, R⁴ is hydrogen or a silyl protecting group, and R⁵ is hydrogen or optionally substituted benzoyl, to react with a compound represented by formula (B1): 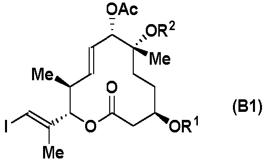
wherein R¹ and R² are each independently hydrogen or a silyl protecting group,
in the presence of a metal catalyst to obtain a compound represented by formula (C2): 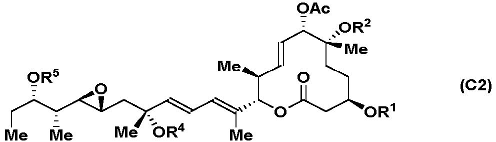
wherein R¹, R², and R⁴ are each independently hydrogen or a silyl protecting group, and R⁵ is hydrogen or optionally substituted benzoyl;
step 2-2) removing the protecting group(s) of the compound represented by formula (C2) obtained in step 2-1 to obtain pladienolide D; and
optionally, step 2-3) converting pladienolide D obtained in step 2-2 to a solvate.

2. The method according to claim 1, wherein R⁵ is hydrogen.

3. The method according to claim 1, wherein R⁵ is 2,4-dinitrobenzoyl.

4. The method according to claim 1, wherein R⁵ is 3,5-dinitrobenzoyl.

5. The method according to any one of claims 1 to 4, wherein R⁴ is hydrogen.

6. The method according to any one of claims 1 to 5, wherein R² is hydrogen.

7. The method according to any one of claims 1 to 6, wherein R¹ is hydrogen.

8. The method according to any one of claims 1 to 6, wherein R¹ is a silyl protecting group.

9. The method according to any one of claims 1 to 6, wherein R¹ is tert-butyl(dimethyl)silyl.

10. The method according to any one of claims 1 to 9, wherein X is hydrogen.

11. The method according to any one of claims 1 to 9, wherein X is optionally substituted boryl.

12. The method according to any one of claims 1 to 9, wherein X is boronic acid, pinacol boronate ester, or trifluoroborate.

13. The method according to any one of claims 1 to 12, wherein the pladienolide D or the salt thereof or the solvate thereof is pladienolide D 2-methyltetrahydrofuran solvate.

14. The method according to any one of claims 1 to 13, wherein the metal catalyst is a palladium catalyst.

15. The method according to any one of claims 1 to 13, wherein the metal catalyst is palladium(II) acetate.

16. The method according to any one of claims 1 to 13, wherein the metal catalyst is tris(dibenzylideneacetone)dipalladium(0).

17. The method according to any one of claims 1 to 13, wherein the metal catalyst is (1,1'-bis(diphenylphosphino)ferrocene)dichloropalladium(II).

18. The method according to any one of claims 1 to 17, wherein the step of removing the protecting group(s) comprises removal using an acid and removal using a base.

19. The method according to any one of claims 1 to 17, wherein the step of removing the protecting group(s) comprises removal using a fluoride ion and removal using a base.

20. The method according to any one of claims 1 to 17, wherein the step of removing the protecting group(s) comprises removal using a base.

21. The method according to any one of claims 1 to 17, wherein the step of removing the protecting group(s) comprises removal using a fluoride ion.

22. The method according to any one of claims 1 to 17, wherein the step of removing the protecting group(s) comprises removal using lithium hydroxide.

23. The method according to any one of claims 1 to 17, wherein the step of removing the protecting group(s) comprises removal using tetrabutylammonium fluoride.

24. The method according to any one of claims 1 to 17, wherein the step of removing the protecting group(s) comprises removal using tetrabutylammonium fluoride and removal using lithium hydroxide.

25. A compound represented by formula (C5): 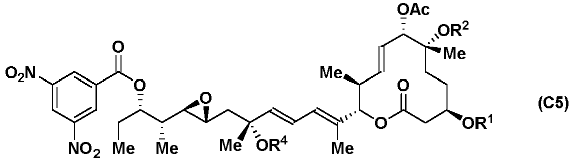 or a salt thereof,
wherein R¹, R², and R⁴ are each independently hydrogen or a silyl protecting group.

26. A compound represented by formula (C7): 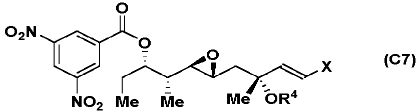 or a salt thereof,
wherein X is hydrogen or optionally substituted boryl and R⁴ is hydrogen or a silyl protecting group.

27. A crystal of pladienolide D 2-methyltetrahydrofuran solvate represented by formula (1d):

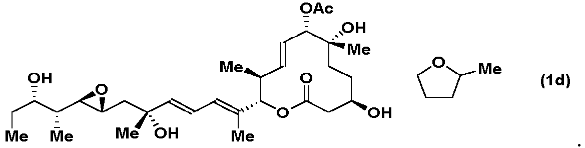

28. The crystal according to claim 27, having one or more diffraction peaks at diffraction angles (2θ ± 0.2°) selected from the group consisting of 10.3°, 10.7°, 11.7°, 13.3°, 14.4°, 17.2°, 18.9°, 21.2°, 22.5°, and 23.4° in powder X-ray diffraction.

29. The crystal according to claim 27 or 28, having one or more peaks at chemical shifts (δ ± 0.5 ppm) selected from the group consisting of 9.1 ppm, 10.0 ppm, 10.5 ppm, 16.2 ppm, 21.4 ppm, 23.9 ppm, 24.2 ppm, 25.4 ppm, 26.8 ppm, 28.0 ppm, 28.5 ppm, 33.3 ppm, 33.7 ppm, 35.9 ppm, 39.9 ppm, 41.1 ppm, 42.7 ppm, 45.7 ppm, 56.0 ppm, 59.3 ppm, 67.9 ppm, 70.4 ppm, 72.6 ppm, 76.5 ppm, 77.7 ppm, 83.0 ppm, 124.0 ppm, 124.4 ppm, 128.0 ppm, 130.2 ppm, 134.4 ppm, 139.1 ppm, 141.0 ppm, 141.3 ppm, 166.9 ppm, and 170.4 ppm in a solid state ¹³C NMR spectrum.
